# EUROPEAN PATENT APPLICATION

(11) **EP 3 725 892 A1**
(43) Date of publication of application: **21.10.2020**
(21) Application number: 20153153.0
(22) Date of filing: 14.03.2013
(51) Int. Cl.: C12Q 1/68, G01N 33/53, A61K 38/00, A61K 39/395, C07K 16/00, C07K 16/24, G01N 33/68, A61P 11/00, C12Q 1/6883

(54) **METHODS OF PROGNOSING, DIAGNOSING AND TREATING IDIOPATHIC PULMONARY FIBROSIS**

(30) Priority: 27.03.2012 US 201261616394 P; 28.09.2012 US 201261707411 P
(62) Divisional of application: 13768451.0
(71) Applicant: F. Hoffmann-La Roche AG, 4070 Basel (CH)
(72) Inventor: Abbas, Alexander R., South San Francisco, California 94080 (US); Arron, Joseph R., South San Francisco, California 94080 (US); Chandriani, Sanjay, San Francisco, California 94110 (US); Jia, Guiquan, South San Francisco, California 94080 (US); Lewin-Koh, Nicholas J. I., South San Francisco, California 94080 (US); Depianto, Daryle, South San Francisco, California 94080 (US)
(74) Representative: Heiroth, Ulrike Hildegard

(57) **Abstract**

Compositions, kits and methods for assessing the prognosis of idiopathic pulmonary fibrosis in patients are provided. In addition, compositions, kits and methods for diagnosing subtypes of idiopathic pulmonary fibrosis are provided. Also provided are methods for treating idiopathic pulmonary fibrosis.

SEQUENCE LISTING

<110> GENENTECH, INC. ET AL.

<120> METHODS OF PROGNOSING, DIAGNOSING AND TREATING IDIOPATHIC PULMONARY
FIBROSIS

<130> P4841R1C1

<140>
<141>

<150> PCT/US2013/31178
<151> 2013-03-14

<150> 61/707,411
<151> 2012-09-28

<150> 61/616,394
<151> 2012-03-27

<160> 14

<170> PatentIn version 3.5

<210> 1
<211> 5
<212> PRT
<213> Artificial Sequence

<220>
<223> Description of Artificial Sequence: Synthetic
      peptide

<400> 1
Ala Tyr Ser Val Asn
1               5


<210> 2
<211> 16
<212> PRT
<213> Artificial Sequence

<220>
<223> Description of Artificial Sequence: Synthetic
      peptide

<400> 2
Met Ile Trp Gly Asp Gly Lys Ile Val Tyr Asn Ser Ala Leu Lys Ser
1               5                   10                  15


<210> 3
<211> 10
<212> PRT
<213> Artificial Sequence

<220>
<223> Description of Artificial Sequence: Synthetic
      peptide

<400> 3

Asp Gly Tyr Tyr Pro Tyr Ala Met Asp Asn
1               5                   10

<210> 4
<211> 15
<212> PRT
<213> Artificial Sequence

<220>
<223> Description of Artificial Sequence: Synthetic
      peptide

<400> 4
Arg Ala Ser Lys Ser Val Asp Ser Tyr Gly Asn Ser Phe Met His
1               5                   10                  15

<210> 5
<211> 7
<212> PRT
<213> Artificial Sequence

<220>
<223> Description of Artificial Sequence: Synthetic
      peptide

<400> 5
Leu Ala Ser Asn Leu Glu Ser
1               5

<210> 6
<211> 9
<212> PRT
<213> Artificial Sequence

<220>
<223> Description of Artificial Sequence: Synthetic
      peptide

<400> 6
Gln Gln Asn Asn Glu Asp Pro Arg Thr
1               5

<210> 7
<211> 117
<212> PRT
<213> Artificial Sequence

<220>
<223> Description of Artificial Sequence: Synthetic
      polypeptide

<400> 7
Val Thr Leu Arg Glu Ser Gly Pro Ala Leu Val Lys Pro Thr Gln Thr
1               5                   10                  15

Leu Thr Leu Thr Cys Thr Val Ser Gly Phe Ser Leu Ser Ala Tyr Ser
            20                  25                  30

Val Asn Trp Ile Arg Gln Pro Pro Gly Lys Ala Leu Glu Trp Leu Ala
        35                  40                  45

Met Ile Trp Gly Asp Gly Lys Ile Val Tyr Asn Ser Ala Leu Lys Ser
    50                  55                  60

Arg Leu Thr Ile Ser Lys Asp Thr Ser Lys Asn Gln Val Val Leu Thr
65                  70                  75                  80

Met Thr Asn Met Asp Pro Val Asp Thr Ala Thr Tyr Tyr Cys Ala Gly
                85                  90                  95

Asp Gly Tyr Tyr Pro Tyr Ala Met Asp Asn Trp Gly Gln Gly Ser Leu
            100                 105                 110

Val Thr Val Ser Ser
        115

<210> 8
<211> 118
<212> PRT
<213> Artificial Sequence

<220>
<223> Description of Artificial Sequence: Synthetic
polypeptide

<400> 8

<210> 9
<211> 112
<212> PRT
<213> Artificial Sequence

<220>
<223> Description of Artificial Sequence: Synthetic
      polypeptide

<400> 9
Asp Ile Val Met Thr Gln Ser Pro Asp Ser Leu Ser Val Ser Leu Gly
1               5                   10                  15

Glu Arg Ala Thr Ile Asn Cys Arg Ala Ser Lys Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln Gln Asn Asn
                85                  90                  95

Glu Asp Pro Arg Thr Phe Gly Gly Gly Thr Lys Val Glu Ile Lys Arg
            100                 105                 110

<210> 10
<211> 443
<212> PRT
<213> Artificial Sequence

<220>
<223> Description of Artificial Sequence: Synthetic
      polypeptide

<400> 10
Val Thr Leu Arg Glu Ser Gly Pro Ala Leu Val Lys Pro Thr Gln Thr
1               5                   10                  15

Leu Thr Leu Thr Cys Thr Val Ser Gly Phe Ser Leu Ser Ala Tyr Ser
            20                  25                  30

Val Asn Trp Ile Arg Gln Pro Pro Gly Lys Ala Leu Glu Trp Leu Ala
        35                  40                  45

Met Ile Trp Gly Asp Gly Lys Ile Val Tyr Asn Ser Ala Leu Lys Ser
    50                  55                  60

Arg Leu Thr Ile Ser Lys Asp Thr Ser Lys Asn Gln Val Val Leu Thr
65                  70                  75                  80

Met Thr Asn Met Asp Pro Val Asp Thr Ala Thr Tyr Tyr Cys Ala Gly
                85                  90                  95

Asp Gly Tyr Tyr Pro Tyr Ala Met Asp Asn Trp Gly Gln Gly Ser Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Glu Ser Lys Tyr Gly Pro Pro Cys Pro
    210                 215                 220

Pro Cys Pro Ala Pro Glu Phe Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser Gln Glu Asp Pro Glu Val Gln Phe
            260                 265                 270

Asn Trp Tyr Val Asp Gly Val Glu Val His Asn Ala Lys Thr Lys Pro
        275                 280                 285

Arg Glu Glu Gln Phe Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Gly Leu Pro Ser Ser Ile Glu Lys Thr Ile Ser Lys Ala
                325                 330                 335

Lys Gly Gln Pro Arg Glu Pro Gln Val Tyr Thr Leu Pro Pro Ser Gln
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Arg Leu Thr Val Asp Lys Ser Arg Trp Gln Glu
                405                 410                 415

Gly Asn Val Phe Ser Cys Ser Val Met His Glu Ala Leu His Asn His
            420                 425                 430

Tyr Thr Gln Lys Ser Leu Ser Leu Ser Leu Gly
        435                 440

<210> 11
<211> 444
<212> PRT
<213> Artificial Sequence

<220>
<223> Description of Artificial Sequence: Synthetic
      polypeptide

<400> 11
Gln Val Thr Leu Arg Glu Ser Gly Pro Ala Leu Val Lys Pro Thr Gln
1               5                   10                  15

Thr Leu Thr Leu Thr Cys Thr Val Ser Gly Phe Ser Leu Ser Ala Tyr
            20                  25                  30

Ser Val Asn Trp Ile Arg Gln Pro Pro Gly Lys Ala Leu Glu Trp Leu
        35                  40                  45

Ala Met Ile Trp Gly Asp Gly Lys Ile Val Tyr Asn Ser Ala Leu Lys
    50                  55                  60

Ser Arg Leu Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Gly Asp Gly Tyr Tyr Pro Tyr Ala Met Asp Asn Trp Gly Gln Gly Ser
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly
        435                 440

<210> 12
<211> 444
<212> PRT
<213> Artificial Sequence

<220>
<223> Description of Artificial Sequence: Synthetic
      polypeptide

<400> 12
Val Thr Leu Arg Glu Ser Gly Pro Ala Leu Val Lys Pro Thr Gln Thr
1               5                   10                  15

Leu Thr Leu Thr Cys Thr Val Ser Gly Phe Ser Leu Ser Ala Tyr Ser
            20                  25                  30

Val Asn Trp Ile Arg Gln Pro Pro Gly Lys Ala Leu Glu Trp Leu Ala
        35                  40                  45

Met Ile Trp Gly Asp Gly Lys Ile Val Tyr Asn Ser Ala Leu Lys Ser
    50                  55                  60

Arg Leu Thr Ile Ser Lys Asp Thr Ser Lys Asn Gln Val Val Leu Thr
65                  70                  75                  80

Met Thr Asn Met Asp Pro Val Asp Thr Ala Thr Tyr Tyr Cys Ala Gly
                85                  90                  95

Asp Gly Tyr Tyr Pro Tyr Ala Met Asp Asn Trp Gly Gln Gly Ser Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Lys Thr Tyr Thr Cys Asn Val Asp His Lys Pro Ser Asn
        195                 200                 205

Thr Lys Val Asp Lys Arg Val Glu Ser Lys Tyr Gly Pro Pro Cys Pro
    210                 215                 220

Pro Cys Pro Ala Pro Glu Phe Leu Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser Gln Glu Asp Pro Glu Val Gln Phe
            260                 265                 270

Asn Trp Tyr Val Asp Gly Val Glu Val His Asn Ala Lys Thr Lys Pro
        275                 280                 285

Arg Glu Glu Gln Phe Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Gly Leu Pro Ser Ser Ile Glu Lys Thr Ile Ser Lys Ala
                325                 330                 335

Lys Gly Gln Pro Arg Glu Pro Gln Val Tyr Thr Leu Pro Pro Ser Gln
            340                 345                 350

Glu Glu Met Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Arg Leu Thr Val Asp Lys Ser Arg Trp Gln Glu
                405                 410                 415

Gly Asn Val Phe Ser Cys Ser Val Met His Glu Ala Leu His Asn His
            420                 425                 430

Tyr Thr Gln Lys Ser Leu Ser Leu Ser Leu Gly Lys
        435                 440

<210> 13
<211> 445
<212> PRT
<213> Artificial Sequence

<220>
<223> Description of Artificial Sequence: Synthetic
polypeptide

<400> 13
Gln Val Thr Leu Arg Glu Ser Gly Pro Ala Leu Val Lys Pro Thr Gln
1               5                   10                  15

Thr Leu Thr Leu Thr Cys Thr Val Ser Gly Phe Ser Leu Ser Ala Tyr
            20                  25                  30

Ser Val Asn Trp Ile Arg Gln Pro Pro Gly Lys Ala Leu Glu Trp Leu
        35                  40                  45

Ala Met Ile Trp Gly Asp Gly Lys Ile Val Tyr Asn Ser Ala Leu Lys
    50                  55                  60

Ser Arg Leu Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Gly Asp Gly Tyr Tyr Pro Tyr Ala Met Asp Asn Trp Gly Gln Gly Ser
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Arg Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys
        435                 440                 445

<210> 14
<211> 218
<212> PRT
<213> Artificial Sequence

<220>
<223> Description of Artificial Sequence: Synthetic
      polypeptide

<400> 14
Asp Ile Val Met Thr Gln Ser Pro Asp Ser Leu Ser Val Ser Leu Gly
1               5                   10                  15

Glu Arg Ala Thr Ile Asn Cys Arg Ala Ser Lys Ser Val Asp Ser Tyr
            20                  25                  30

Gly Asn Ser Phe Met His Trp Tyr Gln Gln Lys Pro Gly Gln Pro Pro
        35                  40                  45

Lys Leu Leu Ile Tyr Leu Ala Ser Asn Leu Glu Ser Gly Val Pro Asp
    50                  55                  60

Arg Phe Ser Gly Ser Gly Ser Gly Thr Asp Phe Thr Leu Thr Ile Ser
65                  70                  75                  80

Ser Leu Gln Ala Glu Asp Val Ala Val Tyr Tyr Cys Gln Gln Asn Asn
                85                  90                  95

Glu Asp Pro Arg Thr Phe Gly Gly Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

## Description

### CROSS-REFERENCE TO RELATED APPLICATIONS

This application claims the benefit of priority of provisional U.S. Application No. 61/616,394 filed on March 27, 2012 and of provisional U.S Application No. 61/707,411 filed on September 28, 2012, both of which are hereby incorporated by reference in their entirety.

### SEQUENCE LISTING

The instant application contains a Sequence Listing which has been submitted in ASCII format via EFS-Web and is hereby incorporated by reference in its entirety. Said ASCII copy, created on March 6, 2013, is named P4841R1_SequenceListing.txt and is 22,866 bytes in size.

### FIELD

Compositions, kits and methods for assessing the prognosis of idiopathic pulmonary fibrosis in patients are provided. In addition, compositions, kits and methods for diagnosing subtypes of idiopathic pulmonary fibrosis are provided. Also provided are methods for treating idiopathic pulmonary fibrosis.

### BACKGROUND

Idiopathic pulmonary fibrosis (IPF) is a restrictive lung disease characterized by progressive interstitial fibrosis of lung parenchyma, affecting approximately 100,000 patients in the United States (Raghu et al., Am J Respir Crit Care Med 174:810-816 (2006)). This interstitial fibrosis associated with IPF leads to progressive loss of lung function, resulting in death due to respiratory failure in most patients. The median survival from the time of diagnosis is 2-3 years (Raghu et al., Am J Respir Crit Care Med 183:788-824 (2011)). The etiology and key molecular and pathophysiological drivers of IPF are unknown. The only treatment shown to prolong survival in IPF patients is lung transplantation (Thabut et al., Annals of internal medicine 151:767-774 (2009)). Lung transplantation, however, is associated with considerable morbidity, not all IPF patients are appropriate candidates for it, and there is a relative paucity of suitable donor lungs. Despite numerous attempts, no drug therapies to date have been shown to substantially prolong survival in a randomized, placebo-controlled interventional trial in IPF patients, although some interventions have appeared to slow the rate of lung function decline in some patients (Raghu et al., Am J Respir Crit Care Med 183:788-824 (2011); Richeldi et al., The New England J. of Med. 365:1079-1087 (2011); Rafii et al., J. Thorac. Dis. 5(1):48-73 (2013)).

Although the prognosis for all IPF patients is dire, there is considerable heterogeneity in disease trajectory (Raghu et al., Am J Respir Crit Care Med 183:788-824 (2011)). Some patients exhibit a relatively indolent course, losing lung function at a relatively constant rate over as long as 10 years or more, while others experience a more rapid decline in lung function, succumbing to death within a year or two of diagnosis. In addition, some patients suffer from acute exacerbations of the disease, typically characterized by sudden dramatic decreases in lung function. Generally, these patients do not fully recover after the acute event and often die during or shortly after an exacerbation. This heterogeneity in disease trajectory suggests that different IPF patients may have different pathophysiological factors underlying their disease, which may be differentially susceptible to molecularly targeted therapeutics.

Only a few clinical or biological markers have been suggested as candidates to predict disease trajectory of IPF patients from a single point in time (Ley et al., Am J Respir Crit Care Med 183:431-440 (2011); Ley et al., Am J Respir Crit Care Med 185:6-7 (2012)). Decreased survival has been most convincingly associated with the rate of loss of lung function over time, with patients losing > 10% of forced vital capacity (FVC) within a 6 month period having much shorter subsequent survival times than patients with more stable FVC (Collard et al., Am J Respir Crit Care Med 168:538-542 (2003)). A larger study of over 1100 patients enrolled in randomized clinical trials found that a 24-week FVC decline of 5-10% was associated with a greater than 2-fold increased risk of mortality in the subsequent year while a decline of >10% was associated with a nearly 5-fold increased risk of mortality in the subsequent year (du Bois et al., Am J Respir Crit Care Med 184:1382-1389 (2011)). However, a disadvantage of using such an assessment to stratify patient enrollment in a clinical study is the six-month run-in period. Certain peripheral blood biomarkers measured at a single point in time have been reported to be prognostic for survival or disease progression, including MMP7, IL-8, ICAM1, VCAM1, S100A12 (Richards et al., Am J Respir Crit Care Med, doi:10.1164/rccm.201101-0058OC (2011)), KL-6 (Yokoyama et al., Respirology 11:164-168 (2006)), CCL18 (Prasse et al., Am J Respir Crit Care Med 179:717-723 (2009)), YKL-40 (Korthagen et al., Respiratory medicine 105:106-113 (2011)), and surfactant proteins (Kinder et al., Chest 135:1557-1563 (2009)). Many of these biomarker studies, however, have been conducted in small cohorts without replication and have employed suboptimal, inconsistent, and/or unvalidated biomarker detection technologies.

Given the limited understanding of targetable molecular mechanisms, the variability in disease trajectory, and time and expense of conducting survival studies in unselected populations of IPF patients, designing appropriately powered clinical studies to assess the potential of a candidate therapeutic to prolong survival in IPF is extremely challenging. Biomarkers that identify the activity of potentially targetable pathways and provide an accurate prognosis of subsequent disease progression would help to appropriately stratify enrollment in interventional trials to better assess the therapeutic value of investigational drug candidates.

Thus, there is a need for more effective means for determining which patients will progress more rapidly than others or which patients will have a shortened survival time compared to the median and for incorporating such determinations into more effective clinical trial designs and treatment regimens for IPF patients.

It would therefore be highly advantageous to have additional prognostic and diagnostic methods, including molecular-based prognostic and diagnostic methods, that can be used to objectively identify the presence of and/or classify the disease in a patient, define pathophysiologic aspects of IPF, clinical activity, and prognosis, including prognosis for survival. In addition, it would be advantageous to have molecular-based diagnostic and prognostic markers associated with various clinical and/or pathophysiological and/or other biological indicators of disease. Thus, there is a continuing need to identify new molecular biomarkers associated with IPF as well as other restrictive lung disorders.

The invention described herein meets the above-described needs and provides other benefits.

The interleukin (IL)-13 is a pleiotropic T helper cell subclass 2 (Th2) cytokine. Like IL4, IL13 belongs to the family of type I cytokines sharing the tertiary structure defined by a 4α-helical hydrophobic bundle core. IL13 has approximately 30% amino acid sequence homology with IL4 and shares many of the properties of IL4 (Wynn, Ann. Rev. Immunol., 21: 425 (2003)). The functional similarity of IL4 and IL13 is attributed to the fact that IL13 can bind IL4 receptor alpha chain (IL4R-α) subsequent to its binding to IL13 receptor alpha chain-1 (IL13Rα1) (Hershey, J. Allergy Clin. Immunol., 111: 677 (2003)). IL4Rα is activated by IL4 and IL13 resulting in Jak1-dependent STAT6 phosphorylation. Both IL4 and IL13 promote B-cell proliferation and induce class switching to IgG4 and IgE in combination with CD40/CD40L costimulation (Punnonen et al., Proc. Natl. Acad. Sci. USA, 90: 3730 (1993), Oettgen et al., J. Allergy Clin. Immunol., 107: 429 (2001)).

However, unlike IL4, IL13 is not involved in the differentiation of naive T cells into Th2 cells (Zurawski et al., Immunol. Today, 15: 19 (1994)). IL13 up-regulates FcεRI and thus helps in IgE priming of mast cells (de Vries, Allergy Clin. Immunol. 102: 165 (1998). In monocytes/macrophages, IL13 up-regulates expression of CD23 and MHC class I and class II antigens, down-regulate the expression of Fcγ and CD14, and inhibit antibody-dependent cytotoxicity (de Waal Malefyt et al., J. Immunol., 151: 6370 (1993), Chomarat et al., Int. Rev. Immunol., 17: 1 (1998)). IL13, but not IL4, promotes eosinophil survival, activation, and recruitment (Horie et al., Intern. Med., 36: 179 (1997), Luttmann et al., J. Immunol. 157: 1678 (1996), Pope et al., J. Allergy Clin. Immunol., 108: 594 (2001). IL13 also manifests important functions on nonhematopoietic cells, such as smooth muscle cells, epithelial cells, endothelial cells and fibroblast cells. IL13 enhances proliferation and cholinergic-induced contractions of smooth muscles (Wills-Karp, J. Allergy Clin. Immunol., 107: 9 (2001). In epithelial cells IL13 is a potent inducer of chemokine production (Li et al., J. Immunol., 162: 2477 (1999), alters mucociliary differentiation (Laoukili et al., J. Clin. Invest., 108: 1817 (2001), decreases ciliary beat frequency of ciliated epithelial cells (Laoukili et al., J. Clin. Invest., 108: 1817 (2001), and results in goblet cell metaplasia (Zhu et al., J. Clin. Invest., 103: 779 (1999), Grunig et al., Science, 282: 2261 (1998)). In endothelial cells IL13 is a potent inducer of vascular cell adhesion molecule 1 (VCAM-1) which is important for recruitment of eosinophils (Bochner et al., J. Immunol., 154: 799 (1995)). In human dermal fibroblasts IL13 induces type 1 collagen synthesis in human dermal fibroblasts (Roux et al., J. Invest. Dermatol., 103: 444 (1994)).

IL-13 antagonists, including anti-IL-13 antibodies have previously been described. See, e.g., Intn'l Patent Application Pub. No. WO 2005/062967. Such antibodies have also been developed as human therapeutics. The results of one clinical study with one anti-IL-13 antibody, lebrikizumab, has been described in Corren et al., New Engl. J. Med. 365:1088-1098 (2011).

All references cited herein, including patent applications and publications, are incorporated by reference in their entirety for any purpose.

### SUMMARY

The compositions and methods of the invention are based, at least in part, on the definition of at least three new and distinct molecular phenotypes (also referred to herein as molecular subtypes) of idiopathic pulmonary fibrois (IPF). The IPF molecular subtypes described herein were defined based on differential gene expression between the subtypes. In addition, compositions and methods of the invention are based, at least in part, on the identification of serum or blood biomarkers that are prognostic for survival of IPF patients. Such biomarkers are particularly useful for identifying and selecting IPF patients for therapeutic treatment according to the methods of the invention. The terms "molecular phenotype" and "molecular subtype" are used interchangeably herein.

Accordingly, in one aspect, methods of prognosing or of aiding prognosis of idiopathic pulmonary fibrosis (IPF) are provided. In certain embodiments, a biological sample is obtained from the patient and the expression of one or a combination of genes, or expression of one or a combination of proteins encoded by the one or the combination of genes is measured. In certain embodiments, an elevated expression level of the one or the combination of genes or elevated expression of the one or the combination of proteins encoded by the one or the combination of genes, is indicative of a prognosis for shortened survival compared to median survival. In certain embodiments, reduced expression of the one or the combination of genes or reduced expression of the one or the combination of proteins encoded by the one or the combination of genes, is indicative of a prognosis for increased survival compared to median survival. In one embodiment, the one or the combination of genes is selected from any of Table 2, Table 3, Table 4, or Table 5. In one embodiment, the one or the combination of genes is selected from MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3, and SAA4. In one embodiment, the one or the combination of genes is selected from CXCR3, CXCR5, CXCL13, CCR6, CCR7, CD19, MS4A1 (CD20), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@, IGHA1, IGKV2-24, IGKV1D-8, IGHM. In one embodiment, the one or the combination of genes is selected from COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3. In certain embodiments, the gene expression level is measured by assaying for mRNA levels. In certain embodiments, the assay comprises a PCR method and/or the use of a microarray chip. In one embodiment, the PCR method is qPCR. In one embodiment, the PCR method is multiplex-PCR. In certain embodiments, kits include at least one enzyme selected from a nuclease, a ligase, and a polymerase. In certain embodiments, the protein expression level is measured by an immunoassay. In certain embodiments, immunoassay kits are provided comprising one or more antibodies that bind to one or more proteins encoded by one of the genes identified above.

In yet a further embodiment of the methods above, the one or the combination of genes is selected from CHI3L1 (YKL-40), CCL11, CCL13, CCL17, CCL18, COMP, CXCL13, MMP3, MMP7, SAA4 (constitutive SAA), POSTN, AND SPP1 (OPN). In another embodiment, the one or the combination of genes is selected from YKL-40, CCL11, CCL13, CCL17, CCL18, MMP7, CXCL13, COMP, SAA and OPN. In still another embodiment, the one or the combination of genes is selected from SAA, MMP3, CXCL13, OPN, COMP, and YKL-40. In another embodiment, the one or the combination of genes is selected from POSTN, MMP3, and CXCL13. In a still further embodiment, the expression level of YKL-40 or the expression level of CCL18 and/or the expression level of CXCL13 is measured. In yet another embodiment, the expression level of MMP3 and/or the expression level of SAA is measured. In certain embodiments, the gene expression level is measured by assaying for mRNA levels. In certain embodiments, the assay comprises a PCR method and/or the use of a microarray chip. In one embodiment, the PCR method is qPCR. In one embodiment, the PCR method is multiplex-PCR. In certain embodiments, kits include at least one enzyme selected from a nuclease, a ligase, and a polymerase. In certain embodiments, the protein expression level is measured by an immunoassay. In certain embodiments, immunoassay kits are provided comprising one or more antibodies that bind to one or more proteins encoded by one of the genes identified above.

In certain embodiments of the methods described above, the patient is on immunomodulatory therapy. In another embodiment, the biological sample is selected from lung tissue, serum, and plasma.

In one aspect, gene expression according to the methods described above is measured by microarray. In another aspect gene expression is measured by real-time quantitative polymerase chain reaction (qPCR). In another aspect, gene expression is measured by multiplex-PCR. According to another embodiment, gene expression is measured by observing protein expression levels of one or more of the genes described above. According to another embodiment, expression of a gene of interest is considered elevated when compared to a healthy control or a reference subject if the relative mRNA level of the gene of interest is greater than 2 fold of the level of a control or reference gene mRNA. According to another embodiment, the relative mRNA level of the gene of interest is greater than 3 fold, fold, 10 fold, 15 fold, 20 fold, 25 fold, or 30 fold compared to a healthy control or reference gene expression level. In one aspect, the gene expression level is measured by a method selected from a PCR method, a microarray method, or an immunoassay method. In one embodiment, the microarray method comprises the use of a microarray chip having one or more nucleic acid molecules that can hybridize under stringent conditions to a nucleic acid molecule encoding a gene mentioned above or having one or more polypeptides (such as peptides or antibodies) that can bind to one or more of the proteins encoded by the genes mentioned above. In one embodiment, the PCR method is qPCR. In one embodiment, the PCR method is multiplex-PCR. According to one embodiment, the immunoassay method comprises binding an antibody to protein expressed from a gene mentioned above in a patient sample and determining if the protein level from the patient sample is elevated. In certain embodiments, the immunoassay method is an enzyme-linked immunosorbent assay (ELISA).

In another aspect, methods of prognosing or of aiding prognosis of IPF in a patient are provided where a biological sample is obtained from the patient and a total baseline biomarker score is determined. In certain embodiments, the determining of the total baseline biomarker score comprises measuring the protein expression level of at least one of CXCL13, OPN, and COMP and assigning a score of 0 if the expression level is below the median for CXCL13, OPN, and COMP, respectively, and assigning a score of 1 if the expression level is above the median for CXCL13, OPN, and COMP, respectively, wherein the determining of the total baseline biomarker score further comprises measuring the protein expression level of YKL-40 and assigning a score of 0 if the expression level is below the median for YKL-40 and assigning a score of 1 if the expression level is above the median for YKL-40, and wherein the determining of the total baseline biomarker score further comprises adding each individual score to obtain the total baseline biomarker score. In certain embodiments, a total baseline biomarker score of two or above is indicative of a prognosis of shortened survival compared to median survival. In certain embodiments, a total baseline biomarker score of zero or one is indicative of a prognosis of increased survival compared to the median survival. In certain embodiments, the protein expression level is measured by an immunoassay. In certain embodiments, immunoassay kits are provided comprising one or more antibodies that bind to one or more proteins encoded by CXCL13, OPN, COMP and/or YKL-40.

In yet still another aspect, if the total baseline biomarker score of the patient is two or above as determined as described above, the patient is selected for treatment with a candidate therapeutic agent in a clinical study. In certain embodiments, the candidate therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120). In one embodiment, the anti-IL-13 agent is lebrikizumab. In one embodiment, the anti-IL-13/anti-IL-4 agent is a bispecific antibody. In one embodiment, the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6. In one embodiment, the anti-IL-13 antibody comprises a heavy chain variable region having the amino acid sequence of SEQ ID NO.: 7 and a light chain variable region having the amino acid sequence of SEQ ID NO.: 9. In one embodiment, the anti-IL-13 antibody comprises a heavy chain having the amino acid sequence of SEQ ID NO.: 10 and a light chain having the amino acid sequence of SEQ ID NO.: 14. In certain embodiments, methods of treating an IPF patient are provided where an effective amount of an IPF therapeutic agent as provided above is administered provided that the patient has a baseline score of two or above determined according to the methods described above.

In another aspect, methods of prognosing or of aiding prognosis of IPF in a patient are provided where a biological sample is obtained from the patient and a total baseline biomarker score is determined, where the determining of the total baseline biomarker score comprises measuring the protein expression level of at least one of MMP3 and COMP and assigning a score of 0 if the expression level is below the median for MMP3 and COMP, respectively, and assigning a score of 1 if the expression level is above the median for MMP3 and COMP, respectively, wherein the determining of the total baseline biomarker score further comprises measuring the protein expression level of YKL-40 and assigning a score of 0 if the expression level is below the median for YKL-40 and assigning a score of 1 if the expression level is above the median for YKL-40, and wherein the determining of the total baseline biomarker score further comprises adding each individual score to obtain the total baseline biomarker score. In certain embodiments, a total baseline biomarker score of one or above is indicative of a prognosis of shortened survival compared to median survival. In certain embodiments, a total baseline biomarker score of zero is indicative of a prognosis of increased survival compared to the median survival. In certain embodiments, the protein expression level is measured by an immunoassay. In certain embodiments, immunoassay kits are provided comprising one or more antibodies that bind to one or more proteins encoded by MMP3, COMP and/or YKL-40.

In yet still another aspect, if the total baseline biomarker score of the patient is one or above as determined as described above with respect to expression of MMP3, COMP, and/or YKL-40, the patient is selected for treatment with a candidate therapeutic agent in a clinical study. In certain embodiments, the candidate therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120). In one embodiment, the anti-IL-13 agent is lebrikizumab. In one embodiment, the anti-IL-13/anti-IL-4 agent is a bispecific antibody. In one embodiment, the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6. In one embodiment, the anti-IL-13 antibody comprises a heavy chain variable region having the amino acid sequence of SEQ ID NO.: 7 and a light chain variable region having the amino acid sequence of SEQ ID NO.: 9. In one embodiment, the anti-IL-13 antibody comprises a heavy chain having the amino acid sequence of SEQ ID NO.: 10 and a light chain having the amino acid sequence of SEQ ID NO.: 14. In certain embodiments, methods of treating an IPF patient are provided where an effective amount of an IPF therapeutic agent as provided above is administered provided that the patient has a baseline score of one or above determined according to the methods described above.

In another aspect, methods of diagnosing a molecular subtype of IPF in a subject are provided. In certain embodiments, the methods comprise measuring in a biological sample obtained from the subject expression of one or a combination of genes, or expression of one or a combination of proteins encoded by the one or the combination of genes. In certain embodiments, the one or the combination of genes is selected from MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3, and SAA4, and elevated expression of the one or the combination of genes, or elevated expression of the one or the combination of proteins, is indicative of the IPF molecular subtype. In certain embodiments, the one or the combination of genes is selected from CXCR3, CXCR5, CXCL13, CCR6, CCR7, CD19, MS4A1 (CD20), TNFRSF17 (BCMA), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@ (immunoglobulin kappa locus), IGHA1, IGKV2-24, IGKV1D-8, IGHM, and elevated expression of the one or the combination of genes, or elevated expression of the one or the combination of proteins, is indicative of the IPF molecular subtype. In certain embodiments, the one or the combination of genes is selected from COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3, and elevated expression of the one or the combination of genes, or elevated expression of the one or the combination of proteins, is indicative of the IPF molecular subtype. In certain embodiments, the gene expression level is measured by assaying for mRNA levels. In certain embodiments, the assay comprises a PCR method and/or the use of a microarray chip. In one embodiment, the PCR method is qPCR. In one embodiment, the PCR method is multiplex-PCR. In certain embodiments, kits include at least one enzyme selected from a nuclease, a ligase, and a polymerase. In certain embodiments, the protein expression level is measured by an immunoassay. In certain embodiments, immunoassay kits are provided comprising one or more antibodies that bind to one or more proteins encoded by one of the genes identified above.

In another aspect, the biological sample for use according to any of the above methods is lung tissue, whole blood, or serum. In certain embodiments, the biological sample is lung tissue or whole blood and the expression of the one or the combination of genes is measured using a PCR method or a microarray chip. In certain embodiments, the biological sample is serum and the expression of the one or the combination of proteins is measured using an immunoassay.

In yet still another aspect, methods of treating IPF in a patient are provided. In certain embodiments, the methods comprise administering an effective amount of an IPF therapeutic agent to the patient to treat the IPF, provided that elevated expression of one or a combination of genes, or expression of one or a combination of proteins encoded by the one or the combination of genes, has been detected in a biological sample obtained from the patient. In one embodiment, the one or the combination of genes is selected from MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3, and SAA4. In one embodiment, the one or the combination of genes is selected from CXCR3, CXCR5, CXCL13, CCR6, CCR7, CD19, MS4A1 (CD20), TNFRSF17 (BCMA), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@ (immunoglobulin kappa locus), IGHA1, IGKV2-24, IGKV1D-8, IGHM. In one embodiment, the one or the combination of genes is selected from COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3. In another embodiment, the one or the combination of genes is selected from CHI3L1 (YKL-40), CCL11, CCL13, CCL17, CCL18, COMP, CXCL13, MMP3, MMP7, SAA4 (constitutive SAA), POSTN, AND SPP1 (OPN). In another embodiment, the one or the combination of genes is selected from YKL-40, CCL11, CCL13, CCL17, CCL18, MMP7, CXCL13, COMP, SAA and OPN. In still another embodiment, the one or the combination of genes is selected from SAA, MMP3, CXCL13, OPN, COMP, and YKL-40. In another embodiment, the one or the combination of genes is selected from POSTN, MMP3, and CXCL13. In still another embodiment, the expression level of YKL-40 or the expression level of CCL18 and/or the expression level of CXCL13 is measured. In yet another embodiment, the expression level of MMP3 and/or the expression level of SAA is measured. In certain embodiments, the gene expression level is measured by assaying for mRNA levels. In certain embodiments, the assay comprises a PCR method and/or the use of a microarray chip. In one embodiment, the PCR method is qPCR. In one embodiment, the PCR method is multiplex-PCR. In certain embodiments, kits include at least one enzyme selected from a nuclease, a ligase, and a polymerase. In certain embodiments, the protein expression level is measured by an immunoassay. In certain embodiments, immunoassay kits are provided comprising one or more antibodies that bind to one or more proteins encoded by one of the genes identified above. In certain embodiments, the IPF therapeutic agent is an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120). In certain embodiments, the IPF therapeutic agent is an anti-IL-13 antibody. In one embodiment, the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6. In one embodiment, the anti-IL-13 antibody comprises a heavy chain variable region having the amino acid sequence of SEQ ID NO.: 7 and a light chain variable region having the amino acid sequence of SEQ ID NO.: 9. In one embodiment, the anti-IL-13 antibody (lebrikizumab) comprises a heavy chain having the amino acid sequence of SEQ ID NO.: 10 and a light chain having the amino acid sequence of SEQ ID NO.: 14. In certain embodiments, lebrikizumab is administered subcutaneously once every four weeks at a flat dose selected from 125 mg, 250 mg, and 500 mg. In one embodiment, lebrikizumab is administered subcutaneously once every four weeks at a flat does of 250 mg.

In a further aspect, methods of treating an IPF patient previously determined to have a prognosis of shortened survival according to the methods above are provided. In certain such embodiments, an effective amount of the IPF therapeutic agent is administered. In one embodiment, the IPF therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120). In one embodiment, the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6. In one embodiment, the anti-IL-13 antibody comprises a heavy chain variable region having the amino acid sequence of SEQ ID NO.: 7 and a light chain variable region having the amino acid sequence of SEQ ID NO.: 9. In one embodiment, the anti-IL-13 antibody (lebrikizumab) comprises a heavy chain having the amino acid sequence of SEQ ID NO.: 10 and a light chain having the amino acid sequence of SEQ ID NO.: 14. In one embodiment, the anti-IL-13 antibody is administered subcutaneously once every four weeks at a flat dose selected from 125 mg, 250 mg, and 500 mg. In one embodiment, the anti-IL-13 antibody is administered subcutaneously once every four weeks at a flat dose of 250 mg.

In yet a still further aspect, the methods of treating an IPF patient with an anti-IL-13 antibody or with lebrikizumab described above extends the time to disease progression compared to no treatment, wherein disease progression is indicated by the first occurrence of one or more of the following: (i) death; (ii) non-elective hospitalization; (iii) 10% or greater decrease from baseline in FVC. In one embodiment, disease progression is further indicated by decrease from baseline of ≥ 15% in DL_{CO} at week 52. In one embodiment, treatment with an anti-IL-13 antibody or lebrikizumab as described above results in a decrease in DL_{CO} from baseline 52 weeks after treatment is less than 15%. In one embodiment, such treatment results in less reduction in decline from baseline in distance walked by the patient in a 6-minute walk test 52 weeks after treatment compared to no treatment. In one embodiment, the reduction in decline from baseline in distance walked is greater than 50 meters, or greater than 30 meters, or greater than 10 meters. In certain embodiments, the treatment extends the time to a first event of acute IPF exacerbation or a first event of IPF deterioration compared to no treatment.

In one aspect, methods of extending the time to disease progression in an IPF patient by administering an anti-IL-13 antibody or lebrikizumab are provided. In certain embodiments, the time to disease progression in treated patients is compared to no treatment. In certain embodiments, disease progression is indicated by the first occurrence of one or more of the following: (i) death; (ii) non-elective hospitalization; (iii) 10% or greater decrease from baseline in FVC. In one embodiment, disease progression is further indicated by decrease from baseline of > 15% in DLco at week 52. In one embodiment, treatment with an anti-IL-13 antibody or lebrikizumab as described above results in a decrease in DLco from baseline 52 weeks after treatment is less than 15%. In one embodiment, such treatment results in less reduction in decline from baseline in distance walked by the patient in a 6-minute walk test 52 weeks after treatment compared to no treatment. In one embodiment, the reduction in decline from baseline in distance walked is greater than 50 meters, or greater than 30 meters, or greater than 10 meters. In certain embodiments, the treatment extends the time to a first event of acute IPF exacerbation or a first event of IPF deterioration compared to no treatment.

In still yet another aspect, methods of monitoring disease progression in an IPF patient are provided. In certain embodiments, the methods comprise obtaining a biological sample from the patient at a first time point and one or more additional time points, measuring in the biological samples the expression of one or a combination of genes, or expression of one or a combination of proteins encoded by the one or the combination of genes, wherein the one or the combination of genes is selected from any of Table 2, Table 3, Table 4, or Table 5, wherein a change in expression level from the first time point to the one or more additional time points is indicative of disease progression. In one embodiment, the one or the combination of genes, or the one or the combination of proteins, is selected from MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3, and SAA4. In one embodiment, the one or the combination of genes is selected from CXCR3, CXCR5, CXCL13, CCR6, CCR7, CD19, MS4A1 (CD20), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@, IGHA1, IGKV2-24, IGKV1D-8, IGHM. In one embodiment, the one or the combination of genes, or the one or the combination of proteins, is selected from COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3. In one embodiment, the one or the combination of genes, or the one or the combination of proteins is selected from CHI3L1 (YKL-40), CCL11, CCL13, CCL17, CCL18, COMP, CXCL13, MMP3, MMP7, SAA4 (constitutive SAA), POSTN, AND SPP1 (OPN). In certain embodiments, a biological sample is selected from lung tissue, serum, and plasma. In certain embodiments, the gene expression level is measured by assaying for mRNA levels. In certain embodiments, the assay comprises a PCR method and/or the use of a microarray chip. In one embodiment, the PCR method is qPCR. In one embodiment, the PCR method is multiplex-PCR. In certain embodiments, kits include at least one enzyme selected from a nuclease, a ligase, and a polymerase. In certain embodiments, the protein expression level is measured by an immunoassay. In certain embodiments, immunoassay kits are provided comprising one or more antibodies that bind to one or more proteins encoded by one of the genes identified above.

In another aspect, the methods of monitoring disease progression as described above further comprise treating the patient with a candidate therapeutic agent in a clinical study. In certain embodiments, the therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120). In one embodiment, the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6. In one embodiment, the anti-IL-13 antibody comprises a heavy chain variable region having the amino acid sequence of SEQ ID NO.: 7 and a light chain variable region having the amino acid sequence of SEQ ID NO.: 9. In one embodiment, the anti-IL-13 antibody comprises a heavy chain having the amino acid sequence of SEQ ID NO.: 10 and a light chain having the amino acid sequence of SEQ ID NO.: 14.

### BRIEF DESCRIPTION OF THE DRAWINGS

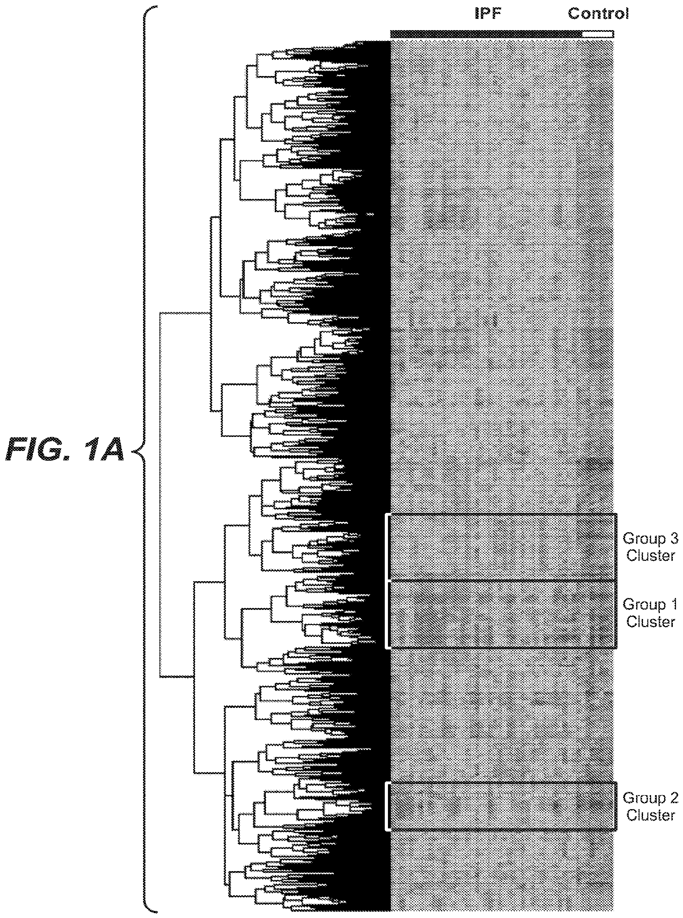

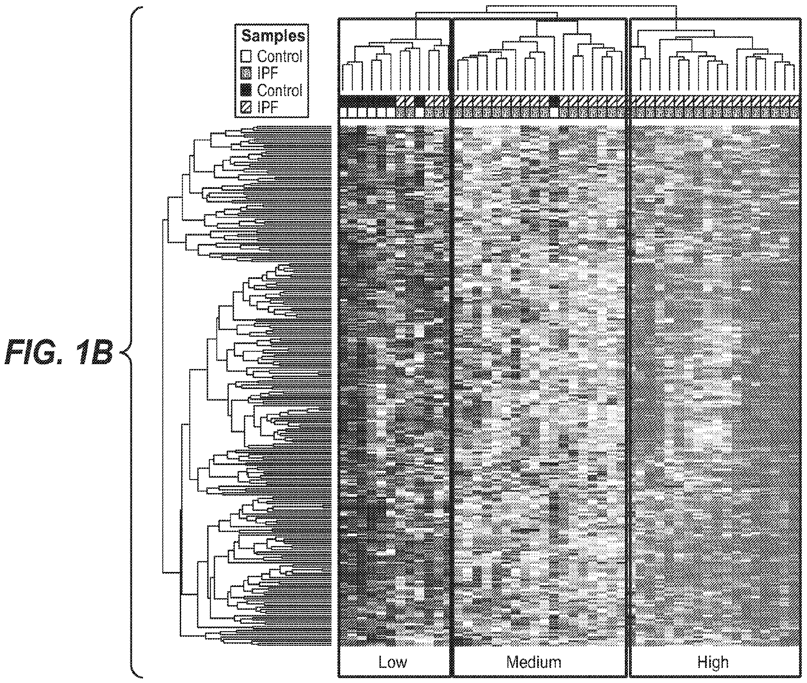

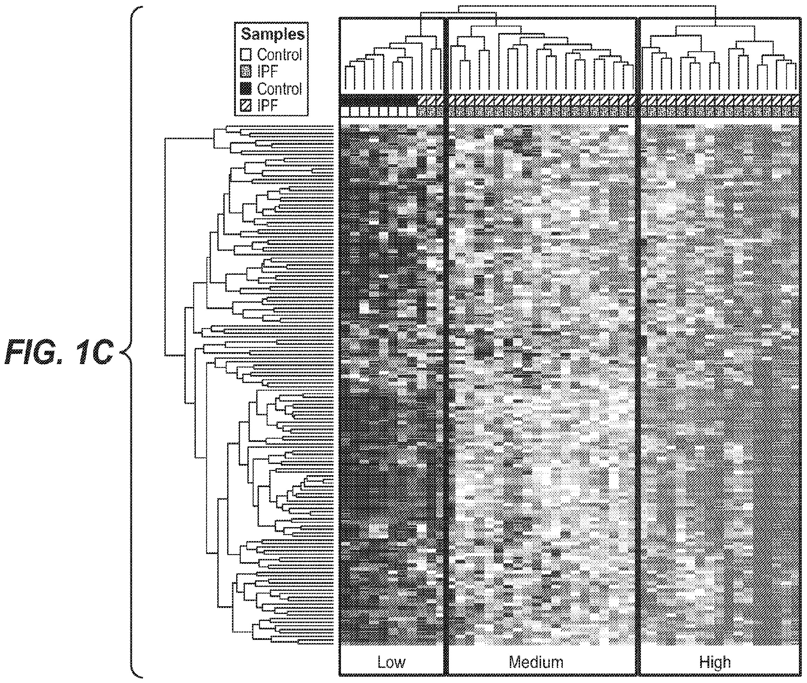

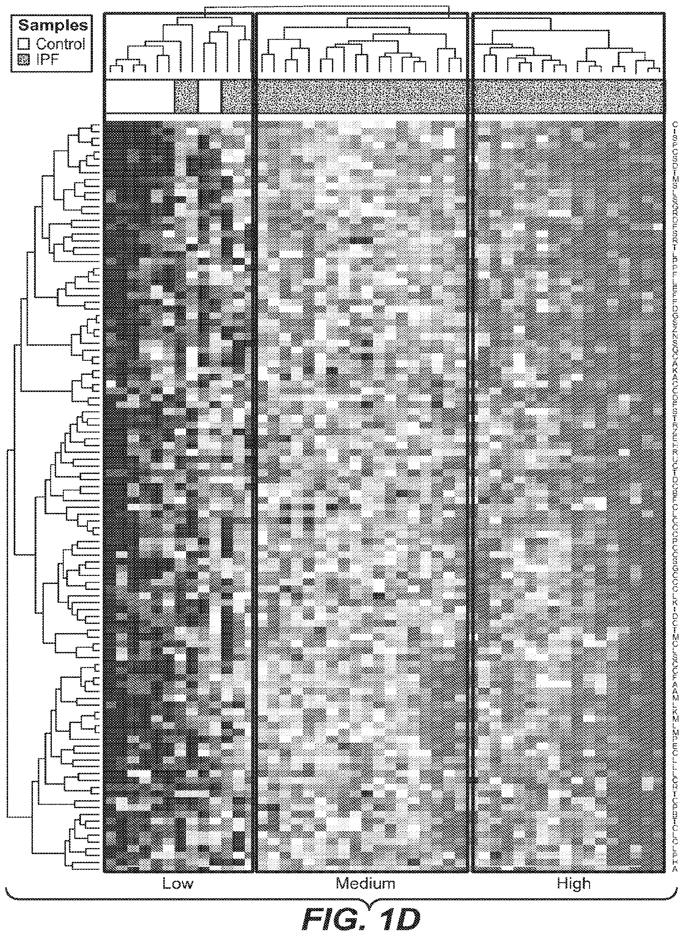

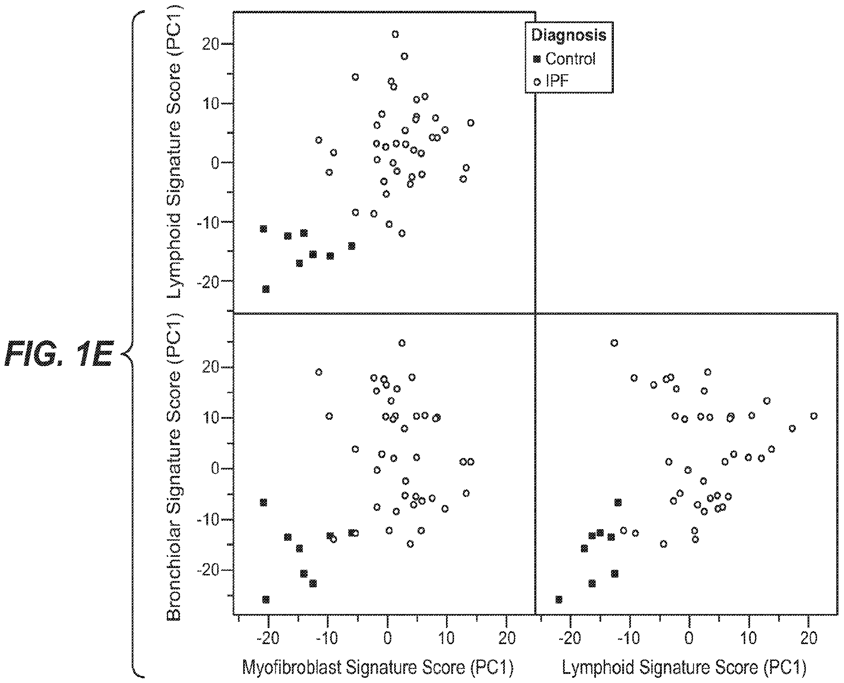

Figure 1 shows gene expression heterogeneity in IPF patients as described in Example 1. (A) Unsupervised 2-way hierarchical clustering of the 2490 DE microarray probes between IPF and controls demonstrated three major clusters (Group 1 cluster, Group 2 cluster, and Group 3 cluster) defined primarily by diagnosis of IPF; (B) re-clustering of the Group 1 cluster identified a heterogeneous gene expression signature in which the majority of IPF patients (and few controls) expressed high levels of genes characteristic of bronchial epithelium (the "bronchiolar signature"); (C) re-clustering of the Group 2 cluster identified a heterogeneous gene expression signature in which most of IPF patients (and none of the controls) expressed high levels of genes characteristic of lymphoid follicles (the "lymphoid signature"); (D) re-clustering of the Group 3 cluster identified a heterogeneous gene expression signature in which IPF patients expressed medium and high levels of genes characteristic of fibroblast differentiation into myofibroblasts while control subjects expressed low levels (the "fibroblast signature," also referred to herein as the "myofibroblast signature"); (E) plot showing that bronchiolar, lymphoid, and myofibroblast gene expression signatures do not co-vary with each other.

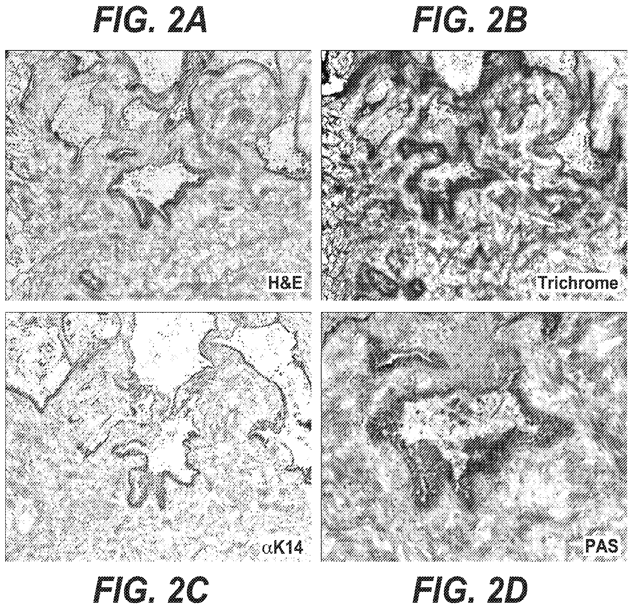

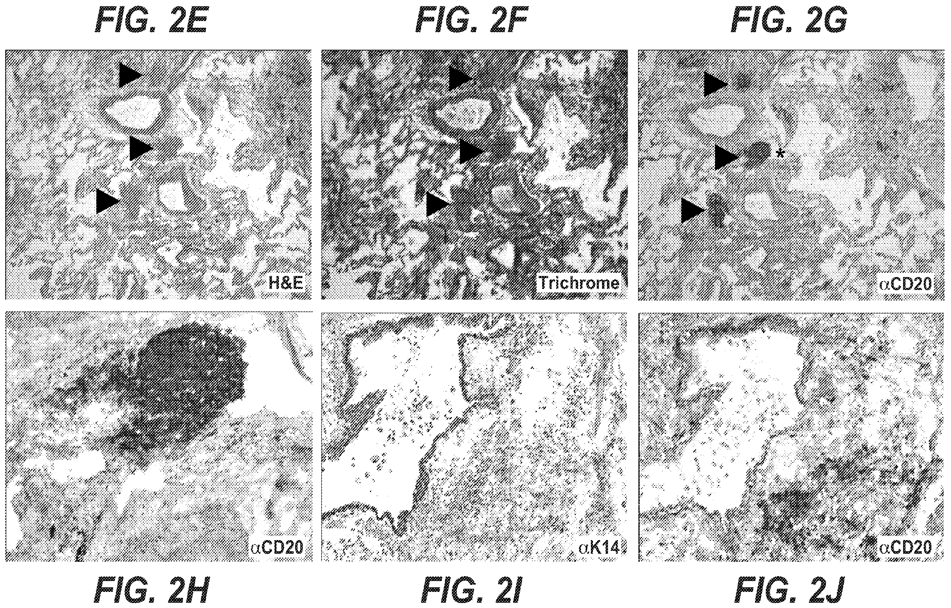

Figure 2 shows immunohistochemistry (IHC) on adjacent frozen sections of biopsy tissue taken from IPF lung explants as described in Example 1. (A) Hemotoxylin and eosin (H&E) staining; (B) trichrome staining staining; (C) anti-keratin 14 staining; (D) periodic acid-Schiff (PAS) staining; (E) H&E staining showing aggregates of cells with darkly staining nuclei (arrowheads); (F) trichrome staining showing collagen deposition surrounding, but not present within, aggregates (arrowheads); (G) anti-CD20 staining showing the aggregates have large concentrations of CD20 positive B cells; (H) higher magnification of area marked with (*) in (G); (I) anti-keratin 14 staining of bronchiolized cyst; (J) anti-CD20 staining of lymphoid aggregate.
Figure 3 shows the gene expression of candidate biomarker genes in lung tissue obtained from IPF patients and from controls as measured by qPCR as described in Example 1. (A) periostin gene expression; (B) CCL13 gene expression; (C) CCL18 gene expression; (D) osteopontin gene expression; (E) COMP gene expression; (F) YKL-40 gene expression; (G) MMP7 gene expression.
Figure 4 shows IPF survival stratified by FVC percent predicted at the median level (69%) as described in Example 1.
Figure 5 shows a Cox model of IPF survival for individual and combined prognostic biomarkers as described in Example 1. (A) Cox model of IPF survival by serum CXCL13 level; (B) Cox model of IPF survival by serum YKL-40 level; (C) Cox model of IPF survival by serum COMP level; (D) Cox model of IPF survival by plasma OPN level.
Figure 6 shows the receiver operating characteristic (ROC) analyses of possible combinations of six biomarkers (SAA, MMP3, CXCL13, OPN, COMP, and YKL-40) as described in Example 1. (A) ROC analysis of each possible combination of the six biomarkers indicated to predict mortality over 1, 2, and 3 years following sample collection; (B) Area under the curve of the ROC analysis at two years and term significance of all possible combinations of the six biomarkers (SAA, MMP3, CXCL13, OPN, COMP, and YKL-40).
Figure 7 shows a combined Cox model of IPF survival by baseline biomarker score for the combination of YKL-40 plus OPN plus COMP plus CXCL13 as described in Example 1.
Figure 8 shows a Kaplan-Meier survival plot of IPF patients expressing above median levels of none, one, two, or all three of the biomarkers MMP3, COMP and YKL-40 as described in Example 1.
Figure 9 shows the quantitative PCR results for IL-13Ra2 expression in lung tissue from control patients (left side) and from IPF patients (right side) as described in Example 2.
Figure 10 shows the induction of IL13Rα2 expression by IL-4 or IL-13 in IMR90 primary lung fibroblast cells as described in Example 2.
Figure 11 shows the effect of TNFα on IL13Rα2 expression (A) and on CCL26 and periostin expression, in the presence or absence of IL-13 (B) as described in Example 2.
Figure 12 shows the gene expression levels of CCL26 and periostin (POSTN) in IMR90 cells pretreated with TNFα and then subjected to various IL-13 and/or IL-4 cytokine and blocking anti-IL-13 mAb1 and/or mAb2 antibody treatments as indicated in the figure and as described in Example 2.
Figure 13 shows microarray data from IMR90 cells treated with IL-13 (A) or with IL-13 and IL-4 (B) and then further treated with either anti-IL-13 mAb2 or anti-IL-13 mAb2 as described in Example 2.
Figure 14 shows GLI1 expression in the IPF cohort as described in Example 2.
Figure 15 shows GLI1 gene expression in primary pulmonary fibroblasts (cultured on matrigel) in response to various added factors as described in Example 2.
Figure 16 shows TGFβ1 gene expression (A) and GLI1 gene expression (B) in primary pulmonary fibroblasts (cultured on matrigel) in response to various added factors as indicated in the figure and described in Example 2

### DETAILED DESCRIPTION

### CERTAIN DEFINITIONS

Unless otherwise defined, all terms of art, notations and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. The techniques and procedures described or referenced herein are generally well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

"Bronchiolar gene signature," "Bronchiolar signature," and "Bronchiolar gene expression signature" are used interchangeably herein and refer to a combination or subcombination of genes as set forth in Table 2, the gene expression pattern of which correlates with certain IPF patients. The genes include MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3, SAA4 (encoding constitutive SAA). The polypeptides of the bronchiolar gene signature are "targeted polypeptides" as described herein.

"Lymphoid gene signature," "Lymphoid signature," and "Lymphoid gene expression signature," "Lymphoid follicle gene signature," "Lymphoid follicle signature," and "Lymphoid follicle gene expression signature"are used interchangeably herein and refer to a combination or subcombination of genes as set forth in Table 3, the gene expression pattern of which correlates with certain IPF patients. The genes include CXCR3, CXCR5, CXCL13, CCR6, CCR7, CD19, MS4A1 (CD20), TNFRSF17 (BCMA), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@ (immunoglobulin kappa locus), IGHA1, IGKV2-24, IGKV1D-8, IGHM. The polypeptides of the lymphoid follicle gene signature are "targeted polypeptides" as described herein.

"Myofibroblast gene signature," "Myofibroblast signature," and "Myofibroblast gene expression signature," "Fibroblast gene signature, "Fibroblast signature," and "Fibroblast gene expression signature"" are used interchangeably herein and refer to a combination or subcombination of genes as set forth in Table 4, the gene expression pattern of which correlates with certain IPF patients. The genes include COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3. The polypeptides of the myofibroblast gene signature are "targeted polypeptides" as described herein.

The term "targeted polypeptide" when used herein refers to "native sequence" polypeptides and variants (which are further defined herein).

A "native sequence" polypeptide comprises a polypeptide having the same amino acid sequence as the corresponding polypeptide derived from nature. Thus, the term "native sequence polypeptide" includes naturally-occurring truncated, augmented, and frameshifted forms of a polypeptide, including but not limited to alternatively spliced forms, isoforms and polymorphisms.

"Naturally occurring variant" means a polypeptide having at least about 60% amino acid sequence identity with a reference polypeptide and retains at least one biological activity of the naturally occurring reference polypeptide. Naturally occurring variants can include variant polypeptides having at least about 65% amino acid sequence identity, at least about 70% amino acid sequence identity, at least about 75% amino acid sequence identity, at least about 80% amino acid sequence identity, at least about 80% amino acid sequence identity, at least about 85% amino acid sequence identity, at least about 90% amino acid sequence identity, at least about 95% amino acid sequence identity, at least about 98% amino acid sequence identity or at least about 99% amino acid sequence identity to a reference polypeptide.

The term "polynucleotide" or "nucleic acid," as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after polymerization, such as by conjugation with a labeling component. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, cabamates, etc.) and with charged linkages (e.g., phosphorothioates, phosphorodithioates, etc.), those containing pendant moieties, such as, for example, proteins (e.g., nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid supports. The 5' and 3' terminal OH can be phosphorylated or substituted with amines or organic capping groups moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-2'-O- allyl, 2'-fluoro- or 2'-azido-ribose, carbocyclic sugar analogs, α- anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S("thioate"), P(S)S ("dithioate"), "(O)NR 2 ("amidate"), P(O)R, P(O)OR', CO or CH 2 ("formacetal"), in which each R or R' is independently H or substituted or unsubstituted alkyl (1-20 C) optionally containing an ether (--O--) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, refers to short, single stranded polynucleotides that are at least about seven nucleotides in length and less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "primer" refers to a single stranded polynucleotide that is capable of hybridizing to a nucleic acid and allowing the polymerization of a complementary nucleic acid, generally by providing a free 3'-OH group.

The term "array" or "microarray" refers to an ordered arrangement of hybridizable array elements, preferably polynucleotide probes (e.g., oligonucleotides), on a substrate. The substrate can be a solid substrate, such as a glass slide, or a semi-solid substrate, such as nitrocellulose membrane.

The term "amplification" refers to the process of producing one or more copies of a reference nucleic acid sequence or its complement. Amplification may be linear or exponential (e.g., PCR). A "copy" does not necessarily mean perfect sequence complementarity or identity relative to the template sequence. For example, copies can include nucleotide analogs such as deoxyinosine, intentional sequence alterations (such as sequence alterations introduced through a primer comprising a sequence that is hybridizable, but not fully complementary, to the template), and/or sequence errors that occur during amplification.

The term "detection" includes any means of detecting, including direct and indirect detection.

The term "molecular subtype," used interchangeably with "molecular phenotype," refers to a subtype or phenotype of IPF characterized by the expression of one or more particular genes or one or more particular proteins, or a particular pattern of expression of a combination of genes or a combination of proteins. The expression of particular genes, proteins or combinations of genes or proteins may be further associated with certain pathological, histological, and/or clinical features of IPF.

The term "multiplex-PCR" refers to a single PCR reaction carried out on nucleic acid obtained from a single source (e.g., a patient) using more than one primer set for the purpose of amplifying two or more DNA sequences in a single reaction.

The term "biomarker" as used herein refers to an indicator of e.g, a pathological state of a patient, which can be detected in a biological sample of the patient. Biomarkers include, but are not limited to, DNA, RNA, protein, carbohydrate, or glycolipid-based molecular markers.

The term "diagnosis" is used herein to refer to the identification or classification of a molecular or pathological state, disease or condition. For example, "diagnosis" may refer to identification of a particular type of IPF or UIP. "Diagnosis" may also refer to the classification of a particular subtype of IPF, e.g., by histopathological or radiographic criteria or by molecular features (e.g., a subtype characterized by expression of one or a combination of particular genes or proteins encoded by said genes).

The term "aiding diagnosis" is used herein to refer to methods that assist in making a clinical determination regarding the presence, or nature, of a particular type of symptom or condition of IPF. For example, a method of aiding diagnosis of IPF can comprise measuring the expression of certain genes in a biological sample from an individual.

The term "prognosis" is used herein to refer to the prediction of the likelihood of survival over time as well as one or more IPF-attributable disease symptoms worsening over time.

A "control subject" refers to a healthy subject who has not been diagnosed as having IPF and who does not suffer from any sign or symptom associated with IPF.

The term "sample," as used herein, refers to a composition that is obtained or derived from a subject of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized.

By "tissue" or "cell sample" is meant a collection of similar cells obtained from a tissue of a subject or patient. The source of the tissue or cell sample may be solid tissue as from a fresh, frozen and/or preserved organ or tissue sample or biopsy or aspirate; blood or any blood constituents; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in nature such as preservatives, anticoagulants, buffers, fixatives, nutrients, antibiotics, or the like. A "reference sample", "reference cell", "reference tissue", "control sample", "control cell", or "control tissue", as used herein, refers to a sample, cell or tissue obtained from a source known, or believed, not to be afflicted with the disease or condition for which a method or composition of the invention is being used to identify. In one embodiment, a reference sample, reference cell, reference tissue, control sample, control cell, or control tissue is obtained from a healthy part of the body of the same subject or patient in whom a disease or condition is being identified using a composition or method of the invention. In one embodiment, a reference sample, reference cell, reference tissue, control sample, control cell, or control tissue is obtained from a healthy part of the body of an individual who is not the subject or patient in whom a disease or condition is being identified using a composition or method of the invention.

For the purposes herein a "section" of a tissue sample is meant a single part or piece of a tissue sample, *e.g.* a thin slice of tissue or cells cut from a tissue sample. It is understood that multiple sections of tissue samples may be taken and subjected to analysis according to the present invention, provided that it is understood that the present invention comprises a method whereby the same section of tissue sample is analyzed at both morphological and molecular levels, or is analyzed with respect to both protein and nucleic acid.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of gene expression analysis or protocol, one may use the results of the gene expression analysis or protocol to determine whether a specific therapeutic regimen should be performed.

The term "gene signature" is used interchangeably with "gene expression signature" and refers to one or a combination of genes whose expression is indicative of a particular subtype of IPF characterized by certain molecular, pathological, histological, radiographic and/or clinical features. In certain embodiments, the expression of one or more genes comprising the gene signature is elevated compared to that in control subjects.

The term "protein signature" is used interchangeably with "protein expression signature" and refers to one or a combination of proteins whose expression is indicative of a particular subtype of IPF characterized by certain molecular, pathological, histological, radiographic and/or clinical features. In certain embodiments, the expression of one or more proteins comprising the protein signature is elevated compared to that in control subjects.

An "IPF therapeutic agent," a "therapeutic agent effective to treat IPF," and grammatical variations thereof, as used herein, refer to an agent that when provided in an effective amount is known, clinically shown, or expected by clinicians to provide a therapeutic benefit in a subject who has IPF.

A "candidate therapeutic agent" refers to an agent that is being tested or will be tested in a clinical trial under conditions (e.g., a particular dose, dosing regimen, indication) for which the agent has not previously received market approval.

"An anti-IL13/IL4 pathway inhibitor" refers to an agent that blocks the IL-13 and/or IL-4 signalling. Examples of an anti-IL13, anti-IL4 or anti-IL13/IL4 inhibitors include, but are not limited to, anti-IL13 binding agents, anti-IL4 binding agents, anti-IL4receptoralpha binding agents, anti-IL13receptoralpha1 binding agents and anti-IL13 receptoralpha2 binding agents. Single domain antibodies that can bind IL-13, IL-4, IL-13Ralpha1, IL-13Ralpha2 or IL-4Ralpha are specifically included as inhibitors. It should be understood that molecules that can bind more than one target are included.

"Anti-IL4 binding agents" refers to agent that specifically binds to human IL-4. Such binding agents can include a small molecule, an apatmer or a polypeptide. Such polypeptide can include, but is not limited to, a polypeptide(s) selected from the group consisting of an immunoadhesin, an antibody, a peptibody and a peptide. According to one embodiment, the binding agent binds to a human IL-4 sequence with an affinity between 1 uM - 1 pM. Specific examples of anti-IL4 binding agents can include soluble IL4Receptor alpha (e.g., extracellular domain of IL4Receptor fused to a human Fc region), anti-IL4 antibody, and soluble IL13receptoralpha1 (e.g., extracellular domain of IL13receptoralpha1 fused to a human Fc region).

"Anti-IL4receptoralpha binding agents" refers to an agent that specifically binds to human IL4 receptoralpha. Such binding agents can include a small molecule, an aptamer or a polypeptide. Such polypeptide can include, but is not limited to, a polypeptide(s) selected from the group consisting of an immunoadhesin, an antibody, a peptibody and a peptide. According to one embodiment, the binding agent binds to a human IL-4 receptor alpha sequence with an affinity between 1 uM - 1 pM. Specific examples of anti-IL4 receptoralpha binding agents can include anti-IL4 receptor alpha antibodies.

"Anti-IL13 binding agent" refers to agent that specifically binds to human IL-13. Such binding agents can include a small molecule, aptamer or a polypeptide. Such polypeptide can include, but is not limited to, a polypeptide(s) selected from the group consisting of an immunoadhesin, an antibody, a peptibody and a peptide. According to one embodiment, the binding agent binds to a human IL-13 sequence with an affinity between 1 uM - 1 pM. Specific examples of anti-IL13 binding agents can include anti-IL13 antibodies, soluble IL13receptoralpha2 fused to a human Fc, soluble IL4receptoralpha fused to a human Fc, soluble IL13 receptoralpha fused to a human Fc. Exemplary anti-IL13 antibodies are described in Intn'l Pub. No. 2005/062967. Other examples of anti-IL13 antibodies are described in WO2008/083695 (e.g., IMA-638 and IMA-026), US2008/0267959, US2008/0044420 and US2008/0248048.

An exemplary "anti-IL13 antibody," referred to as lebrikizumab, means a humanized IgG4 antibody that binds human IL13. In one embodiment, the anti-IL13 antibody comprises three heavy chain CDRs, CDR-H1 (SEQ ID NO.: 1), CDR-H2 (SEQ ID NO.: 2), and CDR-H3 (SEQ ID NO.: 3). In one embodiment, the anti-IL13 antibody comprises three light chain CDRS, CDR-L1 (SEQ ID NO.: 4), CDR-L2 (SEQ ID NO.: 5), and CDR-L3 (SEQ ID NO.: 6). In one embodiment, the anti-IL13 antibody comprises three heavy chain CDRs and three light chain CDRs, CDR-H1 (SEQ ID NO.: 1), CDR-H2 (SEQ ID NO.: 2), CDR-H3 (SEQ ID NO.: 3), CDR-L1 (SEQ ID NO.: 4), CDR-L2 (SEQ ID NO.: 5), and CDR-L3 (SEQ ID NO.: 6). In one embodiment, the anti-IL13 antibody comprises a variable heavy chain region, VH, having an amino acid sequence selected from SEQ ID NOs. 7 and 8. In one embodiment, the anti-IL13 antibody comprises a variable light chain region, VL, having the amino acid sequence of SEQ ID NO.: 9. In one embodiment, the anti-IL13 antibody comprises a variable heavy chain region, VH, having an amino acid sequence selected from SEQ ID NOs. 7 and 8 and a variable light chain region, VL, having an amino acid sequence of SEQ ID NO.: 9. In one embodiment, the anti-IL13 antibody comprises a heavy chain having the amino acid sequence of SEQ ID NO.: 10 or SEQ ID NO.: 11 or SEQ ID NO.: 12 or SEQ ID NO.: 13. In one embodiment, the anti-IL13 antibody comprises a light chain having the amino acid sequence of SEQ ID NO.: 14. In one embodiment, the anti-IL13 antibody comprises a heavy chain having an amino acid sequence selected from SEQ ID NO.: 10, SEQ ID NO.: 11, SEQ ID NO.: 12, and SEQ ID NO.: 13 and a light chain having the amino acid sequence of SEQ ID NO.: 14.

Anti-IL13receptoralphal binding agents" refers to an agent that specifically binds to human IL13 receptoralpha1. Such binding agents can include a small molecule, aptamer or a polypeptide. Such polypeptide can include, but is not limited to, a polypeptide(s) selected from the group consisting of an immunoadhesin, an antibody, a peptibody and a peptide. According to one embodiment, the binding agent binds to a human IL-13 receptor alpha 1 sequence with an affinity between 1 uM - 1 pM. Specific examples of anti-IL13 receptoralpha1 binding agents can include anti-IL13 receptor alpha1 antibodies.

"Anti-IL13receptoralpha2 binding agents" refers to an agent that specifically binds to human IL13 receptoralpha2. Such binding agents can include a small molecule, an aptamer or a polypeptide. Such polypeptide can include, but is not limited to, a polypeptide(s) selected from the group consisting of an immunoadhesin, an antibody, a peptibody and a peptide. According to one embodiment, the binding agent binds to a human IL-13 receptor alpha2 sequence with an affinity between 1 uM - 1 pM. Specific examples of anti-IL13 receptoralpha2 binding agents can include anti-IL13 receptor alpha2 antibodies.

The term "antibody" is used in the broadest sense and specifically covers, for example, monoclonal antibodies, polyclonal antibodies, antibodies with polyepitopic specificity, single chain antibodies, multi-specific antibodies and fragments of antibodies. Such antibodies can be chimeric, humanized, human and synthetic. Such antibodies and methods of generating them are described in more detail below.

The term "variable" refers to the fact that certain segments of the variable domains differ extensively in sequence among antibodies. The V regions mediate antigen binding and define specificity of a particular antibody for its particular antigen. However, the variability is not evenly distributed across the 110-amino acid span of the variable domains. Instead, the V domains consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a beta-sheet configuration, connected by three hypervariable regions, which form loops connecting, and in some cases forming part of, the beta-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" (or "HVR") when used herein refers to the amino acid residues of an antibody which are responsible for antigen-binding. The hypervariable region generally comprises amino acid residues from a "complementarity determining region" or "CDR" (e.g. around about residues 24-34 (L1), 50-56 (L2) and 89-97 (L3) in the VL, and around about 31-35B (H1), 50-65 (H2) and 95-102 (H3) in the VH (Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (L1), 50-52 (L2) and 91-96 (L3) in the VL, and 26-32 (H1), 52A-55 (H2) and 96-101 (H3) in the VH (Chothia and Lesk J. Mol. Biol. 196:901-917 (1987)).

Hypervariable regions may comprise "extended hypervariable regions" as follows: 24-36 (L1), 46-56 (L2) and 89-97 (L3) in the VL and 26-35B (H1), 47-65 (H2) and 93-102 (H3) in the VH. The variable domain residues are numbered according to Kabat et al., supra for each of these definitions.

"Framework" or "FR" residues are those variable domain residues other than the hypervariable region residues as herein defined. For example, light chain framework 1 (LC-FR1), framework 2 (LC-FR2), framework 3 (LC-FR3) and framework 4 (LC-FR4) region may comprise residues numbered 1-23, 35-49, 57-88 and 98-107 of an antibody (Kabat numbering system), respectively. In another example, heavy chain framework 1 (HC-FR1), heavy chain framework 2 (HC-FR2), heavy chain framework 3 (HC-FR3) and heavy chain framework 4 (HC-FR4) may comprise residues 1-25, 36-48, 66-92 and 103-113, respectively, of an antibody (Kabat numbering system).

As referred to herein, the "consensus sequence" or consensus V domain sequence is an artificial sequence derived from a comparison of the amino acid sequences of known human immunoglobulin variable region sequences.

The term "monoclonal antibody" as used herein refers to an antibody from a population of substantially homogeneous antibodies, *i.e.,* the individual antibodies comprising the population are identical and/or bind the same epitope(s), except for possible variants that may arise during production of the monoclonal antibody, such variants generally being present in minor amounts. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones or recombinant DNA clones. It should be understood that the selected target binding sequence can be further altered, for example, to improve affinity for the target, to humanize the target binding sequence, to improve its production in cell culture, to reduce its immunogenicity *in vivo,* to create a multispecific antibody, *etc.,* and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparationis directed against a single determinant on an antigen. In addition to their specificity, the monoclonal antibody preparations are advantageous in that they are typically uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including the hybridoma method (*e.g.,* Kohler et al., Nature, 256:495 (1975); Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681, (Elsevier, N.Y., 1981), recombinant DNA methods (see, *e.g.,* U.S. Patent No. 4,816,567), phage display technologies (see, *e.g.,* Clackson et al., Nature, 352:624-628 (1991); Marks et al., J. Mol. Biol., 222:581-597 (1991); Sidhu et al., J. Mol. Biol. 338(2):299-310 (2004); Lee et al., J.Mol.Biol.340(5):1073-1093 (2004); Fellouse, Proc. Nat. Acad. Sci. USA 101(34):12467-12472 (2004); and Lee et al. J. Immunol. Methods 284(1-2):119-132 (2004) and technologies for producing human or human-like antibodies from animals that have parts or all of the human immunoglobulin loci or genes encoding human immunoglobulin sequences (see, e.g., WO98/24893, WO/9634096, WO/9633735, and WO/91 10741, Jakobovits et al., Proc. Natl. Acad. Sci. USA, 90:2551 (1993); Jakobovits et al., Nature, 362:255-258 (1993); Bruggemann et al., Year in Immuno., 7:33 (1993); U.S. Patent Nos. 5,545,806, 5,569,825, 5,591,669 (all of GenPharm); 5,545,807; WO 97/17852, U.S. Patent Nos. 5,545,807; 5,545,806; 5,569,825; 5,625,126; 5,633,425; and 5,661,016, and Marks et al., Bio/Technology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); and Lonberg and Huszar, Intern. Rev. Immunol., 13: 65-93 (1995).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while portions of the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Methods of making chimeric antibodies are known in the art.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. In some embodiments, humanized antibodies are human immunoglobulins (recipient antibody) in which residues from a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are generally made to further refine and maximize antibody performance. Typically, the humanized antibody will comprise substantially all of at least one variable domain, in which all or substantially all of the hypervariable loops derived from a non-human immunoglobulin and all or substantially all of the FR regions are derived from a human immunoglobulin sequence although the FR regions may include one or more amino acid substitutions to, e.g., improve binding affinity. In one preferred embodiment, the humanized antibody will also comprise at least a portion of an immunoglobulin constant region (Fc), typically that of a human immunoglobulin or a human consensus constant sequence. For further details, see Jones et al., Nature, 321:522-525 (1986); Reichmann et al., Nature, 332:323-329 (1988); and Presta, Curr. Op. Struct. Biol., 2:593-596 (1992). The humanized antibody includes a PRIMATIZED® antibody wherein the antigen-binding region of the antibody is derived from an antibody produced by, e.g., immunizing macaque monkeys with the antigen of interest. Methods of making humanized antibodies are known in the art.

Human antibodies can also be produced using various techniques known in the art, including phage-display libraries. Hoogenboom and Winter, J. Mol. Biol., 227:381 (1991); Marks et al., J. Mol. Biol., 222:581 (1991). The techniques of Cole et al. and Boerner et al. are also available for the preparation of human monoclonal antibodies. Cole et al., Monoclonal Antibodies and Cancer Therapy, Alan R. Liss, p. 77 (1985); Boerner et al., J. Immunol., 147(1):86-95 (1991). See also, Lonberg and Huszar, Int. Rev. Immunol. 13:65-93 (1995). PCT publications WO 98/24893; WO 92/01047; WO 96/34096; WO 96/33735; European Patent No. 0 598 877; U.S. Pat. Nos. 5,413,923; 5,625,126; 5,633,425; 5,569,825; 5,661,016; 5,545,806; 5,814,318; 5,885,793; 5,916,771; and 5,939,598.

"Antibody fragments" comprise a portion of a full length antibody, generally the antigen binding or variable region thereof. Examples of antibody fragments include Fab, Fab', F(ab')2, and Fv fragments; diabodies; linear antibodies; single-chain antibody molecules; and multispecific antibodies formed from antibody fragments.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) may have the ability to recognize and bind antigen, although at a lower affinity than the entire binding site.

"Functional fragments" of the antibodies of the invention are those fragments that retain binding to polypeptide with substantially the same affinity as the intact full chain molecule from which they are derived and are active in at least one assay (e.g., inhibition of fibrosis such as in mouse models or inhibition of a biological activity of the antigen that binds to the antibody fragment in vitro).

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors (*e.g.* B cell receptor); and B cell activation. A "native sequence Fc region" comprises an amino acid sequence identical to the amino acid sequence of an Fc region found in nature.

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc. and the source code has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, preferably digital UNIX V4.0D. All sequence comparison parameters are set by the ALIGN-2 program and do not vary.

The term "Fc region-comprising polypeptide" refers to a polypeptide, such as an antibody or immunoadhesin (see definitions below), which comprises an Fc region. The C-terminal lysine (residue 447 according to the EU numbering system) of the Fc region may be removed, for example, during purification of the polypeptide or by recombinantly engineering the nucleic acid encoding the polypeptide. Accordingly, a composition comprising polypeptides, including antibodies, having an Fc region according to this invention can comprise polypeptides populations with all K447 residues removed, polypeptide populations with no K447 residues removed or polypeptide populations having a mixture of polypeptides with and without the K447 residue.

Throughout the present specification and claims, the Kabat numbering system is generally used when referring to a residue in the variable domain (approximately, residues 1-107 of the light chain and residues 1-113 of the heavy chain) (e.g, Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., Sequences of Proteins of Immunological Interest, 5th Ed. Public Health Service, National Institutes of Health, Bethesda, MD (1991) expressly incorporated herein by reference). Unless stated otherwise herein, references to residues numbers in the variable domain of antibodies means residue numbering by the Kabat numbering system. Unless stated otherwise herein, references to residue numbers in the constant domain of antibodies means residue numbering by the EU numbering system (e.g., see United States Provisional Application No. 60/640,323, Figures for EU numbering).

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, can be identified by those that: (1) employ low ionic strength and high temperature for washing, for example 0.015 M sodium chloride/0.0015 M sodium citrate/0.1% sodium dodecyl sulfate at 50C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42C; or (3) overnight hybridization in a solution that employs 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42C, with a 10 minute wash at 42C in 0.2 x SSC (sodium chloride/sodium citrate) followed by a 10 minute high-stringency wash consisting of 0.1 x SSC containing EDTA at 55C.

"Moderately stringent conditions" can be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50C. The skilled artisan will recognize how to adjust the temperature, ionic strength, etc. as necessary to accommodate factors such as probe length and the like.

As used herein, a subject to be treated is a mammal (e.g., human, non-human primate, rat, mouse, cow, horse, pig, sheep, goat, dog, cat, etc.). The subject may be a clinical patient, a clinical trial volunteer, an experimental animal, etc. The subject may be suspected of having or at risk for having idiopathic pulmonary fibrosis or be diagnosed with idiopathic pulmonary fibrosis. According to one preferred embodiment, the subject to be treated according to this invention is a human.

"Treating" or "treatment" or "alleviation" refers to measures, wherein the object is to prevent or slow down (lessen) the targeted pathologic condition or disorder or relieve some of the symptoms of the disorder. Those in need of treatment include can include those already with the disorder as well as those prone to have the disorder or those in whom the disorder is to be prevented. A subject or mammal is successfully "treated" for idiopathic pulmonary fibrosis if, after receiving a therapeutic agent of the present invention, the patient shows observable and/or measurable decrease or change from baseline in and/or measurable rate of change from baseline over time (e.g., over 3 months [12 weeks], or 6 months [24 weeks], or 9 months [36 weeks], or 12 months [1 year, 52 weeks]) in one or more of the following: forced vital capacity (FVC), diffusion capacity of the lung for carbon monoxide (DL_{CO}), a patient reported outcome tool, such as A Tool to Assess Quality of Life in IPF (ATAQ-IPF) or EuroQol 5-Dimension Questionnaire (EQ-5D), 6-minute walk distance (6MWD), resting oxygen flow rate, radiographic findings on pulmonary high-resolution computed tomography (HRCT), including quantitative lung fibrosis (QLF) score, serum biomarkers including, but not limited to, periostin, CCL18, YKL40, COMP, OPN, CCL13.

As used herein, "IPF exacerbation" means an event that meets the following criteria: unexplained worsening or development of dyspnea within the previous 30 days; radiologic evidence of new bilateral ground-glass abnormality and/or consolidation, superimposed on a reticular or honeycomb background pattern, that is consistent with usual interstitial pneumonitis (UIP); and absence of alternative causes, such as left heart failure, pulmonary embolism, pulmonary infection (on the basis of endotracheal aspirate or bronchoalveolar lavage, if available, or investigator judgment), or other events leading to acute lung injury (e.g., sepsis, aspiration, trauma, reperfusion pulmonary edema).

As used herein, "IPF deterioration" or "IPF deterioration of disease" means an event that meets the following (i), (ii), and (iii): (i) unexplained worsening or development of dyspnea within the previous 30 days and (ii) any two of: (a) radiologic evidence of new bilateral ground-glass abnormality and/or consolidation superimposed on a reticular or honeycomb background pattern that is consistent with UIP; (b) deterioration of lung function by meeting at least 1 of the following criteria: (I) FVC (L), by at least 10%; (II) DL_{CO} (mL CO /min-1/mmHg-1), at least 15%; (III) oxygen saturation (SpO2) at least 4%; and (iii) absence of alternative causes.

An "effective amount" refers to an amount effective, at dosages and for periods of time necessary, to achieve the desired therapeutic or prophylactic result.

The term "therapeutically effective amount" refers to an amount of a polypeptide of this invention effective to "alleviate" or "treat" a disease or disorder in a subject. A therapeutically effective amount of a therapeutic agent may vary according to factors such as the disease state, age, sex, and weight of the individual, and the ability of the antibody to elicit a desired response in the individual. A therapeutically effective amount is also one in which any toxic or detrimental effects of the therapeutic agent are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Forced expiratory volume (FEV1)" refers to a standard test that measures the volume of air expelled in the first second of a forced expiration. FEV1 is measured by a spirometer, which consists of a mouthpiece and disposable tubing connected to a machine that records the results and displays them on a graph. To perform spirometry, a person inhales deeply, closes the mouth tightly around the tube and then exhales through the tubing while measurements are taken. The volume of air exhaled, and the length of time each breath takes is recorded and analyzed. Spirometry results are expressed as a percentage. Examples of normal spirometry results include a FEV1 of 75 percent of vital capacity after one second. An example of abnormal spirometry results include a reading of less than 80 percent of the normal predicted value. An abnormal result usually indicates the presence of some degree of obstructive lung disease such as asthma, emphysema or chronic bronchitis, or restrictive lung disease such as pulmonary fibrosis. For example, FEV1 values (percentage of predicted) can be used to classify the obstruction that may occur with asthma and other obstructive lung diseases like emphysema or chronic bronchitis: FEV1 65 percent to 79 percent predicted = mild obstruction, FEV1 40 percent to 59 percent predicted = moderate obstruction, and FEV1 less than 40 percent predicted = severe obstruction. In addition, obstructive and restrictive lung disease differ in at least the following way. In obstructive disease, the FEV1/FVC ratio may be lower than normal and the FVC may be normal, while in restrictive disease, the FEV1 and FVC may both be lower than normal but the FEV1/FVC ratio may be normal. In such cases, FEV1 is reduced only because FVC is reduced.

As used herein, "FVC" refers to "Forced Vital Capacity" which refers to a standard test that measures the change in lung air volume between a full inspiration and maximal expiration to residual volume (as opposed to the volume of air expelled in one second as in FEV1). It is a measure of the functional lung capacity. In patients with restrictive lung diseases such as interstitial lung disease including IPF, hypersensitivity pneumonitis, sarcoidosis, and systemic sclerosis, the FVC is reduced typically due to scarring of the lung parenchyma.

Examples of nucleic acid probes that may be used to identify the genes (and proteins encoded by genes) asdescribed herein (e.g., by microarray analysis), include, but are not limited to the probes described in Tables 2, 3, and 4.

"Elevated expression level" or "elevated levels" refers to an increased expression of a mRNA or a protein in a patient (e.g., a patient suspected of having or diagnosed as having IPF) relative to a control, such as an individual or individuals who are not suffering from IPF.

### GENERAL TECHNIQUES

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., eds., 1994).

Primers, oligonucleotides and polynucleotides employed in the present invention can be generated using standard techniques known in the art.

Gene expression signatures associated with IPF and certain subtypes of IPF are provided herein. These signatures constitute biomarkers for IPF and/or subtypes of IPF, and/or predispose or contribute to development, persistence and/or progression of IPF and also are prognostic of survival of IPF patients. Accordingly, the invention disclosed herein is useful in a variety of settings, e.g., in methods and compositions related to IPF prognosis, diagnosis and therapy.

### Detection of Gene Expression Levels

Nucleic acid, according to any of the methods described herein may be RNA transcribed from genomic DNA or cDNA generated from RNA. Nucleic acid may be derived from a vertebrate, e.g., a mammal. A nucleic acid is said to be "derived from" a particular source if it is obtained directly from that source or if it is a copy of a nucleic acid found in that source.

Nucleic acid includes copies of the nucleic acid, e.g., copies that result from amplification. Amplification may be desirable in certain instances, e.g., in order to obtain a desired amount of material for detecting variations. The amplicons may then be subjected to a variation detection method, such as those described below, to determine expression of certain genes.

A microarray is a multiplex technology that typically uses an arrayed series of thousands of nucleic acid probes to hybridize with, e.g, a cDNA or cRNA sample under high-stringency conditions. Probe-target hybridization is typically detected and quantified by detection of fluorophore-, silver-, or chemiluminescence-labeled targets to determine relative abundance of nucleic acid sequences in the target. In typical microarrays, the probes are attached to a solid surface by a covalent bond to a chemical matrix (via epoxy-silane, aminosilane, lysine, polyacrylamide or others). The solid surface is for example, glass, a silicon chip, or microscopic beads. Various microarrays are commercially available, including those manufactured, for example, by Affymetrix, Inc. and Illumina, Inc.

### Detection of Protein Expression Levels

Expression levels of proteins may be detected in samples of whole blood, plasma, or serum. Various methods are known in the art for detecting protein expression levels in such biological samples, including various immunoassay methods. A wide range of immunoassay techniques have been previously described, see, e.g., U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used assays. A number of variations of the sandwich assay technique exist, and all are intended to be encompassed by the present invention. Briefly, in a typical forward assay, an unlabeled antibody is immobilized on a solid substrate, and the sample to be tested brought into contact with the bound molecule. After a suitable period of incubation, for a period of time sufficient to allow formation of an antibody-antigen complex, a second antibody specific to the antigen, labeled with a reporter molecule capable of producing a detectable signal is then added and incubated, allowing time sufficient for the formation of another complex of antibody-antigen-labeled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labeled antibody are added simultaneously to the bound antibody. These techniques are well known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to 40°C such as between 25° C and 32° C inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

An alternative method involves immobilizing the target biomarkers in the sample and then exposing the immobilized target to specific antibody which may or may not be labeled with a reporter molecule. Depending on the amount of target and the strength of the reporter molecule signal, a bound target may be detectable by direct labelling with the antibody. Alternatively, a second labeled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, -galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. As in the EIA, the fluorescent labeled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed.

A biological sample may be obtained using certain methods known to those skilled in the art. Biological samples may be obtained from vertebrate animals, and in particular, mammals. In certain instances, a biological sample is lung tissue, whole blood, plasma, serum or peripheral blood mononuclear cells (PBMC). By screening such body samples, a simple early prognosis (e.g., of survival) or diagnosis (e.g., of a molecular subtype) can be achieved IPF. In addition, the progress of therapy can be monitored more easily by testing such body samples for variations in expression levels of target nucleic acids (or encoded polypeptides).

Subsequent to the determination that a subject, or the tissue or cell sample comprises a gene expression signature disclosed herein, it is contemplated that an effective amount of an appropriate IPF therapeutic agent may be administered to the subject to treat the IPF in the subject. Clinical diagnosis in mammals of the various pathological conditions described herein can be made by the skilled practitioner. Clinical diagnostic techniques are available in the art which allow, e.g., for the diagnosis or detection of IPF in a mammal.

An IPF therapeutic agent can be administered in accordance with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Optionally, administration may be performed through mini-pump infusion using various commercially available devices.

### Certain Therapeutic Agents

Certain therapeutic agents have been previously described as candidates or agents for the treatment of IPF. These have been described in the published literature and are reviewed, for example, in Rafii et al., J. Thorac. Dis (2013) 5(1):48-73. Such agents include agents that have antioxidant, immunosuppressant and/or anti-inflammatory activities such as N-acetylcysteine; agents that have antifibrotic, anti-inflammatory and/or antioxidant activities such as pirfenidone, an orally administered pyridine which has been approved for clinical use in the treatment of IPF; agents that inhibit transforming growth factor-β (TGF-β), such as an anti-TGF-β antibody that targets all TGF-β isoforms (e.g., GC1008), or an antibody against αvβ6 integrin (e.g., STX-100); agents that inhibit connective tissue growth factor (CTGF), such as an anti-CTGF antibody (e.g., FG-3019); agents that inhibit somatostatin receptors, such as somatostatin analogs (e.g, SOM230, octreotide); agents that inhibit IL-13, IL-4 and CCL2, such as an anti-IL13 antibody (e.g., QAX576, tralokinumab, lebrikizumab [described further below]), an anti-IL4 antibody, a combination anti-IL13/anti-IL4 agent (e.g., a bispecific anti-IL13/anti-IL4 antibody such as SAR156597), an anti-CCL2 antibody (e.g., CNTO888); agents that have anti-angiogenic, immunomodulatory, and/or anti-inflammatory activities such as thalidomide or minocycline; agents that inhibit the enzyme lysyl oxidase-like 2 (LOXL2), such as an anti-LOXL2 antibody (e.g., GS-6624 [simtuzumab]); agents that inhibit angiogenesis such as the tyrosine kinase inhibitor, BIBF 1120, tetrathiomolybdate; agents that inhibit deposition of extracellular matrix and/or disrupt collagen deposition, such as doxycycline; agents that target the renin-angiotensin system such as losartan; and other agents having anti-proliferative and/or anti-fibrotic activities such as carbon monoxide.

A certain therapeutic agent for the treatment of idiopathic pulmonary fibrosis is provided herein. In one embodiment, therapeutic agent is an anti-IL13 antibody, also referred to as lebrikizumab. Lebrikizumab as an IgG4 antibody. In one embodiment, the anti-IL13 antibody comprises three heavy chain CDRs, CDR-H1 (SEQ ID NO.: 1), CDR-H2 (SEQ ID NO.: 2), and CDR-H3 (SEQ ID NO.: 3). In one embodiment, the anti-IL13 antibody comprises three light chain CDRS, CDR-L1 (SEQ ID NO.: 4), CDR-L2 (SEQ ID NO.: 5), and CDR-L3 (SEQ ID NO.: 6). In one embodiment, the anti-IL13 antibody comprises three heavy chain CDRs and three light chain CDRs, CDR-H1 (SEQ ID NO.: 1), CDR-H2 (SEQ ID NO.: 2), CDR-H3 (SEQ ID NO.: 3), CDR-L1 (SEQ ID NO.: 4), CDR-L2 (SEQ ID NO.: 5), and CDR-L3 (SEQ ID NO.: 6). In one embodiment, the anti-IL13 antibody comprises a variable heavy chain region, VH, having an amino acid sequence selected from SEQ ID NOs. 7 and 8. In one embodiment, the anti-IL13 antibody comprises a variable light chain region, VL, having the amino acid sequence of SEQ ID NO.: 9. In one embodiment, the anti-IL13 antibody comprises a variable heavy chain region, VH, having an amino acid sequence selected from SEQ ID NOs. 7 and 8 and a variable light chain region, VL, having an amino acid sequence of SEQ ID NO.: 9. In one embodiment, the anti-IL13 antibody comprises a heavy chain having the amino acid sequence of SEQ ID NO.: 10 or SEQ ID NO.: 11 or SEQ ID NO.: 12 or SEQ ID NO.: 13. In one embodiment, the anti-IL13 antibody comprises a light chain having the amino acid sequence of SEQ ID NO.: 14. In one embodiment, the anti-IL13 antibody comprises a heavy chain having an amino acid sequence selected from SEQ ID NO.: 10, SEQ ID NO.: 11, SEQ ID NO.: 12, and SEQ ID NO.: 13 and a light chain having the amino acid sequence of SEQ ID NO.: 14. Anti-IL13 antibodies are further described in Intn'l Pub. No. 2005/062967.

In another aspect, an anti-IL-13 antibody comprises a heavy chain variable domain (VH) sequence having at least 90%, 91%, 92%, 93%, 94%, 95%, 96%, 97%, 98%, 99%, or 100% sequence identity to the amino acid sequence of SEQ ID NO.: 8. In certain embodiments, a VH sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-IL-13 antibody comprising that sequence retains the ability to bind to human IL-13. In certain embodiments, a total of 1 to 10 amino acids have been substituted, altered inserted and/or deleted in SEQ ID NO.: 8. In certain embodiments, substitutions, insertions, or deletions occur in regions outside the CDRs (i.e., in the FRs). Optionally, the anti-IL13 antibody comprises the VH sequence in SEQ ID NO.: 8, including post-translational modifications of that sequence.

In another aspect, an anti-IL-13 antibody is provided, wherein the antibody comprises a light chain variable domain (VL) having at least 90%, 91%, 92%, 93%, 94%, 95%, 96%, 97%, 98%, 99%, or 100% sequence identity to the amino acid sequence of SEQ ID NO.: 9. In certain embodiments, a VL sequence having at least 90%, 91%, 92%, 93%, 94%, 95%, 96%, 97%, 98%, or 99% identity contains substitutions (e.g., conservative substitutions), insertions, or deletions relative to the reference sequence, but an anti-IL-13 antibody comprising that sequence retains the ability to bind to IL-13. In certain embodiments, a total of 1 to 10 amino acids have been substituted, inserted and/or deleted in SEQ ID NO.: 9. In certain embodiments, the substitutions, insertions, or deletions occur in regions outside the CDRs (i.e., in the FRs). Optionally, the anti-IL-13 antibody comprises the VL sequence in SEQ ID NO.: 9, including post-translational modifications of that sequence.

In yet another embodiment, the anti-IL-13 antibody comprises a VL region having at least 90%, 91%, 92%, 93%, 94%, 95%, 96%, 97%, 98%, 99%, or 100% sequence identity to the amino acid sequence of SEQ ID NO.: 9 and a VH region having at least 90%, 91%, 92%, 93%, 94%, 95%, 96%, 97%, 98%, 99%, or 100% sequence identity to the amino acid sequence of SEQ ID NO.: 8.

### Kits

For use in the applications described or suggested herein, kits or articles of manufacture are also provided. Such kits may comprise a carrier means being compartmentalized to receive in close confinement one or more container means such as vials, tubes, and the like, each of the container means comprising one of the separate elements to be used in the method. For example, one of the container means may comprise a probe that is or can be detectably labeled. Such probe may be a polynucleotide specific for a polynucleotide comprising one or more genes of a gene expression signature. Where the kit utilizes nucleic acid hybridization to detect the target nucleic acid, the kit may also have containers containing nucleotide(s) for amplification of the target nucleic acid sequence and/or a container comprising a reporter means, such as a biotin-binding protein, such as avidin or streptavidin, bound to a reporter molecule, such as an enzymatic, florescent, or radioisotope label.

Kits will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific therapy or non-therapeutic application, and may also indicate directions for either *in vivo* or *in vitro* use, such as those described above. Other optional components in the kit include one or more buffers (*e.g.,* block buffer, wash buffer, substrate buffer, etc), other reagents such as substrate (*e.g*., chromogen) which is chemically altered by an enzymatic label, epitope retrieval solution, control samples (positive and/or negative controls), control slide(s) etc.

### Methods of Marketing

The invention herein also encompasses a method for marketing an IPF therapeutic agent or a pharmaceutically acceptable composition thereof comprising promoting to, instructing, and/or specifying to a target audience, the use of the agent or pharmaceutical composition thereof for treating a patient or patient population with IPF from which a sample has been obtained showing the expression levels of a gene or protein or combination of genes and proteins as disclosed herein.

Marketing is generally paid communication through a non-personal medium in which the sponsor is identified and the message is controlled. Marketing for purposes herein includes publicity, public relations, product placement, sponsorship, underwriting, and sales promotion. This term also includes sponsored informational public notices appearing in any of the print communications media designed to appeal to a mass audience to persuade, inform, promote, motivate, or otherwise modify behavior toward a favorable pattern of purchasing, supporting, or approving the invention herein.

The marketing of the diagnostic method herein may be accomplished by any means. Examples of marketing media used to deliver these messages include television, radio, movies, magazines, newspapers, the internet, and billboards, including commercials, which are messages appearing in the broadcast media.

The type of marketing used will depend on many factors, for example, on the nature of the target audience to be reached, *e.g*., hospitals, insurance companies, clinics, doctors, nurses, and patients, as well as cost considerations and the relevant jurisdictional laws and regulations governing marketing of medicaments and diagnostics. The marketing may be individualized or customized based on user characterizations defined by service interaction and/or other data such as user demographics and geographical location.

All publications (including patents and patent applications) cited herein are hereby incorporated in their entirety by reference.

Throughout this specification and claims, the word "comprise," or variations such as "comprises" or "comprising," will be understood to imply the inclusion of a stated integer or group of integers but not the exclusion of any other integer or group of integers.

The foregoing written description is considered to be sufficient to enable one skilled in the art to practice the invention. The following Examples are offered for illustrative purposes only, and are not intended to limit the scope of the present invention in any way. Indeed, various modifications of the invention in addition to those shown and described herein will become apparent to those skilled in the art from the foregoing description and fall within the scope of the appended claims.

All references cited herein, including patent applications and publications, are incorporated by reference in their entirety for any purpose.

### EXAMPLES

### Example 1: Identification of Systemic Biomarkers for Survival Prognosis in IPF

To identify molecular biomarkers that could be useful for prognosing IPF survival, we first conducted a gene expression analysis using microarray and qPCR methods in lung tissue from 40 IPF patients and 8 unused donor controls ("Cohort 1"). From the gene expression results, we then identified candidate prognostic serum biomarkers. The serum levels of each of the candidate prognostic serum biomarkers and lung function were assessed in a separate cohort of 80 IPF patients ("Cohort 2") collected at the time of presentation to the interstitial lung disease clinic at the University of California, San Francisco. Vital status was followed for 2-8 years after sample collection.

### METHODS

### Human Lung Tissue

Tissues were harvested in the University of California, San Francisco Lung Center from IPF patients at the time of biopsy or lung transplant. Non-IPF controls were harvested from donor lungs. Further details are provided in the Results section.

### Tissue Culture

IMR-90 cells (ATCC, Manassas, VA; Catalog # CCL-186) were cultured in DMEM medium supplemented with 10% FBS (Sigma, St. Louis, MO; Catalog # F2442) and Penicillin/Streptomycin (Invitrogen, Carlsbad, CA; Catalog # 15140). Cells were plated on a bed of growth factor reduced Matrigel™ (GFR Matrigel™; BD Biosciences, Bedford, MA; Catalog #354230). Matrigel™ was thawed overnight on ice and a bed volume of 450ul/well in a 12-well plate was produced by allowing Matrigel™ to harden at 37°C for 30 minutes. Cells (1E5-2E5) were then plated onto Matrigel™ bed. Unless otherwise indicated, IL-13 stimulation was performed with 10 ng/ml (IL-13 and TNFα) and 3 ng/ml (IL-4).

### RNA Preparation

Snap frozen lung biopsy samples were pulverized in a pre-cooled (in liquid nitrogen) Bessman tissue pulverizer (Spectrum Laboratories, Rancho Dominguez, CA; catalog #189475). Trizol® was added to pulverized material and pipetted several times. Lysates were incubated on ice for 10-15 minutes. Lysates were stored at -80°C until further processing. RNA was isolated from Trizol® lysates according to the manufacturer's protocol. Trizol®-isolated RNA was then subjected to another purification step using the Qiagen RNeasy columns according to the manufacturer's protocol.

Tissue cultured cells were harvested by adding 1ml (per well in 12-well plate) Trizol® and pipetting mixture several times. Trizol® lysates were homogenized using the steel bead/Tissuelyser (Qiagen) method (20 s⁻¹, 4 minutes) as indicated in the Qiagen RNeasy protocol. Upon homogenization, RNA was isolated following the Trizol® manufacturer's protocol. Trizol®-isolated RNA was then subjected to another purification step using the Qiagen RNeasy column according to the manufacturer's protocol.

RNA concentrations were determined by spectrophotometry (Nanodrop, Thermo Scientific) and all microarray samples were analyzed by Bioanalyzer (Agilent).

### RT-qPCR

100-300ng of total RNA was subjected to reverse transcription using random primers with the High Capacity cDNA Reverse Transcription Kit (ABI, Catalog # 4368814) according to the manufacturer's protocol. Real-time qPCR was performed using the Taqman assays. All reactions were run on either the ABI 7900HT instrument or the Fluidigm platform (48.48 or 96.96 format).

### IL-13 Reporter Assay

L-Luc-BEAS-2B (cell line constructed by Genentech; derived from BEAS-2B, ATCC, Manassas, VA; cat. no. CRL-9609) were grown in GFR Matrigel™ embedded with the following reagents as indicated: 12ng/ml IL-13 (R&D Systems, Catalog #213-IL), and an anti-IL-13 blocking antibody (Genentech). Firefly Luciferase activity was measure using the Dual-Glo Luciferase Assay System (Promega, catalog #E2920) according the manufacturer's protocol, with the modification that only Firefly luciferase was measured.

### Microarray Analysis

RNA was amplified and labeled using the Quick Amp labeling kit (Agilent Technologies, Cat. No. 5190-0444) to generate labeled cRNA from 1ug of total RNA. Experimental samples were labeled with Cy5; Universal Human Reference RNA (Stratagene, La Jolla, CA) was used for the reference channel and was labeled with Cy3. Cy5 and Cy3 labeled cRNA was competitively hybridized to the two-color Whole Human Genome 4 × 44K gene expression microarray platform (Agilent Technologies, Cat. No. G4112F). Hybridized microarrays were washed according to the manufacturer's protocol (Agilent) and all feature intensities were collected using the Agilent Microarray Scanner. TIFF images of scanned slides were analyzed using Feature Extraction Software version 7.5 (Agilent), protocol GE2-v5_95 (Agilent). Flagged outliers were not included in any subsequent analyses. All data are reported as log₂ values of the dye-normalized Cy5/Cy3 ratios. The log2 ratios of all samples were normalized to the average log2 ratios of the corresponding mock-treated samples (or non-IPF controls).

When different expression profiling datasets were compared, publicly available datasets were first filtered, normalized and centered as in the original study. The Kaminski dataset (GSE10667) (Konishi et al., Am. J. Respir. Crit. Care Med. 180(2):167-75 (2009)) was run on whole human genome Agilent microarray platform, therefore, platform specific probe identifiers were used to connect the datasets. Heatmaps were generated with Java Treeview.

Differentially expressed genes were identified by building a linear model that incorporated three factors: diagnosis, source of tissue and gender (inferred from expression analysis of Y-linked genes) in R (LIMMA).

### Blood Biomarker Assays

Levels of the following markers were assessed in serum using commercially available assays according to manufacturers' instructions: COMP (Abnova, Taipei, Taiwan, catalog #KA0021); MMP7 (R&D Systems, Minneapolis, MN, catalog #DMP700); CXCL13 (R&D Systems, Minneapolis, MN, catalog #DCX130); CCL13 (R&D Systems, Minneapolis, MN, catalog #DY327); YKL-40 (R&D Systems, Minneapolis, MN, catalog #DY2599); MMP3 (Meso Scale Discovery, Gaithersburg, MD, catalog #K15034C); SAA (Meso Scale Discovery, Gaithersburg, MD, catalog #K151EOC-1); CCL11 (eotaxin) and CCL17 (TARC) (Meso Scale Discovery, Gaithersburg, MD, catalog #15031C). Levels of OPN were assessed in plasma using a commercially available assay (R&D Systems, catalog #DOST00) according to the manufacturer's instructions. Serum periostin was analyzed using a proprietary assay as previously described (see, e.g., Intn'l Patent App. No PCT/US2011/065410). A commercially available assay for serum CCL18 was used (R&D Systems, catalog #DY394) with modifications to the manufacturer's protocol to ameliorate matrix interference as follows: 96-well plates were coated overnight at 4°C with mouse anti-human CCL18 monoclonal antibodies and were then blocked with a buffer containing 1X PBS pH 7.4, 0.5% BSA, 0.05% Tween 20, 0.25% CHAPS, 5mM EDTA, 0.35M NaCl, and 15PPM Proclin. Serum samples diluted 1:1000 with assay diluent containing 5% FBS were added in duplicate, and the plates were incubated for 2 hours at room temperature (20°C). After washing, biotinylated goat anti-human CCL18 monoclonal antibodies in assay diluent containing 5% goat serum were added to the plates and incubated for 1 hour at room temperature (20°C). Color was developed after washing with streptavidin-peroxidase and substrate TMB. The detection limit of this assay was ∼7.8 pg/ml.

### RESULTS

### Characteristics of Study Cohorts

For samples obtained from Cohort 1, biopsy tissue from 40 IPF patients and 8 controls was used in microarray and qPCR studies. Eleven of the IPF samples were obtained from thoracoscopic biopsies and 29 samples were obtained from explants taken at the time of lung transplantation. All of the control tissue was explanted from unused donor lungs. Donor lungs were generally taken from subjects with no evidence of interstitial lung disease that died of nonpulmonary causes (e.g. trauma) but were not able to be used for transplantation for reasons such as excessive time since death or unavailability of a suitable recipient due to blood type, vascular, or HLA mismatch. Adjacent tissue from all of the IPF biopsy specimens was confirmed to have a usual interstitial pneumonia (UIP) pattern. A replication dataset available for public download consisting of 31 IPF and 15 control samples has been previously described (Konishi et al., Am. J. Respir. Crit. Care Med. 180(2):167-75 (2009)).

For samples obtained from Cohort 2, both serum and plasma were collected from each of 80 IPF patients at the time of presentation to the interstitial lung disease clinic at the University of California, San Francisco. Clinical and demographic characteristics of this cohort are described in Table 1 below. Serum (N=29) or plasma (N=10) from healthy controls was used to establish a normal range for biomarker levels.

**Table 1. Clinical and Demographic Characteristics of Cohort 2.**

| **Variable** | **Median (IQR) Unless Otherwise Indicated** |
|---|---|
| Sex (M:F) | 62:16 |
| Age at initial visit, median (range) | 70 (50-87) |
| Subsequent lung transplant (Y:N) | 7:63 |
| Ever a smoker (Y:N) | 58:16 |
| Current smoker (Y:N) | 1:73 |
| Systemic steroid ± azathioprine (Y:N) | 21:59 |
| FVC % predicted | 69 (57-81) |
| TLC % predicted | 70 (59-78) |
| DLco % predicted | 48 (36-60) |
| Dyspnea score¹ | 10 (5.5-16) |
| Radiographic score² | 12 (7.7-20.4) |

| | |
|---|---|
| FVC = Forced Vital Capacity; TLC = Total Lung Capacity; DL_{CO} = diffusing capacity (measured with carbon monoxide); IQR = inter-quartile range, 25th-75th percentile; ¹ = Dyspnea score on a scale of 1-20 according to Watters et al., Ann. Rev. Resp. Dis. 133:97 (1986); ² = Radiographic score estimates of percent of lung that is fibrotic according to the methods described in Best et al., Radiology 246:935 (2008). | |

### Identification of Differentially Expressed Genes in IPF

We performed a genome wide transcriptome analysis of RNA isolated from lung tissue from Cohort 1 (40 IPF patients and 8 unused donor control subjects) using Whole Human Genome microarrays (Agilent Technologies, Cat. No. G4112F). Limited available metadata (sex, tissue source (biopsy or tissue harvested at time of lung transplant), diagnosis) were included in a linear model used to identify differentially expressed (DE) genes between IPF patients and controls. We identified 2940 microarray probes as differentially expressed (q<0.05, fold-change > 1.5) in IPF tissues compared to control tissues (a partial list is shown in Table 2). To reduce the possibility that the overall IPF expression profiles observed here were affected by systematic biases incurred by sample collection methods or other confounding factors, we compared expression profiles to a similar, but independently generated IPF dataset (Konishi et al., Am. J. Respir. Crit. Care Med. 180(2):167-75 (2009)). This dataset (GSE10667) described expression profiles of lung biopsies from 31 IPF patients and 15 control lung tissues, which were harvested from surrounding, non-malignant regions after lung tumor resection. We calculated T-statistics describing the deviation of expression level of IPF samples compared to the control samples for each gene. When plotted against each other, we found a high degree of similarity (data not shown). Because the two datasets were independently generated, the similar t-statistics support the validity of each study and reduce the likelihood that transcriptome-wide effects were due to study-specific factors or technical artifacts.

### Bronchiolar, lymphoid, and fibroblast/myofibroblast gene expression signatures in IPF

Unsupervised 2-way hierarchical clustering of the 2490 DE microarray probes between IPF and controls (as discussed above) demonstrated three major clusters defined primarily by diagnosis (e.g., IPF or control) as shown in Figure 1A; Group 1 Cluster, Group 2 Cluster, and Group 3 Cluster. We re-clustered Group 1 (heatmap shown in Figure 1B), Group 2 (heatmap shown in Figure 1C), and Group 3 (heatmap shown in Figure 1D). Within the heatmaps shown in Figs.1B-D, we observed several groups of co-regulated genes that appeared to be heterogeneous within the IPF patients. Three of these groups of co-regulated genes were enriched for genes expressed in bronchial epithelium (see Table 2; also referred to as the Group 1 cluster), lymphoid aggregates, also referred to as follicles (see Table 3, also referred to as Group 2 cluster), and myofibroblasts (see Table 4, also referred to as Group 3 cluster), respectively.

The Group 1 cluster or bronchiolar group contained mucins (MUCL1, MUC4, MUC20); proline-rich secreted factors (PRR7, PRR15, SPRR1B, SPRR2D); keratins (KRT5, KRT6B, KRT13, KRT14, KRT15, KRT17), serine protease inhibitors (SERPINB3, SERPINB4, SERPINB5, SERPINB13), ion channels and associated factors (CLCA2, TRPV4), and cilium components (BBS5) as well as MMP3 and SAA4. These genes are typical of differentiated bronchial epithelium rather than the alveolar epithelium that might be expected in normal lung parenchyma and the expression of such genes is consistent with prior reports of abnormal "bronchiolization" of alveolar spaces in IPF, which is likely to represent epithelialization of honeycombed cystic spaces related to profound scarring (Chilosi et al., Lab Invest. 82(10): 1335-45 (2002)). The bronchiolar signature is strikingly similar to a gene signature reported to be upregulated in lung tissue from patients with concomitant pulmonary fibrosis and pulmonary artery hypertension, including TP63, CLCA2, FGF14, PTPRZ1, SOX2, DSC3, CP, MMP1, MUC4, SERPINB3, SERPINB13, and multiple keratins (Mura et al., Chest 141:661-673 (2012)). Hence it is likely that the decreased tissue compliance and impaired gas exchange associated with scarring, honeycombing, and bronchiolization contributes to increased pulmonary vascular resistance and subsequent pulmonary hypertension. We also noted that certain genes were down-regulated within this cluster and these included Notch4, cadherins, Wnt7A, and DKK2.

**Table 2. Partial list of differentially expressed genes (> 1.5-fold up-regulated, q<0.05) associated with a bronchiolar gene expression signature.**

| **ProbeI**D | **Gene Symbol** | **Gene Name** | **Entrez ID** | L**ogFC** | **p-value** | **adj. p-value** |
|---|---|---|---|---|---|---|
| A_23_P150979 | MUCL1 | mucin-like 1 | 118430 | 1.61921533 | 0.00665823 | 2.60E-02 |
| A_24_P208825 | MUC4 | mucin4, cell surface associated | 4585 | 2.11873294 | 2.50E-05 | 2.72 E-04 |
| A_23_P92222 | MUC20 | mucin 20, cell surface associated | 200958 | 1.23642525 | 0.00022861 | 1.70E-03 |
| A_23_P30464 | PRR7 | proline rich 7 (synaptic) | 80758 | 1.04150995 | 3.35E-06 | 5.34E-05 |
| A_32_P154911 | PRR15 | proline rich 15 | 222171 | 1.58791565 | 0.00882268 | 3.23E-02 |
| A_23_P159406 | SPRR1B | small proline-rich protein 1B | 6699 | 1.76462785 | 5.26E-05 | 5.02E-04 |
| A_23_P11644 | SPRR2D | small proline-rich protein 2D | 6703 | 1.15788562 | 0.00025497 | 1.86E-03 |
| A_23_P218047 | KRT5 | keratin 5 | 3852 | 2.52682152 | 5.44E-06 | 7.87E-05 |
| A_23_P76249 | KRT6B | keratin 6B | 3854 | 2.90550487 | 9.76E-05 | 8.39E-04 |
| A_24_P228149 | KRT13 | keratin 13 | 3860 | 1.22487349 | 0.00497946 | 2.06E-02 |
| A_24_P265346 | KRT14 | keratin 14 | 3861 | 3.71369769 | 1.31E-10 | 1.53E-08 |
| A_23_P27133 | KRT15 | keratin 15 | 3866 | 1.8933655 | 0.00097625 | 5.56E-03 |
| A_23_P96158 | KRT17 | keratin 17 | 3872 | 4.14444061 | 1.20E-11 | 2.15E-09 |
| A_23_P55632 | SERPINB3 | serpin | 6317 | 3.73336698 | 0.00023029 | 1.71E-03 |
| | | peptida se inhibitor, clade B (ovalbumin), member 3 | | | | |
| A_23_P502413 | SERPINB4 | serpin peptidase inhibitor, clade B (ovalbumin), member 4 | 6318 | 3.54380018 | 0.00032106 | 2.25E-03 |
| A_23_P208126 | SERPINB5 | serpin peptidase inhibitor, clade B (ovalbumin), member 5 | 5268 | 2.18225921 | 9.31E-05 | 8.03E-04 |
| A_23_P432978 | SERPINB13 | serpin peptidase inhibitor, clade B (ovalbumin), member 13 | 5275 | 1.33876843 | 0.01364005 | 4.56E-02 |
| A_23_P397248 | CLCA2 | chloride channel accessory 2 | 9635 | 2.34309417 | 7.25E-05 | 6.52E-04 |
| A_24_P13381 | TRPV4 | transient receptor potential cation channel, subfamily V, member 4 | 59341 | 1.144662 | 0.00052144 | 3.34E-03 |
| A_23_P5785 | BBS5 | Bardet-Biedl syndrome 5 | 129880 | 0.60446077 | 0.00254271 | 1.20E-02 |
| A_23_P161698 | MMP3 | matrix metallopeptidas e 3 (stromelysin 1, progelatinase) | 4314 | 2.33843742 | 3.32E-06 | 2.09E-05 |
| A_23_P87238 | SAA4 | serum amyloid A4, constitutive | 6291 | 1.73735951 | 0.01243724 | 0.0131173 |

| | | | | | | |
|---|---|---|---|---|---|---|
| ProbeID = the identification number of the probe on the Agilent Whole Human Genome Microarray; Gene Symbol = NCBI Entrez Gene Symbol; Gene Name = NCBI Entrez Gene Name; ENTREZ ID = NCBI Entrez Gene ID; logFC = mean of the log base 2 expression levels in IPF samples minus the mean of the log base 2 expression levels in healthy control samples; p-value = calculated using t-test for differential expression between IPF and control per probe; adj. p-value = adjusted for multiple testing using Bonferroni's method. | | | | | | |

A second cluster of highly co-regulated genes that exhibited heterogeneity in IPF (Group 2 cluster; Fig. 1A) included genes related to lymphoid follicles (also referred to as aggregates). The following groups of genes were up-regulated in the Group 2 cluster in IPF patients: B cell-specific genes (CD19, CD20 [MS4A1], BCMA [TNFRSF17], BLK, and BLNK); multiple immunoglobulin genes and CD79A and CD79B; T cell and APC genes (CD27, CD28, CD1); Fc receptor genes (FCRLA, FCRL2, FCRL5); and chemokines and their receptors (CXCL13, CXCR3, CXCR5, CCR6, CCR7). This pattern of gene expression is consistent with prior reports showing increased numbers of lymphocytes in regions of active fibrosis in IPF biopsies (Nuovo et al., Mod. Pathol. (2011) 1-18). We selected this group of highly co-regulated genes and re-clustered the dataset restricted to the expression patterns of these "lymphoid" genes (Figure 1C). This analysis yielded three major subsets characterized by low, medium, or high coordinate expression of lymphoid genes. The low expressing group comprised all the controls and a few IPF subjects, while the medium and high expressing groups comprised the remaining IPF subjects.

**Table 3. Partial list of differentially expressed genes (> 1.5-fold up-regulated, q<0.05) associated with a lymphoid follicle gene expression signature.**

| **ProbeID** | **Gene Symbol** | **Gene Name** | **Entrez ID** | L**ogFC** | **p-value** | **adj. p-value** |
|---|---|---|---|---|---|---|
| A_23_P114299 | CXCR3 | chemokine (C-X-C motif) receptor 3 | 2833 | 1.46371763 | 6.45E-09 | 3.29E-07 |
| A_24_P252945 | CXCR5 | chemokine (C-X-C motif) receptor 5 | 643 | 2.37673236 | 2.81E-05 | 2.99E-04 |
| A_23_P121695 | CXCL13 | chemokine (C-X-C motif) ligand 13 | 10563 | 3.4378017 | 0.00012013 | 9.96E-04 |
| A_24_P234921 | CCR6 | chemokine (C-C motif) receptor 6 | 1235 | 2.56106982 | 1.49E-10 | 1.65E-08 |
| A_23_P343398 | CCR7 | chemokine (C-C motif) receptor 7 | 1236 | 3.29053652 | 2.33E-12 | 5.59E-10 |
| A_23_P113572 | CD19 | CD19 molecule | 930 | 2.01576251 | 3.16E-05 | 3.30E-04 |
| A_23_P116371 | MS4A1 (CD20) | membrane-spanning 4-domains, subfamily A, member 1 | 931 | 2.39890071 | 1.31E-05 | 2.86E-05 |
| A_23_P37736 | TNFRSF17 (BCMA) | tumor necrosis factor receptor superfamily, member 17 | 608 | 2.44042243 | 2.62E-05 | 5.09E-05 |
| A_23_P31725 | BLK | B lymphoid tyrosine kinase | 640 | 1.58760468 | 0.0035697 | 1.58E-02 |
| A_24_P64344 | BLNK | B-cell linker | 29760 | 0.90832362 | 0.00025269 | 1.84E-03 |
| A_23_P46039 | FCRLA | Fc receptor-like A | 84824 | 1.64791428 | 0.00036031 | 2.47E-03 |
| A_23_P160751 | FCRL2 | Fc receptor-like 2 | 79368 | 1.44435616 | 0.00408379 | 1.75E-02 |
| A_23_P201211 | FCRL5 | Fc receptor-like 5 | 83416 | 2.47293587 | 2.31E-06 | 3.96E-05 |
| A_23_P107735 | CD79A | CD79a molecule, immunoglobulin-associated alpha | 973 | 1.45014719 | 0.0006872 | 4.19E-03 |
| A_23_P207201 | CD79B | CD79b molecule, immunoglobulin-associated beta | 974 | 1.13800823 | 0.00031683 | 2.22E-03 |
| A_23_P48088 | CD27 | CD27 molecule | 939 | 1.4445514 | 1.87E-06 | 3.38E-05 |
| A_23_P91095 | CD28 | CD28 molecule | 940 | 3.18508221 | 1.28E-13 | 5.59E-11 |
| A_23_P63167 | CD1A | CD1a molecule | 909 | 2.71941185 | 1.34E-08 | 6.07E-07 |
| A_23_P351844 | CD1B | CD1b molecule | 910 | 1.13359253 | 0.00323118 | 1.45E-02 |
| A_23_P51767 | CD1C | CD1c molecule | 911 | 2.00435627 | 4.79E-09 | 2.57E-07 |
| A_23_P201160 | CD1E | CD1e molecule | 913 | 1.75899432 | 0.00031177 | 2.20E-03 |
| A_24_P367432 | IGHV1-69 | immunoglobulin heavy variable 1-69 | 28461 | 3.31557265 | 6.44E-10 | 5.39E-08 |
| A_24_P605563 | IGLJ3 | immunoglobulin lambda joining 3 | 28831 | 3.1763507 | 2.66E-08 | 1.07E-06 |
| A_23_P167168 | IGJ | immunoglobulin J polypeptide, linker protein for immunoglobulin alpha and mu polypeptides | 3512 | 2.99945067 | 2.33E-06 | 3.98E-05 |
| A_24_P204727 | IGHV3-48 | immunoglobulin heavy variable 3-48 | 28424 | 2.68775596 | 1.20E-07 | 3.66E-06 |
| A_24_P465799 | IGLV3-21 | immunoglobulin lambda variable 3-21 | 28796 | 2.40390705 | 5.16E-08 | 1.87E-06 |
| A_32_P722809 | IGKV1-5 | immunoglobulin kappa variable 1-5 | 28299 | 2.25836578 | 1.69E-05 | 1.97E-04 |
| A_23_P390209 | IGHG1 | immunoglobulin heavy constant gamma 1 (G1m marker) | 3500 | 2.23089237 | 0.00120748 | 6.60E-03 |
| A_24_P263786 | IGKC | immunoglobulin kappa constant | 3514 | 2.17465687 | 0.00103502 | 5.84E-03 |
| A_24_P361816 | IGLV6-57 | immunoglobulin lambda variable 6-57 | 28778 | 2.15352717 | 1.79E-06 | 3.25E-05 |
| A_24_P677559 | IGK@ | immunoglobulin kappa locus | 50802 | 2.02637845 | 8.18E-08 | 2.70E-06 |
| A_24_P395415 | IGHA1 | immunoglobulin heavy constant alpha 1 | 3493 | 1.93646716 | 0.00186451 | 9.26E-03 |
| A_24_P306905 | IGKV2-24 | immunoglobulin kappa variable 2-24 | 28923 | 1.89765893 | 4.50E-06 | 6.74E-05 |
| A_24_P93523 | IGKV1D-8 | immunoglobulin kappa variable 1D-8 | 28904 | 1.82178807 | 4.23E-05 | 4.21E-04 |
| A_24_P417352 | IGHM | immunoglobulin | 3507 | 0.71477012 | 0.00015195 | 1.21E-03 |
| | | heavy constant mu | | | | |

| | | | | | | |
|---|---|---|---|---|---|---|
| ProbeID = the identification number of the probe on the Agilent Whole Human Genome Microarray; Gene Symbol = NCBI Entrez Gene Symbol; Gene Name = NCBI Entrez Gene Name; ENTREZ ID = NCBI Entrez Gene ID; logFC = mean of the log base 2 expression levels in IPF samples minus the mean of the log base 2 expression levels in healthy control samples; p-value = calculated using t-test for differential expression between IPF and control per probe; adj. p-value = adjusted for multiple testing using Bonferroni's method. | | | | | | |

A third cluster of highly co-regulated genes that exhibited heterogeneity in IPF included genes related to fibroblast differentiation into myofibroblasts and wound healing. This cluster included multiple genes encoding collagens (COL1A1, COL1A2, COL5A2, COL12A1, COL14A1, COL15A1, COL16A1, COL18A1, CTHRC1); growth factors (HGF, IGFBP7, SCGF); lysyl oxidases (LOXL1, LOXL2); mediators of hedgehog signaling (GLI1, GLI2, SMO); mediators of Wnt signaling (SFRP2, DIO2), CDH11, periostin, and TGFB3. This pattern is consistent with prior reports showing elevated expression of many of these mediators associated with myofibroblasts and/or fibroblastic foci in IPF (Elliott et al., J. Cell Science 125:121-132 (2012); Barry-Hamilton et al., Nature Med. 16:1009-1017 (2010); Schneider et al., FASEB 26:503-512 (2012); Okamoto et al., The European respiratory journal : official journal of the European Society for Clinical Respiratory Physiology 37:1119-1127 (2011); Guy et al., Hepatology doi: 10.1002/hep.25559 (2011), Lam et al., Curr. Op. in Rheum. 23:562-567 (2011); Lomas et al., Intn'l J. Clin. and Exp. Pathol. 5:58-71 (2102)). We selected this group of highly co-regulated genes and re-clustered the dataset restricted to the expression patterns of these "myofibroblast" genes (Figure 1D). This analysis yielded three major subsets characterized by low, medium, or high coordinate expression of myofibroblast-related genes. The low expressing group comprised all the controls and a few IPF subjects, while the medium and high expressing groups comprised the remaining IPF subjects.

**Table 4. Partial list of differentially expressed genes (> 1.5-fold up-regulated, q<0.05) associated with a myofibroblast gene expression signature.**

| **ProbeID** | **Gene Symbol** | **Gene Name** | **Entrez ID** | L**ogFC** | **p-value** | **adj. p-value** |
|---|---|---|---|---|---|---|
| A_24_P254789 | COL14A1 | collagen, type XIV, alpha 1 | 7373 | 3.41189889 | 2.34E-16 | 2.58E-14 |
| A_23_P112554 | COL15A1 | collagen, type XV, alpha 1 | 1306 | 2.26212501 | 2.40E-10 | 1.65E-09 |
| A_23_P207520 | COL1A1 | collagen, type I, alpha 1 | 1277 | 2.12583648 | 3.83E-08 | 1.56E-07 |
| A_24_P277934 | COL1A2 | collagen, type I, alpha 2 | 1278 | 1.10628708 | 0.00034794 | 0.00045028 |
| A_23_P33196 | COL5A2 | collagen, type V, alpha 2 | 1290 | 1.05662729 | 0.00154581 | 0.00171757 |
| A_24_P291814 | COL12A1 | collagen, type XII, alpha 1 | 1303 | 0.97095129 | 0.00038437 | 0.00048047 |
| A_23_P211212 | COL18A1 | collagen, type XVIII, alpha 1 | 80781 | 0.96802064 | 7.89E-06 | 1.58E-05 |
| A_23_P160318 | COL16A1 | collagen, type XVI, alpha 1 | 1307 | 0.7694129 | 0.00031612 | 0.00042374 |
| A_23_P111888 | CTHRC1 | collagen triple helix repeat containing 1 | 115908 | 2.96408731 | 5.87E-10 | 3.80E-09 |
| A_23_P153489 | CLEC11A | C-type lectin domain family 11, member A | 6320 | 0.63080724 | 0.00020336 | 0.00028316 |
| A_23_P93787 | HGF | hepatocyte growth factor (hepapoietin A; scatter factor) | 3082 | 0.82101019 | 8.11E-05 | 0.00012564 |
| A_23_P353035 | IGFBP7 | insulin-like growth factor binding protein 7 | 3490 | 0.96418065 | 3.14E-06 | 6.91E-06 |
| A_23_P124084 | LOXL1 | lysyl oxidase-like 1 | 4016 | 1.19477688 | 7.34E-07 | 2.02 E-06 |
| A_23_P111995 | LOXL2 | lysyl oxidase-like 2 | 4017 | 0.71925849 | 0.00080392 | 0.00094075 |
| A_23_P105251 | GLI1 | GLI family zinc finger 1 | 2735 | 2.08216778 | 4.61E-11 | 3.84E-10 |
| A_23_P500034 | GL12 | GLI family zinc finger 2 | 2736 | 1.53294083 | 7.06E-09 | 3.24E-08 |
| A_23_P70818 | SMO | smoothened, frizzled family receptor | 6608 | 1.50997486 | 6.02E-12 | 8.28E-11 |
| A_24_P137501 | SFRP2 | secreted frizzled-related protein 2 | 6423 | 4.337804 | 1.89E-14 | 6.94E-13 |
| A_23_P48740 | DIO2 | deiodinase, iodothyronine, type II | 1734 | 3.33676014 | 5.27E-15 | 2.90E-13 |
| A_23_P152305 | CDH11 | cadherin 11, type 2, OB-cadherin (osteoblast) | 1009 | 0.7647008 | 0.00102531 | 0.00115274 |
| A_24_P347411 | POSTN | periostin, osteoblast specific factor | 10631 | 2.13051858 | 1.37E-07 | 4.85E-07 |
| A_24_P373096 | TGFB3 | transforming growth factor, beta 3 | 7043 | 1.91410548 | 0.00011172 | 0.0001617 |

| | | | | | | |
|---|---|---|---|---|---|---|
| ProbeID = the identification number of the probe on the Agilent Whole Human Genome Microarray; Gene Symbol = NCBI Entrez Gene Symbol; Gene Name = NCBI Entrez Gene Name; ENTREZ ID = NCBI Entrez Gene ID; logFC = mean of the log base 2 expression levels in IPF samples minus the mean of the log base 2 expression levels in healthy control samples; p-value = calculated using t-test for differential expression between IPF and control per probe; adj. p-value = adjusted for multiple testing using Bonferroni's method. | | | | | | |

To determine whether the bronchiolar, lymphoid, and myofibroblast signatures are related to each other in individual subjects, we derived indices of expression for each signature by taking the normalized mean expression of all the genes in each cluster. This permitted us to compare the intensity of each signature on an individual patient basis. While most IPF subjects had higher bronchiolar, lymphoid, or myofibroblast signature scores than controls, there was a wide range of variability in each score and the two signatures did not display any significant patterns of correlation within individual subjects with IPF (Fig. 1E). This result suggests that the three signatures may reflect orthogonal processes heterogeneously expressed across IPF patients and/or differential sampling of heterogeneous processes reflected in IPF tissue.

To determine whether selected genes included in each of the three signatures were expressed in spatially distinct regions of IPF lung tissue, we performed immunohistochemistry (IHC) on frozen sections of biopsy tissue taken from IPF lung explants (N=5). We found that regions of bronchiolization, as characterized by honeycombed cysts, were lined with columnar epithelial cells (Fig. 2A) with abundant cytoplasm, near to but distinct from regions of collagen deposition (Fig. 2B). These epithelial cells stained positively for keratin 14, a protein encoded by a gene included in the bronchiolar signature (Fig. 2C), and expressed mucins as detected by PAS stain (Fig. 2D). In spatially distinct areas, we observed aggregates of cells with darkly staining nuclei by H&E (Fig. 2E), near to but distinct from regions of high collagen deposition (Fig. 2F). These aggregates stained positively for CD20, a B cell surface marker encoded by a gene included in the lymphoid signature (Fig. 2G-H). Bronchiolized regions were spatially distinct from lymphoid aggregates (Figs. 2I and J), and both bronchiolized regions and lymphoid aggregates often occurred near to, but spatially distinct from, regions of high collagen deposition (Figs. 2 B and F); multiple collagen genes are included in the fibroblast signature.

### Identification of differentially expressed genes encoding candidate serum biomarkers

To narrow down the list of candidate genes to evaluate as encoding candidate serum biomarkers as well as to otherwise identify candidate blood proteins to evaluate as biomarkers, we applied a more stringent cutoff of > 2 fold upregulation in IPF (q<0.05) in both UCSF Cohort 1 dataset and the GSE10667 dataset (Konishi et al., Am. J. Respir. Crit. Care Med. 180(2):167-75 (2009)). This analysis yielded 291 genes commonly upregulated in both datasets (see Table 5). Among these common genes were several that encoded extracellular and/or secreted proteins that might be detectable in peripheral blood and thus, would be good candidates for further evaluation as possible serum or other biomarkers. Based on these results and previously published data and readily available assays for serum biomarker detection, we selected YKL-40, COMP, OPN, MMP7, periostin, CXCL13, CCL11 (eotaxin), CCL13, CCL17 (TARC), CCL18, MMP3 and serum amyloid A (SAA), and in particular constitutive SAA, SAA4, as candidate prognostic blood biomarkers in IPF. (See, e.g., Korthagen et al., Resp. Med. 105:106-113 (2011), Pardo et al., PLoS Med. 2(9):891-903 (2005), Richards et al., Am JRespir Crit Care Med, doi:10.1164/rccm.201101-0058OC (2011), Yokoyama et al., Respirology 11:164-168 (2006), Kinder et al., Chest 135:1557-1563 (2009)). In addition, CCL18 has been previously reported to be a prognostic biomarker for survival in IPF (Prasse et al., Am J. Respir. Crit. Care Med. 179:717-723 (2009)), although it was not detected as significantly DE in our microarray experiment.

The blood biomarkers were selected according to various categories related to their predicted biology. CCL11, CCL13, CCL17, and CCL18 are chemokines associated with type 2 inflammation. We have previously shown CCL13 and CCL17 to be pharmacodynamically modulated in response to IL13 blockade in asthma (Corren The New England journal of medicine 365:1088-1098 (2011)). Periostin is related to IL13 biology (Corren The New England journal of medicine 365:1088-1098 (2011); Woodruff et al., Proc Natl Acad Sci U S A 104:15858-15863 (2007); Woodruff et al., Am JRespir Crit Care Med 180:388-395 (2009); Jia et al., The Journal of allergy and clinical immunology 130(3):647-654.e10 (2012)) and is also a representative gene in the fibroblast gene signature described above. CXCL13 is a B cell chemoattractant highly expressed in lymphoid aggregates and is a representative gene in the lymphoid gene signature described above. MMP3 and SAA4 are representative genes in the bronchiolar gene signature described above. YKL-40 is a chitinase-like protein associated with myeloid cell activation and systemic levels are elevated in multiple disease states. A previously published study has shown that YKL-40 is highly expressed in IPF and serum levels of YKL-40 are prognostic for survival (Korthagen et al., Respiratory medicine 105:106-113 (2011)). COMP is induced by TGFβ and is highly expressed in myofibroblasts in scleroderma (Farina et al., Matrix biology : journal of the International Society for Matrix Biology 25:213-222 (2006); Farina et al., Annals of the rheumatic diseases 68:435-441 (2009)). OPN and MMP7 are highly expressed in IPF tissue; blood levels of OPN have been shown to be negatively correlated with lung function in IPF (Kadota et al., Respiratory medicine 99:111-117 (2005)) and blood levels of MMP7 have been shown to be prognostic for survival in IPF (Richards et al., Am J Respir Crit Care Med 185(1):67-76 (2012)).

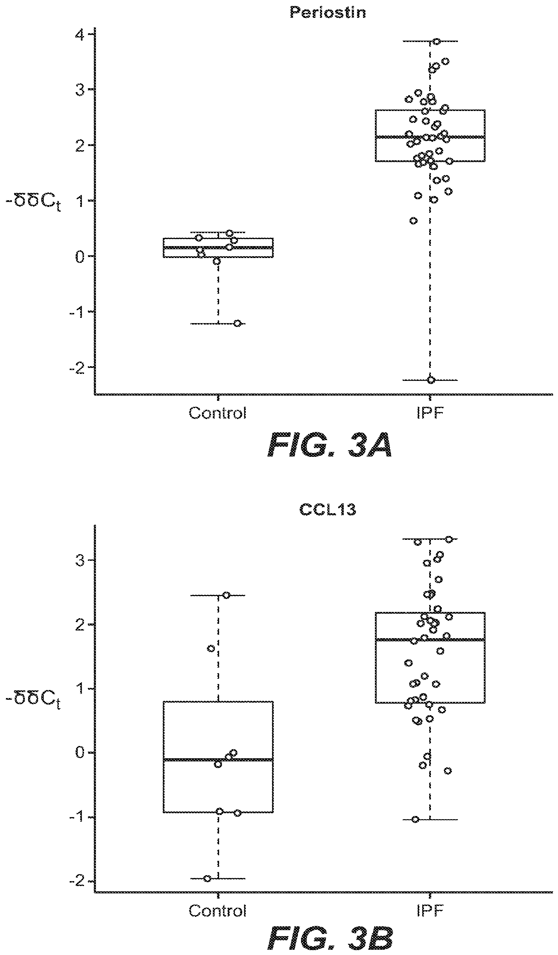

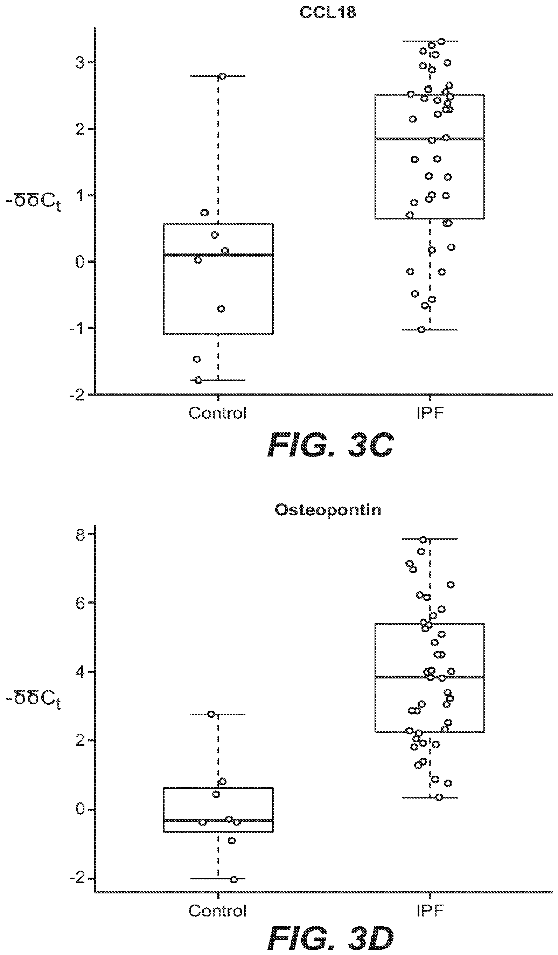

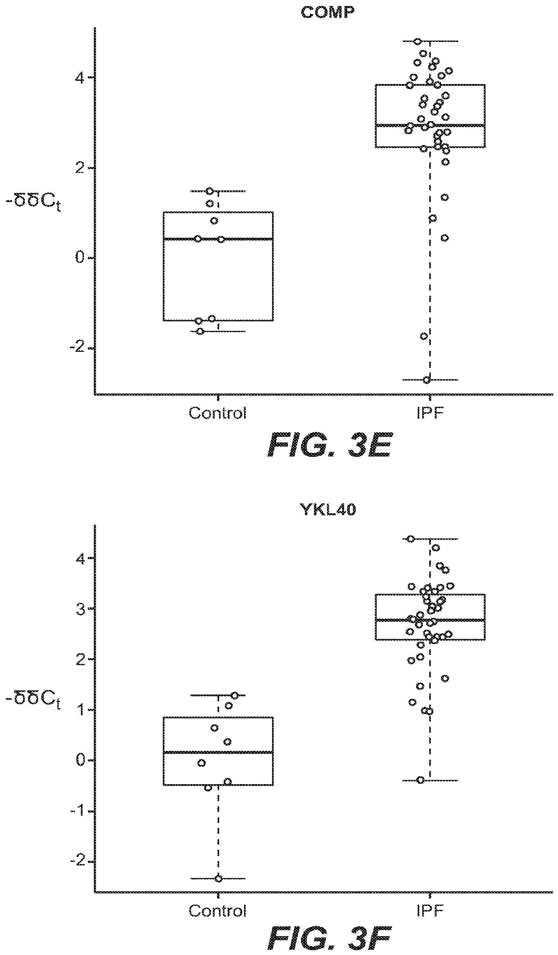

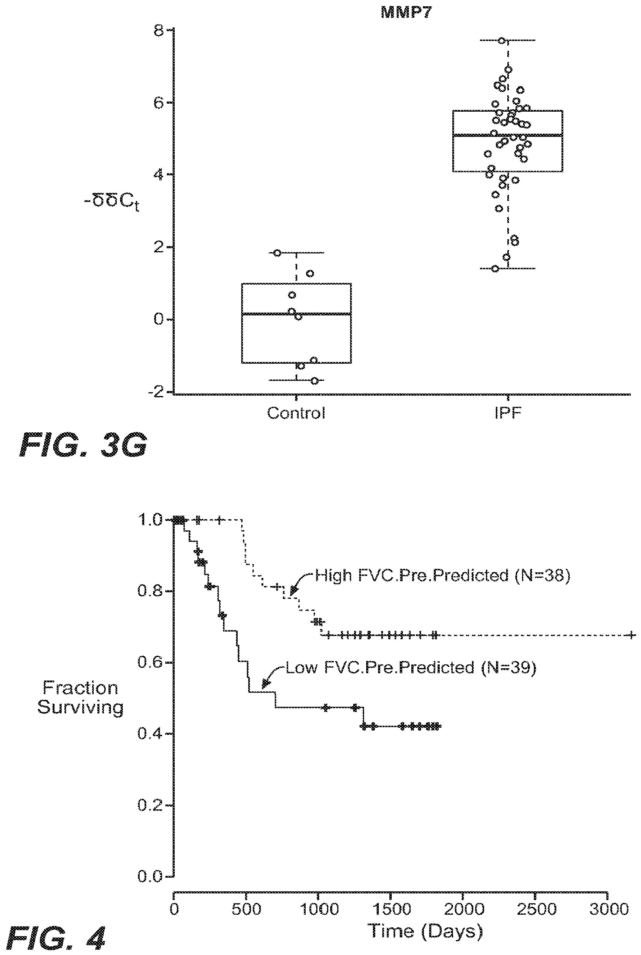

To confirm the differential expression of these candidate biomarker genes in IPF lung, we performed quantitative RT-PCR (qPCR) (see Methods) on the same RNA samples used in the microarray experiment. Table 6 shows the microarray gene expression data and Figs. 3A-G show the qPCR data. As shown in Table 6 and Figs. 3A-G, we observed significantly elevated expression of each selected candidate biomarker gene in IPF patients relative to controls.

**Table 5. Partial list of differentially expressed genes (>2.0-fold up-regulated, q<0.05) in two independent IPF datasets.**

| **Gene Symbol** | **GSE10667 adj. p-value** | **GSE10667 logFC** | **UCSF Cohort 1 adj. p-value** | **UCSF Cohort 1 logFC** |
|---|---|---|---|---|
| CHI3L1 (YKL-40) | 0.010960171 | 1.39622824 | 1.19997E-07 | 2.972810559 |
| CCL13 | 0.000480298 | 1.650926419 | 0.029881609 | 1.104407622 |
| COMP | 1.64133E-10 | 4.382169308 | 2.0246E-06 | 2.52240034 |
| CXCL13 | 0.005238542 | 2.637421496 | 0.000629905 | 3.437801699 |
| MMP7 | 3.26412E-05 | 2.874722338 | 6.8955E-11 | 3.413531235 |
| POSTN | 8.94681E-05 | 2.473564333 | 8.46908E-06 | 2.103941264 |
| SPP1 (OPN) | 5.94635E-07 | 3.762235672 | 8.37419E-05 | 3.64564152 |

| | | | | |
|---|---|---|---|---|
| Gene Symbol = NCBI Entrez Gene Symbol; logFC = mean of the log base 2 expression levels in IPF samples minus the mean of the log base 2 expression levels in healthy control samples; adj. p-value = adjusted for multiple testing using Bonferroni's method.. | | | | |

**Table 6. Gene expression of candidate biomarker genes in IPF biopsies as determined by microarray**

| **Gene Symbol** | **Fold change, microarray (log₂)** | **q-value, microarray** |
|---|---|---|
| SPP1 (OPN) | 3.65 | 8.4 x 10⁻⁵ |
| MMP7 | 3.41 | 6.9 x 10⁻¹¹ |
| CXCL13 | 3.44 | 6 x 10⁻⁴ |
| CHI3L1 (YKL-40) | 2.97 | 1.3 x 10⁻⁶ |
| COMP | 2.52 | 2.0 x 10⁻⁶ |
| POSTN | 2.10 | 8.5 x 10⁻⁶ |
| CCL13 | 1.10 | 0.03 |

To determine whether the elevated gene expression levels of the selected markers in IPF relative to control corresponded to elevated levels of proteins encoded by those genes in peripheral blood, we evaluated their levels in plasma (OPN) or serum (all others) in Cohort 2 IPF samples (N = 80) and in samples from healthy controls. For the plasma controls, N = 10; for the serum controls, N = 29. Each of the blood biomarker assays was performed as described above.

Comparing all IPF patients to all controls, the peripheral blood levels of YKL-40, COMP, OPN, MMP7, CCL11, CCL13, CCL17, CCL18, SAA and CXCL13 were significantly elevated in IPF (Table 7). Using Wilcoxon rank-sum for IPF vs. control, we obtained the following p-values: YKL-40, COMP, MMP7, CCL13, CCL18 and CXCL13, p<10⁻⁴; OPN, p=0.04. Some IPF patients had elevated levels of serum periostin or MMP3 relative to controls but overall this difference did not reach statistical significance. A previous report suggested that elevated levels of periostin could be detected in IPF lungs and peripheral blood (Okamoto et al., The European respiratory journal: official journal of the European Society for Clinical Respiratory Physiology 37:1119-1127 (2011)). It has previously been shown that immunomodulatory therapy (specifically, corticosteroids) can reduce the gene expression levels of periostin (Woodruff et al., Proc. Natl. Acad. Sci. 104(40):15858-63 (2007); Woodruff et al., Am J. Respir. Crit. Care Med. 180(5):388-95 (2009)) and it is anticipated that peripheral blood levels of periostin would be similarly reduced in the presence of such therapy. Thus, the variation in serum periostin levels in IPF patients and the lack of significant difference to controls could be explained by the fact that 21/80 IPF patients were on immunomodulatory therapy (IT, systemic corticosteroids or azathioprine) at the time of sample collection.

We thus examined whether peripheral blood levels of any of the biomarkers, including periostin, were different between IPF patients on IT vs. IPF patients not on IT. IPF patients on IT exhibited trends for lower levels of COMP and periostin compared to IPF patients not on IT. In contrast, IPF patients on IT had significantly elevated levels of CXCL13, MMP3, and SAA compared to IPF patients not on IT (Table 7). There were no significant differences or trends toward differences in levels of any of the other biomarkers according to IT status.

**Table 7. Range and distribution of blood biomarker levels in IPF patients and in controls.**

| **Biomarker** | **Median Level (IQR) in Controls¹** | **Median Level (IQR) in IPF Patients¹** | **Median Level (IQR) in IPF Patients Not on IT (N=59)** | **Median Level (IQR) in IPF Patients on IT (N=21)** |
|---|---|---|---|---|
| POSTN (ng/ml) | 26.6 (23.9-33.8) | 28.6 (23.3-38.4) | 30.0 (23.4-39.4) | 25.0 (21.5-35.1)³ |
| CCL11 (pg/ml) | 257 (188-292) | 225 (259-447)² | 335 (259-440) | 340 (255-456) |
| CCL13 (pg/ml) | 159 (111-215) | 299 (226-400)² | 300 (244-387) | 297 (193-441) |
| CCL17 (pg/ml) | 275 (228-411) | 510 (304-823)² | 453 (298-760) | 572 (353-1083) |
| CCL18 (ng/ml) | 42 (29-51) | 94 (76-122)² | 94 (77-119) | 100 (74-124) |
| MMP3 (ng/ml) | 20 (14-26) | 22 (16-42) | 20 (11-26) | 45 (26-112)² |
| SAA (ng/ml) | 695 (286-1038) | 1618 (773-4625)² | 1234 (630-2543) | 4625 (2910-8815)² |
| CXCL13 (pg/ml) | 28 (22-39) | 80 (55-136)² | 69 (50-125) | 113 (82-221)² |
| COMP (ng/ml) | 540 (432-720) | 901 (706-1163)² | 943 (756-1191) | 749 (576-1101)³ |
| OPN (ng/ml) | 51 (41-68) | 69 (53-85)² | 68 (53-83) | 70 (47-89) |
| YKL-40 (ng/ml) | 41 (23-54) | 105 (76-170)² | 104 (74-158) | 110 (77-195) |
| MMP7 (ng/ml) | 2.7 (2.4-3.5) | 7.4 (5.9-9.4)² | 7.5 (6.2-9.4) | 6.6 (5.0-9.8) |

| | | | | |
|---|---|---|---|---|
| = For all biomarkers except osteopontin and MMP3, N = 29 Controls and N = 80 IPF Patients and the values are serum levels; for osteopontin, N = 10 Controls and N = 80 IPF Patients and the values are plasma levels; for MMP3, N= 29 Controls and N= 78 IPF Patients; IQR = inter-quartile range, 25th-75th percentile. ² = Significantly higher in IPF than control, p < 0.05 by Wilcoxon rank-sum test. ³ = Trend toward significantly different among IPF patients by immunomodulatory therapy (IT) status, p < 0.1 by Wilcoxon rank-sum test. | | | | |

We next evaluated correlations between individual biomarkers and demographic and clinical variables. Of the 8 biomarkers tested, only OPN exhibited a significant difference by prior smoking status, where the plasma OPN levels were lower in former smokers than never smokers (p=0.01, data not shown). In comparing clinical variables, we observed nominally significant positive correlations between total lung capacity (TLC) % predicted and forced vital capacity (FVC) % predicted, as expected, as well as between each spirometric variable and diffusing capacity (DL_{CO} % predicted). We also observed negative intercorrelations between dyspnea score (Watters et al., Am. Rev. Respir. Dis. 133(1):97-103 (1986)), FVC % predicted, and DLco % predicted (Table 8). Among the blood biomarkers, we observed generally weak intercorrelations between MMP7, periostin, and COMP; and between OPN, YKL-40, SAA, MMP3, CCL11 and CXCL13; with a strong intercorrelation between CCL13 and CCL17. The generally low correlation coefficients observed suggest that, in total, levels of the biomarkers are largely orthogonal to one another across the population (Table 8). We also observed relatively few signficant correlations between individual biomarkers and clinical/demographic variables; of these, CXCL13 was negatively correlated with DLco % predicted and positively correlated with dyspnea score; CCL11 was positively correlated with radiographic score; SAA was negatively correlated with TLC % predicted, and positively correlated with radiographic score and dyspnea score; MMP3 was negatively correlated with FVC % predicted and positively correlated with dyspnea score; and OPN and COMP were weakly correlated with age (Table 8).

**Table 8. Correlations between biomarkers and clinical/demographic features.**

| **Variable** | **by Variable** | **rs** | **p-value** |
|---|---|---|---|
| TLC %Predicted | FVC %Predicted | 0.83 | 0.0001 |
| TLC %Predicted | DLCO %Predicted | 0.34 | 0.0177 |
| DLCO %Predicted | FVC %Predicted | 0.34 | 0.0044 |
| Dyspnea Score | DLCO %Predicted | -0.43 | 0.0004 |
| Dyspnea Score | FVC %Predicted | -0.29 | 0.0118 |
| Radiographic score | FVC %Predicted | -0.37 | 0.0326 |
| CXCL13 (pg/ml) | DLCO %Predicted | -0.35 | 0.0035 |
| CXCL13 (pg/ml) | Dyspnea Score | 0.40 | 0.0004 |
| CCL11 | Radiographic score | 0.44 | 0.0086 |
| SAA | TLC %Predicted | -0.30 | 0.0427 |
| SAA | Radiographic score | 0.43 | 0.0108 |
| SAA | Dyspnea score | 0.35 | 0.0024 |
| MMP3 | FVC %Predicted | -0.25 | 0.0313 |
| MMP3 | Dyspnea score | 0.24 | 0.0427 |
| OPN (ng/ml) | Age at initial visit | 0.23 | 0.0395 |
| COMP (ng/ml) | Age at initial visit | 0.26 | 0.0239 |
| MMP7 (ng/ml) | POSTN (ng/mL) | 0.40 | 0.0002 |
| MMP7 | SAA | -0.29 | 0.0107 |
| COMP (ng/ml) | POSTN (ng/mL) | 0.24 | 0.0337 |
| COMP (ng/ml) | MMP7 (ng/ml) | 0.24 | 0.0342 |
| OPN (ng/ml) | YKL40 (ng/mL) | 0.23 | 0.0385 |
| CXCL13 (pg/ml) | YKL40 (ng/ml) | 0.26 | 0.0180 |
| CXCL13 (pg/ml) | OPN (ng/ml) | 0.27 | 0.0145 |
| CXCL13 | MMP3 | 0.37 | 0.0008 |
| CXCL13 | SAA | 0.37 | 0.0009 |
| CCL11 | MMP3 | 0.23 | 0.0449 |
| CCL11 | YKL40 | 0.24 | 0.0333 |
| CCL11 | CCL13 | 0.25 | 0.0245 |
| CCL11 | CXCL13 | 0.29 | 0.0101 |
| CCL13 | CCL17 | 0.43 | 0.0001 |
| SAA | MMP3 | 0.41 | 0.0002 |

| | | | |
|---|---|---|---|
| rₛ = Spearman's rank correlation; p-value refers to the Spearman correlation. | | | |

### Establishing the Prognostic Value of Blood Biomarkers

Previous studies have indicated weak to no prognostic value for single timepoint values of lung function, diffusing capacity, smoking status, and age for subsequent survival in IPF patients. Consistent with those findings, we did not observe any dramatic differences in survival for any of those variables taken individually with the exception of FVC % predicted, where patients presenting with greater than the median FVC % predicted for the population (> 69%) had a survival advantage over patients presenting with FVC% predicted < 69% (HR = 0.37, p=0.02, Figure 4).

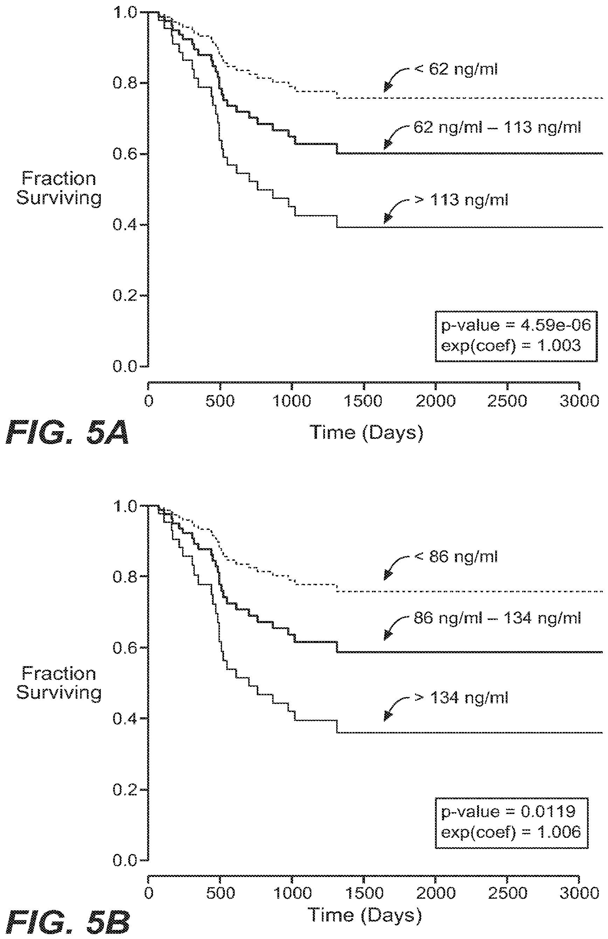

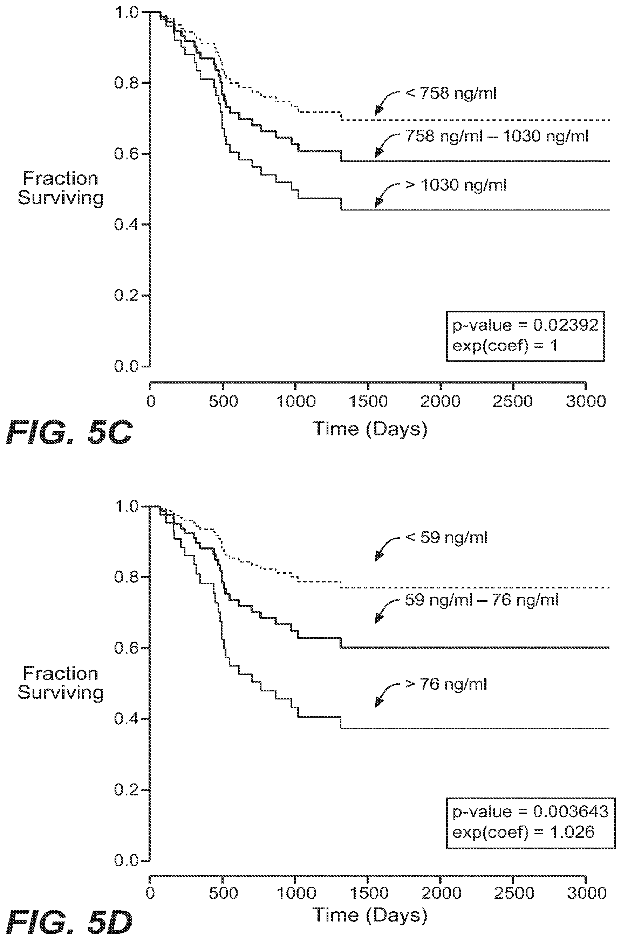

To assess the prognostic value for subsequent survival of various blood biomarkers, we applied a Cox proportional hazards model to assess the prognostic value of each of the blood biomarkers individually (periostin, CCL13, CCL18, osteopontin, COMP, YKL-40, MMP3, MMP7, SAA and CXCL13). Of those biomarkers, we found that CXCL13, YKL-40, COMP, OPN, MMP3 and SAA levels at baseline significantly differentiated IPF patients by subsequent transplant-free survival (Figures 5A-D and Table 9). Contrary to prior published reports (Richards et al., Am J Respir Crit Care Med, doi: 10.1164/rccm.201101-0058OC (2011); Prasse et al., Am J Respir Crit Care Med 179:717-723 (2009)), we did not observe any prognostic value for levels of MMP7 or CCL18 in this cohort, nor did we observe statistically significant prognostic value for any of the other biomarkers (including CCL13 and periostin) tested in this cohort.

**Table 9. Prognostic value of individual biomarkers in IPF patients.**

| **Biomarker** | **Exp (coefficient)** | **p-value** |
|---|---|---|
| Periostin | 1.017 | 0.2 |
| CCL11 | 1.002 | 0.05 |
| CCL13 | 1.000 | 0.7 |
| CCL17 | 1.000 | 0.4 |
| CCL18 | 0.994 | 0.3 |
| MMP3 | 1.018 | ***5.0x10⁻⁸*** |
| SAA | 1.000 | ***3.0x10⁻⁴*** |
| CXCL13 | 1.003 | ***7.3x10⁻⁶*** |
| COMP | 1.000 | ***0.030*** |
| OPN | 1.026 | ***0.004*** |
| YKL-40 | 1.006 | ***0.010*** |
| MMP7 | 1.053 | 0.1 |

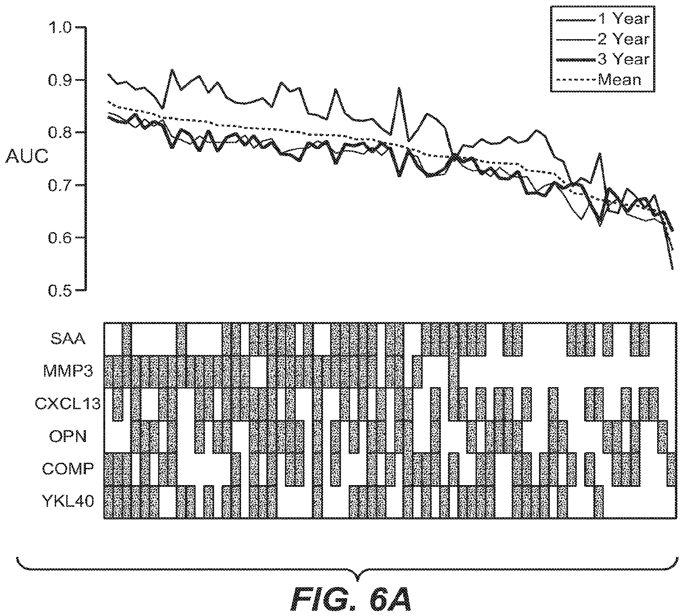

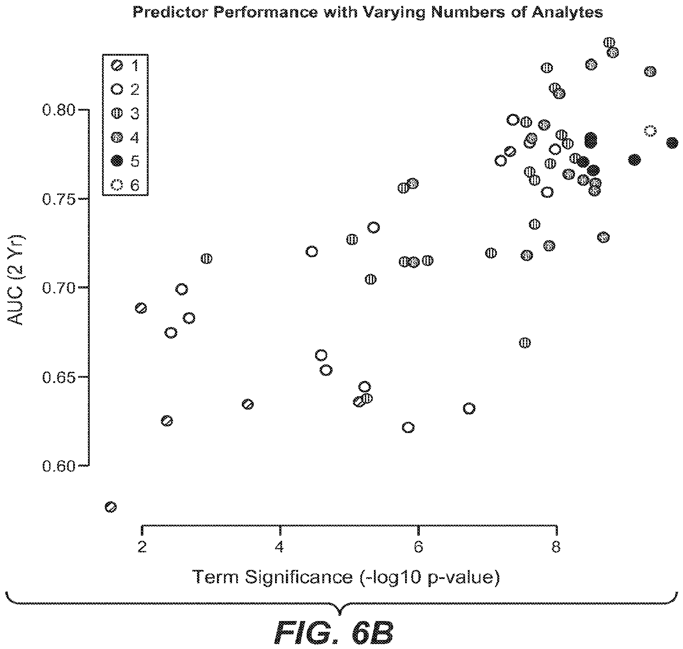

As the levels of the individual prognostic markers CXCL13, YKL-40, OPN, COMP, MMP3, and SAA were not highly intercorrelated within IPF patients, we examined whether combinations of these markers might further enrich for prognostic value. We assessed the composite value of each possible combination of the six biomarkers indicated above to predict mortality over 1, 2, and 3 years following sample collection using a receiver operating characteristic (ROC) analysis. We found that, in general, single biomarkers or pairs of biomarkers performed unfavorably as compared to combinations of three or more biomarkers (Fig. 6A). To determine an optimal minimum number of biomarkers in combination, we assessed the area under the curve (AUC) of the ROC analysis at 2 years and term significance of all possible combinations of the six biomarkers, and found that many combinations of 3 biomarkers performed comparably to combinations of 4 or more biomarkers (Fig. 6B).

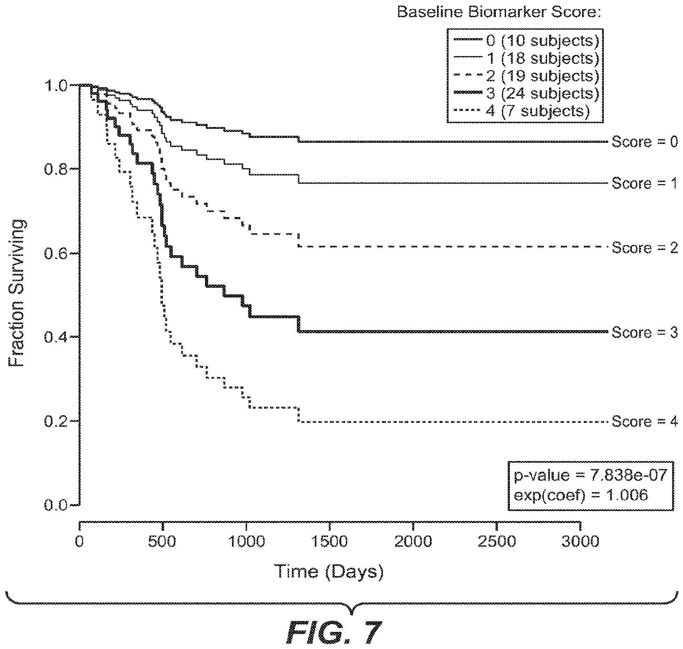

To achieve a relatively comparable distribution of numbers of IPF patients across categories, we devised a simple scoring system whereby baseline biomarker levels above the median level for the entire cohort earned a score of 1 for each biomarker. Thus patients having biomarker levels below the median level for all 4 biomarkers were assigned a score of 0, above the median for one biomarker and below the median for the other 3 biomarkers were assigned a score of 1, and so on. We derived the following values for each biomarker. The median level for CXCL13 was 80 pg/ml; the median level for YKL-40 was 105 ng/ml; the median level for COMP was 901 ng/ml; and the median level for OPN was 69 ng/ml (see Table 7). This scoring system dramatically differentiated groups of patients by survival using a Cox proportional hazards model (p<10⁻⁶, Fig. 7).

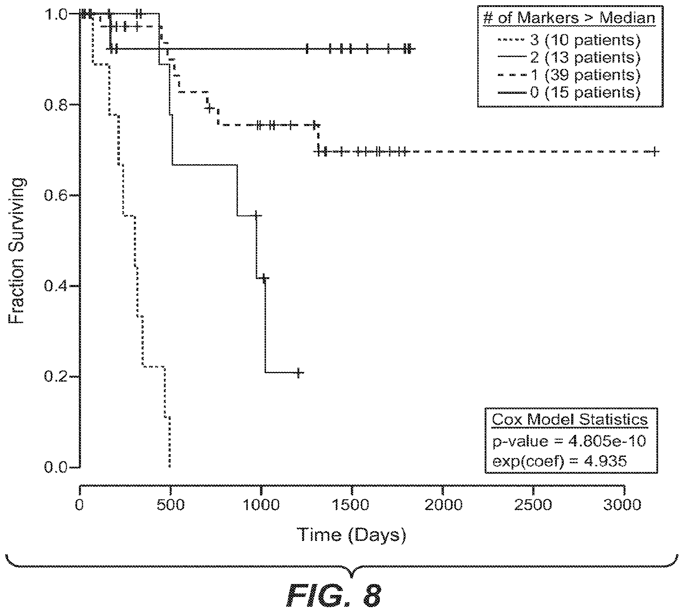

Using the top performing combination of three biomarkers in the ROC analysis (MMP3, COMP, and YKL40), to achieve a relatively comparable distribution of numbers of IPF patients across categories, we applied the scoring system described above where patients below the median for all 3 biomarkers were assigned a score of 0, above the median for one biomarker and below the median for the other 2 biomarkers were assigned a score of 1, above the median for any 2 biomarkers but below the median for the third biomarker were assigned a score of 2, and above the median for all 3 biomarkers were assigned a score of 3. This scoring system dramatically differentiated groups of patients by survival using a Cox proportional hazards model (p<10⁻⁹, Figure 8; Kaplan-Meier survival data are plotted). Of the 15 patients with a score of 0, only one death was recorded in the follow-up period, whereas all of the patients with a score of 3 died within 500 days of sample collection.

Accordingly, we have shown that measuring the blood levels of certain biomarkers, CXCL13, YKL-40, COMP, OPN, MMP3, and SAA and determining whether the values of each biomarker fall above or below the median level can be used to assess the survival prognosis of an IPF patient at the time of diagnosis. In addition, use of the scoring system described herein to assess whether the blood biomarker level is above or below the median level for IPF patients for none, one, two, three, or all four of the biomarkers CXCL13, YKL-40, COMP and OPN, or for none, one, two, or all three of the biomarkers MMP3, COMP, and YKL-40, can increase the accuracy of the survival prognosis. Such a system is useful for identifying patients for aggressive therapeutic intervention or for lung transplant as well as for stratifying patients for clinical trials of therapeutic agents.

Risk prediction in IPF is important for making treatment and patient management decisions such as lung transplantation and is useful for stratifying enrollment in clinical trials of investigational therapeutics. As direct sampling of the lung is often impractical or infeasible in patients with IPF, noninvasive assessments of disease activity and progression is of significant value. Imaging via high-resolution computed tomography (HRCT), spirometry, and other measurements of lung function such as diffusing capacity and exercise tolerance have some utility in monitoring the progression of an individual patient's disease via repeated measures over time but single point assessments of lung function are relatively less informative on a population basis(Ley et al., Am J. Respir. Crit. Care Med. 183:431-440 (2011)). Blood-based biomarkers as described here have the potential to enable drug development for and clinical management of IPF on several levels: as predictive, prognostic, pharmacodynamic, and surrogate measures of disease activity.

Predictive biomarkers provide evidence of the activity of a particular molecular pathway prior to treatment and identify a subpopulation of patients most likely to benefit from a targeted therapy. A given pathway may be heterogeneously expressed across a population of IPF patients and the ability to identify clinical benefit from agents targeting that pathway may be compromised if only a subset of patients that cannot otherwise be prospectively identified exhibits benefit. A predictive biomarker that identifies IPF patients most likely to benefit from a targeted therapeutic could help stratify enrollment in clinical trials to more rigorously test the therapeutic hypothesis.

Prognostic biomarkers stratify the risk of future disease progression or death. Given the high mortality in IPF, a successful therapeutic intervention might be expected to significantly prolong lifespan relative to placebo. Given the variability in disease trajectory, however, it is challenging to assess survival benefits in early-stage clinical trials without treating large numbers of patients for many years. A prognostic biomarker that identifies IPF patients most likely to suffer significant disease progression or death within a 1-2 year period is useful to stratify enrollment in clinical trials to assess whether there is a short-term survival benefit in patients most likely to progress during the trial. Furthermore, it is possible that a given therapeutic intervention benefits patients with relatively good prognoses but is ineffective in patients whose disease has progressed past a certain point of no return. For example, a subgroup analysis of the recent phase 2 study of BIBF1120 showed that most of the placebo-adjusted benefit on lung function was observed in patients with greater than 70% predicted FVC at baseline, whereas patients with less than 70% predicted FVC did not show much benefit (Richeldi L et al, abstract, ERS 2011 Annual Congress).

Pharmacodynamic biomarkers should reflect the proximal activity of a particular molecular pathway involved in the disease process and should change in response to a specific therapeutic intervention. Changes in pharmacodynamic markers upon treatment indicate whether and to what extent the molecular intervention is affecting its target; thus these markers may help enable appropriate dose selection in a dose-ranging study. In a disease with poorly defined short-term clinical outcome measures such as IPF, significant pharmacodynamic effects in the absence of any clinical benefit may help discriminate between inappropriate target selection and inappropriate dosing as the reason for failure of a trial. Surrogate biomarkers, like pharmacodynamic markers, should change in response to treatment but may be distal to the targeted pathway and are linked more closely to downstream manifestations of disease and clinical outcomes. Changes in surrogate biomarkers over the short term may indicate the likely long-term efficacy of continued treatment, e.g. on survival outcomes. A given biomarker may represent any, several, or all of the predictive, prognostic, pharmacodynamic, and surrogate categories.

IL-13 is a mediator of fibrosis and has been implicated as a potential therapeutic target in IPF (Wynn, TA, J. Exp. Med. 208:1339-1350 (2011)). As discussed above, we have found that IL-13 and genes potentially regulated by IL-13 such as IL13Rα2, CCL11, CCL13, CCL17, CCL18, and periostin are expressed at elevated levels in lung biopsies from IPF patients. In one Phase II study, therapeutic IL-13 blockade demonstrated clinical benefit in asthma, and the benefit was enhanced in a subset of patients predicted to have elevated IL-13 expression in their airways as determined by serum periostin levels (Corren et al., New Engl. J. Med. 365:1088-1098 (2011)). In the experiments discussed above, we found that across the entire cohort of IPF patients, serum periostin levels were not significantly higher than those measured in healthy controls (Table 7). However, periostin levels were clearly elevated in a subset of IPF patients (Table 7), and the distribution of serum periostin levels in IPF is similar to that observed in severe asthma (Jia et al., The Journal of allergy and clinical immunology 130(3):647-654.e10 (2012)). Periostin is expressed in the fibroblast/myofibroblast signature described here and has been localized to fibroblastic foci in IPF by IHC (Okamoto et al., The European respiratory journal : official journal of the European Society for Clinical Respiratory Physiology 37:1119-1127 (2011)). If IL-13 blockade has clinical efficacy in IPF patients, it may be evident only in a subset of patients with elevated pretreatment levels of serum periostin. Similarly, we found that CCL13 and CCL17 levels are elevated in asthma patients and significantly decrease in response to therapeutic IL-13 blockade (Corren et al., New Engl. J. Med. 365:1088-1098 (2011)). As CCL13 and CCL17 levels are elevated in IPF patients, CCL13 and CCL17 may also serve as pharmacodynamic markers of IL-13 pathway activity in IPF. CCL11 is a product of stromal cells that is upregulated in response to IL-13 (Matsukura et al., Am. J. of Respir. Cell and Mol. Biol. 24:755-761 (2001)). CCL18 is a product of alternatively activated macrophages (Prasse et al., Arthritis and Rheum. 56:1685-1693 (2007)), which themselves may be a source of IL-13 (Kim et al., Nature Med. 14:633-640 (2008)). Hence, CCL11 and CCL18 may also serve as predictive and/or pharmacodynamic biomarkers in a therapeutic study of IL-13 blockade in IPF.

Serum levels of OPN, YKL-40, COMP, CXCL13, MMP3, and SAA each significantly predicted subsequent disease progression in IPF. However, their levels were not dramatically intercorrelated within patients across the population examined. Each marker may be produced by different cellular sources and reflect activity of different pathogenic processes within IPF lesions.

OPN is expressed at elevated levels in IPF (Pardo et al, PLoS Med 2:e251 (2005)) and its expression is localized to fibroblastic foci and hyperplastic alveolar epithelium in UIP lesions (Kelly et al., Am J Respir Crit Care Med 174:557-565 (2006)). OPN is thought to play roles in cell adhesion, migration, inflammation, and tissue remodeling (O'Regan, A., Cytokine & growth factor reviews 14:479-488 (2003)), and OPN-deficient mice are protected from bleomycin-induced pulmonary fibrosis (Berman et al., American journal of physiology Lung cellular and molecular physiology 286:L1311-1318 (2004)). Plasma OPN levels have been reported by other investigators to be elevated in IPF patients, and within IPF, OPN levels were negatively correlated with oxygen saturation (Kadota et al., Respiratory medicine 99:111-117 (2005)).

YKL-40 is a chitinase-like protein produced primarily by myeloid-derived cells such as activated macrophages (Rehli et al., The Journal of biological chemistry 278:44058-44067 (2003)). Its systemic levels are elevated in a number of inflammatory and neoplastic conditions associated with tissue remodeling (Lee et al., Annual review of physiology 73:479-501 (2011)), and our findings are consistent with a prior report showing that baseline serum YKL-40 levels are prognostic for subsequent mortality in IPF patients (Korthagen et al., Respiratory medicine 105:106-113 (2011)).

COMP is an extracellular matrix protein expressed primarily by fibroblasts in cartilage, ligament, tendon, and bone. In patients with systemic sclerosis, lesional skin myofibroblasts were found to express elevated levels of COMP, and its expression could be induced by TGFβ (Farina et al., Matrix biology : journal of the International Society for Matrix Biology 25:213-222 (2006); Farina et al., Annals of the rheumatic diseases 68:435-441 (2009)).

CXCL13 is a chemokine produced by follicular dendritic cells. It recruits B cells to secondary and tertiary lymphoid structures by binding to its cognate receptor CXCR5 and both are required for lymphoid follicle formation (Ansel et al., Nature 406:309-314 (2000)). Recently, lymphoid aggregates have been reported to associate with UIP lesions in IPF biopsies (Nuovo et al., Mod. Pathol. (2011) 1-18) and B cell infiltrates with high CXCL13 expression have been reported in renal fibrosis (Heller et al., The American journal of pathology 170:457-468 (2007)). Serum CXCL13 has been described as a biomarker of the severity of joint erosions (Meeuwisse et al., Arthritis and rheumatism 63:1265-1273 (2011)) and of B cell repopulation after rituximab treatment in rheumatoid arthritis (Rosengren et al., Rheumatology (Oxford) 50:603-610 (2011)). CXCL13 expression clusters with the lymphoid signature described here.

MMP3 and SAA4 are expressed in the bronchiolar signature described here. Elevated MMP3 protein levels have been described in BAL fluid from IPF patients (Richter et al., Thorax 64:156-161 (2009)), but systemic levels of MMP3 as a biomarker for IPF has not been previously described. SAA is an acute-phase reactant that is elevated in many inflammatory conditions but has not been described as a biomarker for IPF.

Taken together, systemic levels of each of these six biomarkers (OPN, YKL-40, COMP, CXCL13, MMP3, and SAA) in IPF patients may reflect variable relative contributions of distinct pathogenic processes.

Given the geographic heterogeneity and patchy nature of IPF pathology, peripheral blood levels of these biomarkers may give a more comprehensive picture of the cumulative disease burden in IPF than gene expression levels from a single small biopsy. Although most of the six biomarkers (OPN, YKL-40, COMP, CXCL13, MMP3, and SAA) were generally elevated in IPF patients compared to healthy controls, the relative levels of each of these biomarkers within the IPF population varied widely and may impact differently on disease progression. Hence the combinatorial value of various biomarkers as described herein may provide even further benefit than single markers. It will be interesting to assess the dynamics of these markers over time with respect to clinical disease progression.

### Example 2 - Characterization of Signaling Pathways in IPF

IL-13, TGFβ, and mediators of epithelial-mesenchymal communication, including the Hedgehog (Hh) pathway, have been implicated in the pathogenesis of idiopathic pulmonary fibrosis (IPF). For example, transgenic IL-13 overexpression in mouse lung has been shown sufficient to induce profound pulmonary fibrosis (Zhu et al., J. Clin. Invest. 103(6):779-88 (1999)), and mice deficient in IL-13 were partially protected from bleomycin-induced fibrosis as well as from schistosome-induced hepatic fibrosis. IL-13 binds to a heteromeric receptor complex comprising IL-13Rα1 and IL-4Rα, whereupon Jak family kinases phosphorylate STAT6, a transcription factor that mediates signaling downstream of IL-13 and IL-4. A second IL13 receptor, IL13Rα2, has been implicated as a nonsignaling decoy receptor that can compete for IL13 binding to the IL13Rα1/IL4Rα complex, thereby reducing STAT6 activation. IL13Rα2 can be induced by STAT6-dependent as well as STAT6-independent signals and is expressed at elevated levels in fibrotic tissue. Some reports (Fichtner-Feigl et al., Nat. Med. 12(1):99-106 (2006); Mandal et al., Inflamm Bowel Dis. 16(5):753-64 (2010)) have suggested that IL13Rα2 can transduce STAT6-independent signals that may contribute to fibrosis, although these studies have relied on ectopic IL13Rα2 overexpression in cell lines and the mechanism for IL13Rα2 signaling remains unclear. IL13Rα2-deficient animals exhibit increased tissue fibrosis while IL13Rα1-deficient animals exhibit decreased tissue fibrosis (Wilson et al., J. Clin. Invest. 117(10):2941-51 (2007); Mentink-Kane et al., Gastroenterology 141(6):2200-2209 (2011)). While these studies conducted in knockout animals are informative with respect to the roles of IL13 and its receptors in the initiation of fibrosis, they are more difficult to interpret in the context of established fibrosis as in human fibrotic diseases. While one body of evidence suggests that IL13Rα2 forms a decoy receptor that is unable to signal upon ligand-receptor engagement, an apparently contradictory body of evidence points to a positive role for IL13Rα2 in fibrogenesis. Thus, the roles of endogenous IL13 and IL13Rα2 in human IPF remain poorly understood.

To enhance our understanding of molecular pathways in IPF, we characterized genome-wide transcriptional profiles in IPF and in non-IPF, control tissues. These experiments led to the identification and characterization of signaling pathways downstream of IL13 in primary pulmonary fibroblasts.

### MATERIALS AND METHODS

### Human Lung Tissue Samples

Human lung tissue samples were obtained as described above in Example 1.

### Tissue Culture

IMR-90 cells (ATCC, Manassas, VA; Catalog # CCL-186) were cultured in DMEM medium supplemented with 10% FBS (Sigma, St. Louis, MO; Catalog # F2442) and Penicillin/Streptomycin (Invitrogen, Carlsbad, CA; Catalog # 15140). Cells were plated on a bed of growth factor reduced Matrigel (GFR Matrigel) [BD Biosciences, Bedford, MA; Catalog #354230]. Matrigel was thawed overnight on ice and a bed volume of 450µl/well in a 12-well plate was produced by allowing matrigel to harden at 37°C for 30 minutes. Cells (1E5-2E5) were then plated onto Matrigel bed. Unless otherwise indicated, IL-13 stimulation was performed with 10 ng/ml (IL-13 and TNFα) and 3 ng/ml (IL4).

### RNA Preparation

Snap frozen lung biopsy samples were pulverized in a pre-cooled (in liquid nitrogen) Bessman tissue pulverizer (Spectrum Laboratories, Rancho Dominguez, CA; catalog #189475). Trizol was added to pulverized material and pipetted several times. Lysates were incubated on ice for 10-15 minutes. Lysates were stored at -80°C until further processing. RNA was isolated from Trizol lysates according to the manufacturer's protocol. Trizol-isolated RNA was then subjected to another purification step using the Qiagen RNeasy columns, as per the manufacturer's protocol.

Tissue cultured cells were harvested by adding 1ml (per well in 12-well plate) Trizol and pipetting mixture several times. Trizol lysates were homogenized using the steel bead/Tissuelyser (Qiagen) method (20 s-1, 4 minutes) as indicated in the Qiagen RNeasy protocol. Upon homogenization, RNA was isolated following the Trizol manufacturer's protocol. Trizol-isolated RNA was then subjected to another purification step using the Qiagen RNeasy column, as per the manufacturer's protocol.

RNA concentrations were determined by Nanodrop and all microarray samples were analyzed by Bioanalyzer (Agilent).

### RT-qPCR

100-300ng of total RNA was subjected to reverse transcription using random primers with the High Capacity cDNA Reverse Transcription Kit (ABI, Catalog # 4368814) according to the manufacturer's protocol. Real-time qPCR was performed using Taqman assays. All reactions were run on either the ABI 7900HT instrument or the Fluidigm platform (48.48 or 96.96 format).

### IL-13 Reporter Assay

L-Luc-Beas2B cells (ATCC Cat No. CRL-9609 were grown in GFR matrigel embedded with the following reagents: 12ng/ml IL-13 (R&D Systems, Catalog #213-IL), and anti-IL13 blocking antibody (Genentech). Firefly luciferase activity was measure using the Dual-Glo Luciferase Assay System (Promega, catalog #E2920) according the manufacturer's protocol, with the modification that only Firefly luciferase was measured.

### Microarray Analysis

RNA was amplified and labeled using the Quick Amp labeling kit (Agilent) to generate labeled cRNA from 1ug of total RNA. Experimental samples were labeled with Cy5; Universal Human Reference RNA (Stratagene, La Jolla, CA) was used for the reference channel and was labeled with Cy3. Cy5 and Cy3 labeled cRNA was competitively hybridized to the two-color Whole Human Genome 4 × 44K gene expression microarray platform. Hybridized microarrays were washed according to the manufacturer's protocol (Agilent) and all feature intensities were collected using the Agilent Microarray Scanner. TIFF images of scanned slides were analyzed using Feature Extraction Software (Agilent), protocol GE2-v5_95 (Agilent). Flagged outliers were not included in any subsequent analyses. All data are reported as log₂ values of the dye-normalized Cy5/Cy3 ratios. The log2 ratios of all samples were normalized to the average log2 ratios of the corresponding mock-treated samples (or non-IPF controls).

When different expression profiling datasets were compared, publicly available datasets were first filtered, normalized and centered as in the original study. The Kaminski dataset (GSE10667) was run on whole human genome Agilent microarray platform, therefore, platform specific probe identifiers were used to connect the datasets. Heatmaps were generated with Java Treeview.

Differentially expressed genes were identified by building a linear model that incorporated three factors: diagnosis, source of tissue and sex (inferred from expression analysis of Y-linked genes) in R-code (LIMMA).

### RESULTS

### I113Rα2 is the most differentially expressed gene in IPF lung tissue

We performed a genome wide transcriptome analysis of biopsied lung tissue from 40 IPF patients and 8 non-IPF control subjects. We isolated RNA from these clinical samples and analyzed transcripts using Whole Human Genome Agilent microarrays (GEO accession ID). Limited available metadata [sex, tissue source (biopsy or tissue harvested at time of lung transplant), diagnosis] were included in a linear model used to identify differentially expressed genes.

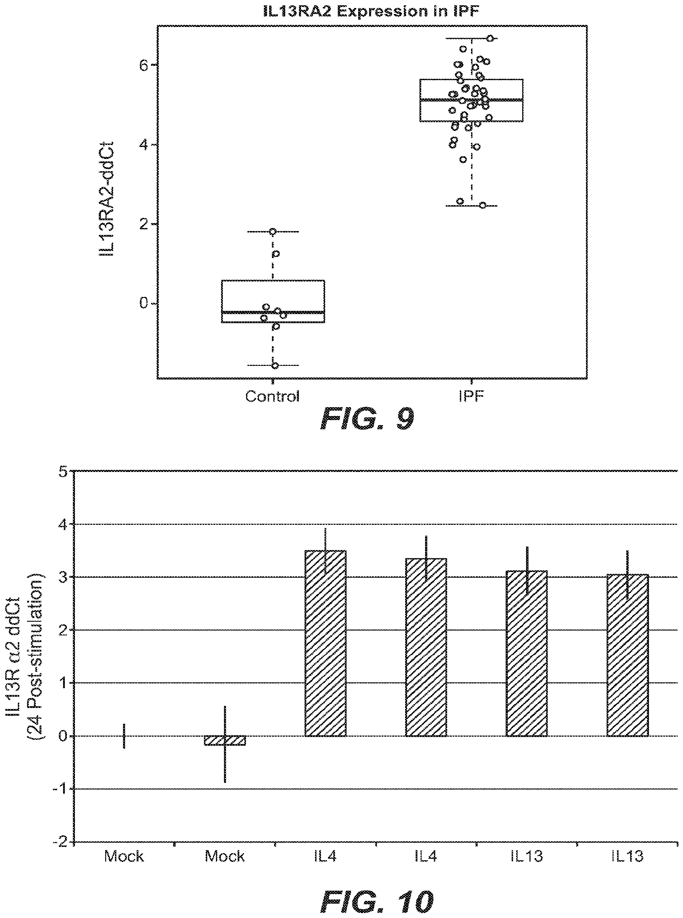

We identified 1508 genes as differentially expressed (q<0.05, fold-change > |2|) in IPF tissues compared to control tissues. A partial list is shown in Table 10. Among the significantly upregulated genes in IPF were matrix metalloproteinases (MMP1, 3, 7, 10, 11, 12, 13, 16), collagens (COL15A, 10A1, 8A2, 1A1, 24A1, 6A3, 7A1, 1A2, 5A2, 3A1, 17A1, 14A1) and IL-13 pathway genes (IL-13, IL13Rα2, POSTN, CCL13). In fact, the most significantly elevated gene in IPF was IL13Rα2, which displayed a 44-fold change (q = 3.4 x 10-14). We verified this observation by performing quantitative RT-PCR (Figure 9).

**Table 10. Partial list of differentially expressed genes (> 1.5-fold up-regulated, q<0.05) associated with IL-13 signaling in IPF.**

| **ProbeID** | **Gene Symbol** | **Gene Name** | **Entrez ID** | **LogFC** | **p-value** | **adj. p-value** |
|---|---|---|---|---|---|---|
| A_23_P85209 | IL13RA2 | interleukin 13 receptor, alpha 2 | 3598 | 5.46608533 | 1.06E-17 | 5.45E-14 |
| A_23_P251031 | IL13 | interleukin 13 | 3596 | 3.60162356 | 8.56E-06 | 1.14E-04 |
| A_23_P55632 | SERPINB3 | serpin peptidase | 6317 | 3.73336698 | 0.00023029 | 1.71E-03 |
| | | inhibitor, clade B (ovalbumin), member 3 | | | | |
| A_24_P347411 | POSTN | periostin, osteoblast specific factor | 10631 | 2.10394126 | 5.96E-07 | 1.35E-05 |
| A_24_P125335 | CCL13 | chemokine (C-C motif) ligand 13 | 6357 | 1.10440762 | 0.00986751 | 3.53E-02 |
| A_23_P70818 | SMO | smoothened homolog (Drosophila) | 6608 | 1.44870366 | 1.65E-11 | 2.81E-09 |
| A_23_P105251 | GLI1 | GLI family zinc finger 1 | 2735 | 2.08669039 | 1.87E-10 | 1.96E-08 |
| A_23_P500034 | GLI2 | GLI family zinc finger 2 | 2736 | 1.57463899 | 8.12E-09 | 3.98E-07 |
| A_24_P398572 | IGF1 | insulin-like growth factor 1 (somatomedin C) | 3479 | 1.70343868 | 0.00081381 | 4.79E-03 |
| A_23_P153571 | IGFL2 | IGF-like family member 2 | 147920 | 4.11862384 | 5.27E-08 | 1.90E-06 |
| A_23_P102113 | WNT10A | wingless-type MMTV integration site family, member 10A | 80326 | 2.83702687 | 1.14E-17 | 5.45E-14 |

| | | | | | | |
|---|---|---|---|---|---|---|
| ProbeID = the identification number of the probe on the Agilent Whole Human Genome Microarray; Gene Symbol = NCBI Entrez Gene Symbol; Gene Name = NCBI Entrez Gene Name; ENTREZ ID = NCBI Entrez Gene ID; logFC = mean of the log base 2 expression levels in IPF samples minus the mean of the log base 2 expression levels in healthy control samples; p-value = the nominal p-value of effect significance of the diagnosis term in the linear model from the limma analysis; adj. p-value = the estimated false discovery rate at the largest p-value for which the gene would be statistically significant. | | | | | | |

### IL13Rα2 expression is induced by IL-13 in cultured primary lung fibroblasts

As discussed above, IL13Rα2 was the mostly highly differentially expressed gene in IPF samples as compared to the control, non-IPF samples, exhibiting a mean 44-fold greater expression in IPF than in controls. We sought to develop a tractable and relevant pulmonary tissue culture system to better understand the role of IL13Rα2. Primary lung fibroblast cells (IMR90) were grown on growth-factor reduced (GFR) matrigel to simulate a more physiological growth substrate than plastic. (Unless otherwise stated, all culture experiments in this Example were performed with cells grown on GFR-matrigel.) IMR90 cells stimulated with IL-13 or IL-4 led to a substantial (∼8-10 fold) induction of IL13Rα2 (Figure 10). Thus, this provides an experimental system to study the function of endogenously induced, rather than ectopically overexpressed IL13Rα2 using blocking antibodies and small molecule inhibitors.

### IL13Rα2 induction attenuates signals from IL13Rα1/IL4Rα in primary lung fibroblasts

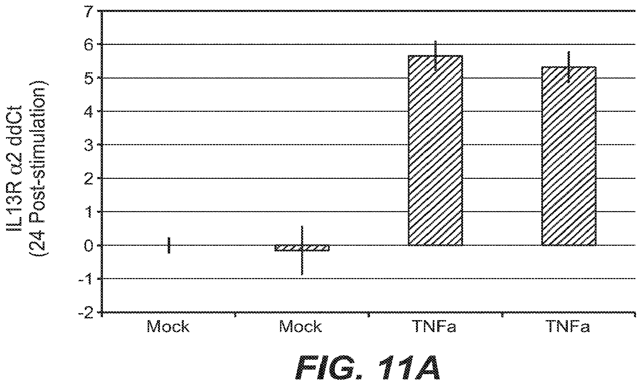

IL13Rα2 has a short cytoplasmic tail that lacks canonical signaling domains. Some evidence supports the model that the extracellular domain of IL13Rα2, which can bind IL-13, functions as a decoy to the IL13Rα1/IL4Rα receptor complex and attenuates STAT6-dependent IL-13 signals. During the development of the tissue culture system, we observed that TNFα stimulation induced IL13Rα2 expression in IMR90 cells (Figure 11A). Although TNFα has been shown to potentiate IL-13 dependent IL13Rα2 induction, TNFα treatment alone has not been shown to upregulate IL13Rα2 in other systems. One key difference in this culture system is that cells were grown on Matrigel. To test the possibility that IL-13 was present in the GFR matrigel, we performed a sensitive IL-13 bioassay, which showed that IL-13 was not detectable in cells cultured on growth factor reduced Matrigel (data not shown).

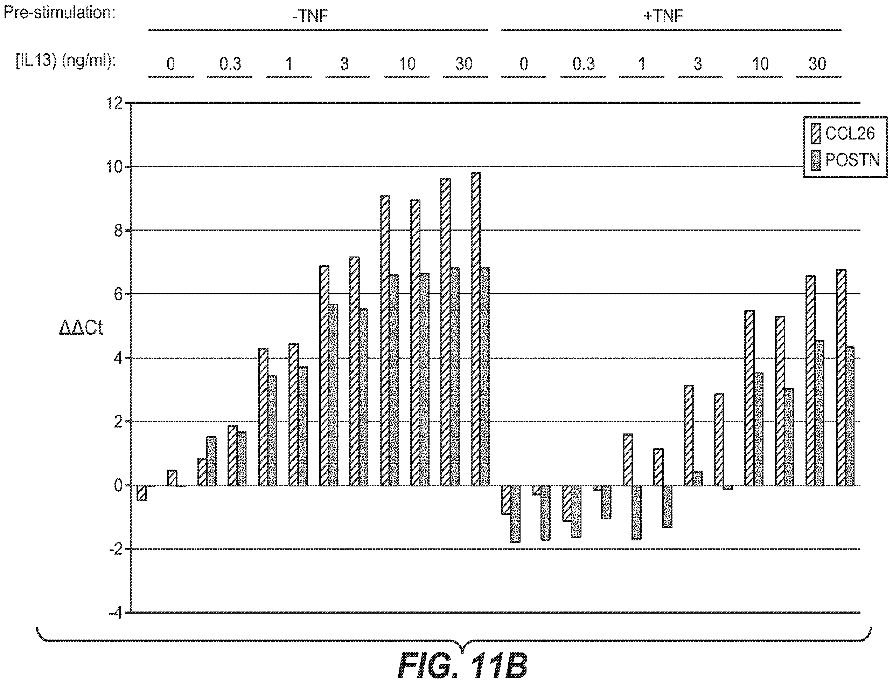

TNFα-stimulated IMR90 cells displaying elevated IL13Rα2 expression provided an opportunity to investigate the effect of elevated IL13Rα2 expression on events downstream of IL-13 stimulation. We monitored dose responsiveness to IL-13 and IL-4 by measuring the transcript levels of known STAT6 responsive genes (CCL26 and periostin) whose expression was not affected by TNFα treatment (Figure 11B). Cells that had been pre-stimulated with TNFα, which resulted in increased IL13Rα2 expression, displayed reduced sensitivity to IL-13, but not IL-4 (data not shown), as assessed by CCL26 and periostin gene induction (Figure 11B), which could be overcome by increasing the dose of IL-13. Nearly tenfold higher concentrations of IL-13 were required to achieve the same level of CCL26 or periostin gene induction by IL-13 in cells that been pre-stimulated with TNFα and display elevated IL13Rα2 expression, while sensitivity to IL-4 stimulation (data not shown) was unaltered by TNFα pretreatment. Taken together, these data show that TNFα-mediated IL13Rα2 induction specifically attenuates the ability of IL-13 to bind to and signal through the IL13Rα1/IL4Rα receptor complex, consistent with a role for IL13Rα2 as a decoy receptor.

### Different anti-IL13 antibodies selectively block binding to IL13Rα1/IL4Rα or IL13Rα2

Although the above data are consistent with a model in which IL13Rα2 is a decoy receptor with respect to IL13Rα1/IL4Rα signaling, the possibility remains that alternative downstream signals originate from IL13Rα2. We took an unbiased, gene expression profiling approach to identify potential IL13Rα2 signals by using specific blocking antibodies that disrupt signaling from specific IL13/IL4 receptor complexes. One antibody (anti-IL-13 mAb1) blocks the ability of IL-13 to recruit IL4Rα to IL13Rα1 but does not affect IL-13 binding to IL13Rα2; the second antibody (anti-IL-13 mAb2) blocks the ability of IL-13 to bind to both IL13Rα1 and IL13Rα2. While both antibodies can prevent IL13Rαl/IL4Rα-dependent STAT6 activation without affecting IL-4 signaling through the IL13Rα1/IL4Rα receptor complex, only anti-IL-13 mAb2 can also prevent IL-13 binding to IL13Rα2. To evaluate the activity of each of these antibodies in the primary fibroblast culture system, cells were treated with IL-13 and/or IL-4 in the presence or absence of the mAb1 or mAb2 blocking anti-IL-13 antibodies. As described below, we measured transcript levels of CCL26 and periostin, which are markers of IL-13/IL-4 signaling downstream of STAT6.

We first characterized the gene expression profiles induced by IL-13 or IL-4 alone in the primary pulmonary fibroblast culture system. Cells were stimulated with either 10 ng/ml IL-13 or 3 ng/ml IL-4 for 48hr, after which RNA was extracted and subjected to genome-wide expression profiling. The profiles induced by each cytokine displayed nearly identical gene expression changes relative to the mock treated cells (data not shown). We identified no statistically significant differences between IL-4 and IL-13 stimulated conditions, strongly suggesting that both cytokines activate identical signaling pathway(s) downstream of the IL13Rα1/IL4Rα receptor complex.

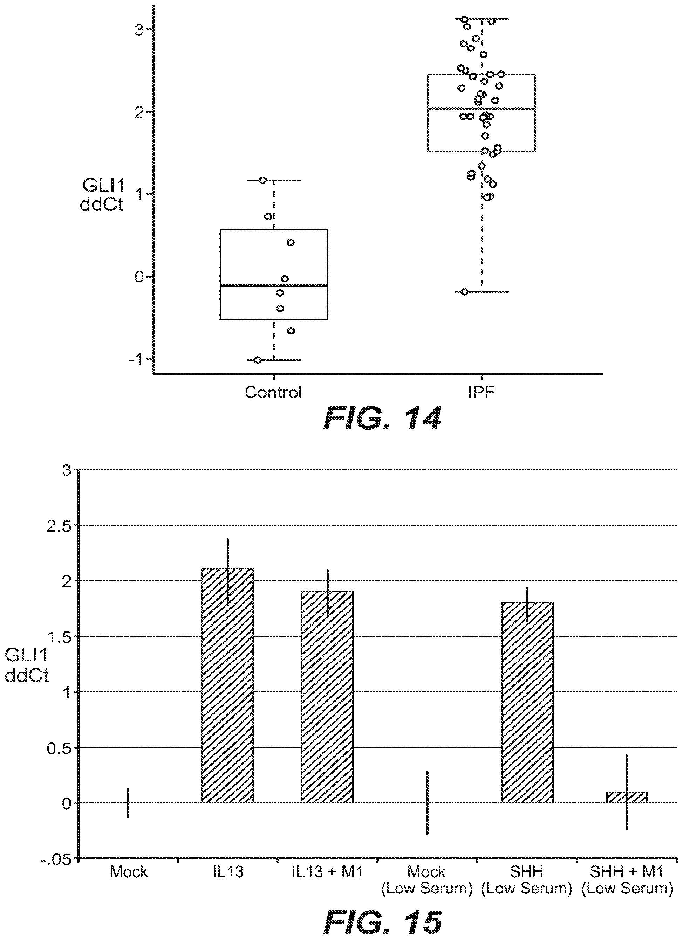

We next measured the transcript levels of CCL26 and periostin (POSTN) in the IMR90 primary pulmonary fibroblast culture system (pre-stimulated with TNFα) under various conditions of cytokine treatment with and without blocking anti-IL-13 antibodies mAb1 and/or mAb2. In Figure 14, the various treatment conditions are indicated in the figure along with the gene expression results for each condition.

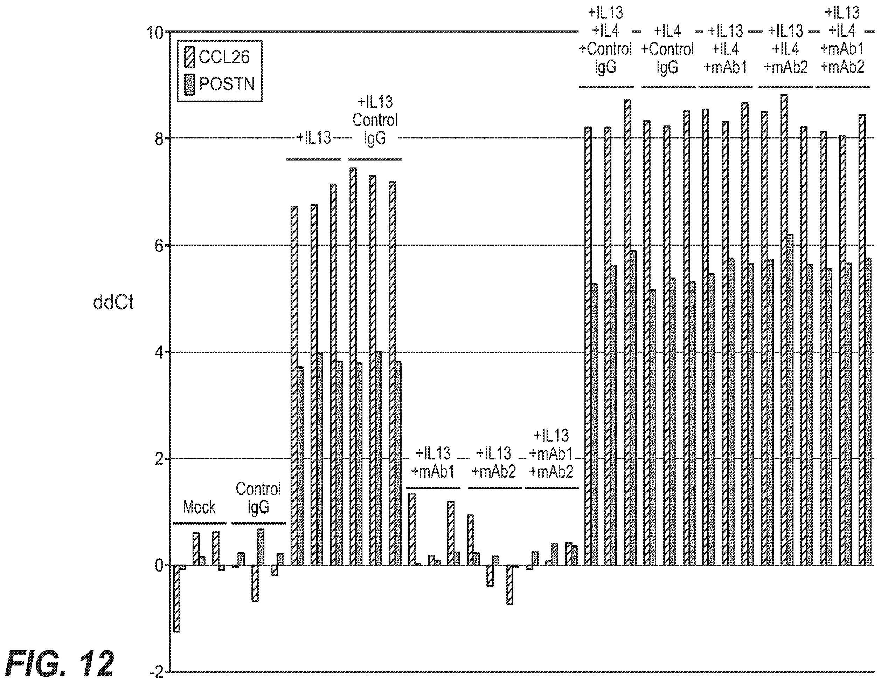

As IL13Rα2 is endogenously upregulated in response to prior signals suggesting its role is to modulate subsequent IL-13 signaling, for the experimental results shown in Fig. 12, we induced IL13Rα2 expression by pre-treating the cells with TNFα prior to secondary stimulation. Upon stimulation with IL-4 or sufficiently high levels of IL-13 to overcome the negative regulatory effects of IL13Rα2, CCL26 and periostin transcripts were significantly induced (i.e. CCL26 ∼ > 100x; periostin ∼ >10x) (Fig. 12). When cells were treated with IL-13 and either anti-IL-13 mAb1 or anti-IL-13 mAb2, or the combination of mAbs, the induction of CCL26 and periostin was almost completely abrogated (Figure 12). However, neither anti-IL-13 mAb1 nor anti-IL-13 mAb2 blocked IL-4-mediated induction of CCL26 or periostin (Figure 12), consistent with the selectivity of each mAb for IL-13 over IL-4. By measuring CCL26 and periostin expression levels, no discernable differences could be detected by blocking IL13Rα1 (with anti-IL-13 mAb1) versus blocking both IL13Rα1 and IL13Rα2 (with anti-IL-13 mAb2).

We next sought to assess the contribution of IL13Rα2 to the overall gene expression profile. One concern we had when setting up these experiments was the possibility that IL13Rα2 signaling would require a simultaneous co-signal emanating from the IL4Rα/IL13Rα1 receptor complex. Because IMR90 cells lack the common gamma chain (IL2Ry, data not shown), IL-4-mediated signaling occurs only via activation of the IL4Rα/IL13Rα1 receptor complex. Accordingly, treating IMR90 cells with the cytokine IL-4 will activate signaling through IL4Rα/IL13Rα1 in a manner analogous to treatment with IL-13 and thus, IL-4 treatment of these cells is a surrogate for IL-13 signaling through the IL4Rα/IL13Rα1 receptor complex.

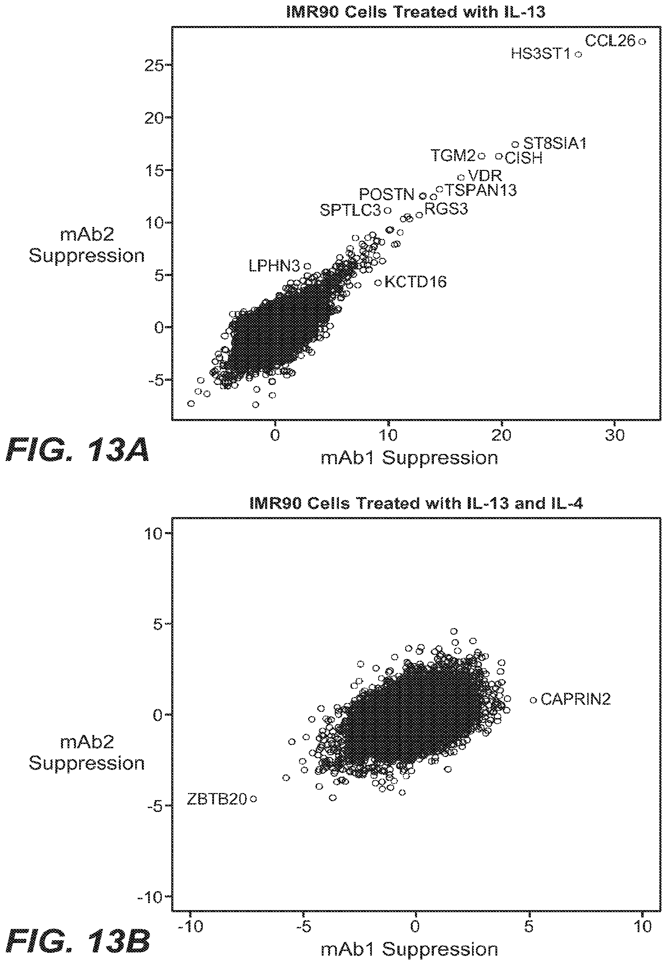

Figure 13A shows an analysis of the microarray data from cells treated with IL-13 or IL-13 and IL-4 and in each case with either mAb1 or mAb2. As can be seen, this analysis revealed no significantly differentially expressed genes (adjusted p-value < 0.01) between treatment of the cells with IL-13 and either mAb1 or mAb2 nor between treatment of the cells with IL-13 and IL-4 and either mAb1 or mAb2 (in all cases cells were pre-treated with TNFα). In IL-13-stimulated cells in the absence of IL-4, the suppression by mAb1 and mAb2 was nearly identical (Figure 13A); whereas in the presence of IL-4, neither mAb1 nor mAb2 resulted in meaningful changes in gene expression (Figure 13B). Taken together, these data suggest that ligand-receptor IL13-IL13Rα2 engagement does not induce signaling events that result in gene expression changes.

### Elevated GLI1 expression in IPF and in IL13/IL4 stimulated primary lung fibroblasts

Among the most significantly expressed genes in the IPF cohort was GLI1 (Figure 14), a critical transcription factor in developmental pathways. Hedgehog (Hh) and TGFβ signaling have been shown to induce GLI1 expression. GLI1 and IL13Rα2 expression levels were significantly correlated within IPF samples (rs = 0.69, q-value < 0.01). In cell culture microarray experiments, we found that GLI1 expression was upregulated by IL13 stimulation (data not shown). Thus, we hypothesized that GLI1, like IL13Rα2, may be regulated directly or indirectly by IL13 signaling. To confirm this observation, we stimulated primary pulmonary fibroblasts (cultured on matrigel) with IL13 and measured GLI1 expression by RT-qPCR. GLI1 expression was induced (∼4x) by IL13 stimulation (data not shown).

### Blockade of Hh or TGFβ signaling does not abrogate IL13 induced GLI1 expression

Because Hh pathway activity can induce GLI1 expression, we hypothesized that IL13 stimulation leads to Hh-dependent autocrine/paracrine stimulation of the Hh pathway. To test this possibility, we used a small molecule Hh pathway inhibitor that blocks the ability of Hh ligands to induce smoothened derepression via patched binding (referred to herein as M1) (Yauch et al., Nature 455:406-410 (2008), which refers to Cur-691 or HhAntag691). Pretreatment with M1 blocked SHH mediated GLI1 induction, but did not affect IL13 dependent GLI1 induction (Figure 15).

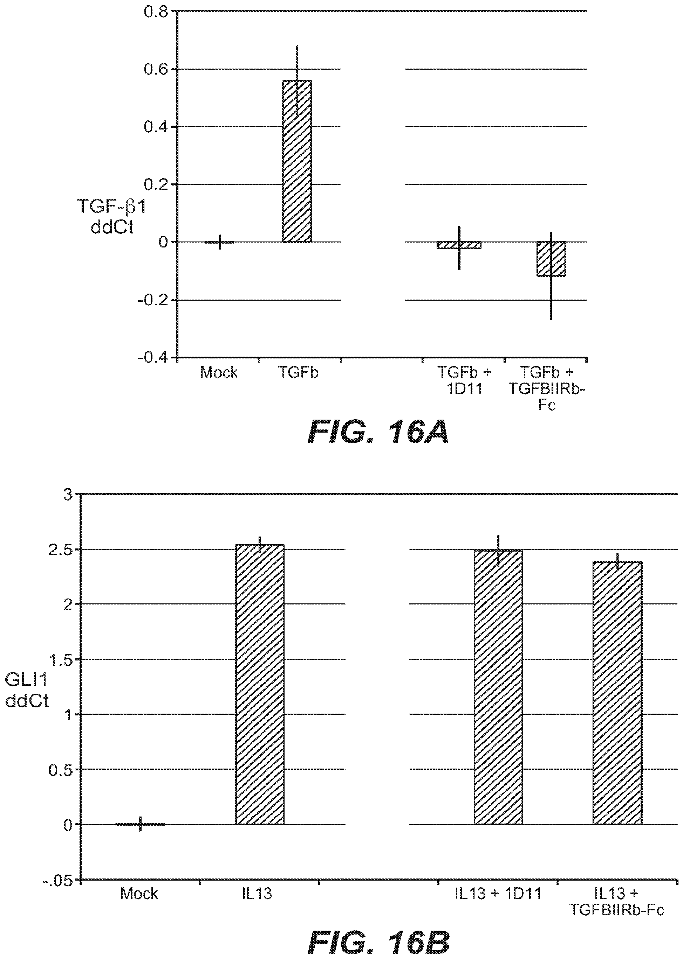

Stimulation of cells by TGFβ has been shown to induce GLI1 expression, and IL13 can induce TGFβ activity in some experimental systems. To evaluate whether IL13-dependent induction of GLI1 occurs via a TGFβ dependent autocrine/paracrine mechanism, cells were pre-treated with two TGFβ pathway inhibitors (antibody 1D11 [Edwards et al., J.of Bone and Mineral Res. 25:2419-2426, 2010] and TGFBIIRb-Fc [R&D Systems, Cat. No. 1003-RT-050). While both TGFβ pathway inhibitors blocked TGFβ-mediated induction of TGFβ1 (a known TGFβ responsive gene) (Fig. 16A), neither affected IL13 dependent GLI1 induction (Fig. 16B).

These data suggest a previously undescribed relationship between IL13 and GLI1 induction, which is not mediated by previously characterized Hh and TGFβ signaling pathways.

Gene expression analysis of lung tissues reveals a remarkably altered transcriptome in IPF patients when contrasted to non-IPF controls. Consistent with the fibrotic diathesis, we found that many genes involved in remodeling the extracellular matrix were expressed at elevated levels in IPF. Furthermore, the remarkable degree of transcriptome-wide similarity between the present study and previously published expression microarray data from a similarly sized study supports our conclusions that the gene expression patterns observed in this study are broadly indicative of processes that are legitimately active in human IPF rather than false signals due to misdiagnosis or technical or analytical artifacts.

IL13 has been implicated in preclinical models as a potential driver of fibrosis. In IPF biopsies, we observed transcriptional evidence of IL13 pathway activity, with elevated expression of previously described IL13 target genes including periostin, CCL13, CCL18 and IL13Rα2; furthermore, IL13 itself was expressed at elevated levels in IPF. The most strongly and consistently upregulated gene in IPF was IL13Rα2. Previous studies have reached different conclusions regarding the role of IL13Rα2, namely that 1) IL13Rα2 can transduce signals that may support increased fibrosis or 2) IL13Rα2 is a decoy receptor for IL13 and has no signaling capabilities. Given these contrasting possibilities, it is challenging to determine whether the IL13-related gene expression patterns in our biopsy microarray data indicates that the high expression of IL13Rα2 reflects a net positive or net negative role for IL13 activity in IPF, and what the likely function of IL13Rα2 is.

To elucidate the role of IL13Rα2 in a tractable system that is relevant to IPF, we developed an *in vitro* system using IMR90 cells (a human primary pulmonary fibroblast cell line) grown on growth factor reduced Matrigel. When stimulated with IL13, these cells displayed increased expression of IL13Rα2. We observed that IL13Rα2 was also strongly inducible by TNFα in this system. Because TNFα is not known to signal through STAT6, we exploited this feature to endogenously, rather than ectopically modulate IL13Rα2 levels and determine the impact of IL13Rα2 expression on IL13 signaling. When cells were pre-stimulated with TNFα, they exhibited decreased sensitivity to IL13 stimulation, as assessed by two IL13 target genes (periostin and CCL26); cells pre-stimulated with TNFα required approximately ten times the IL13 concentration to achieve comparable periostin and CCL26 induction relative to cells not pre-stimulated with TNFα. However, TNFα pre-stimulation did not appear to affect the intrinsic signaling capacity of the IL13Rα1/IL4Rα complex, as the cells remained similarly sensitive to IL4 regardless of TNFα pre-treatment. These observations supported a model in which IL13Rα2 acts as a decoy receptor for IL13-IL13Rα1/IL4Rα interactions; however, these data do not exclude that possibility that IL13Rα2 could give rise to a unique, intracellular signal of its own. To investigate that possibility more thoroughly, we took advantage of reagents that allowed selective blockade of IL13's interactions with the two receptor complexes . MAb2 is a blocking antibody that disrupts IL13's ability to bind to both IL13Rα1 and IL13Rα2. MAb1, however, blocks IL13's ability to bind to and signal via IL4Rα but does not block IL13Rα2 binding. We reasoned that by comparing the expression profiles of cells treated with IL13 in the presence of either one of these blocking antibodies, we would identify IL13Rα2-specific downstream transcriptional events, if any. We were unable to identify a single statistically significant difference between conditions in which cells were blocked with either antibody, which strongly suggests that in this cell culture system, IL13Rα2 engagement by IL13 alone does not give rise to an intracellular signaling cascade that impacts gene expression. Because IL13-IL13Rα2 binding likely occurs concurrently with IL13 engagement of IL13Rα1/IL4Rα *in vivo,* we sought to determine whether an IL13Rα2 signal may be dependent on simultaneous engagement of the IL13Rα1/IL4Rα complex. However, a reagent that selectively blocks IL13-IL13Rα2 interactions without affecting IL13-IL13Rα1/IL4Rα signaling has not been identified. Because IL13 and IL4 both bind to and signal via the IL13Rαl/IL4Rα signaling complex and give rise to identical expression profiles, we reasoned that we could mimic an active IL13Rα1/IL4Rα signal by introducing IL4 present to the MAb1 vs MAb2 blocking experiment as described above. In this context, we were still unable to identify any IL13Rα2-dependent signaling events. Taken together, these data strongly support a model in which IL13Rα2 is a decoy receptor in pulmonary fibroblasts.

We have also shown that GLI1, known to be important in lung development, EMT, tumorigenesis and tissue repair is up-regulated in IPF patients relative to control subjects. We also observed that IL13 lead to an increase in GLI1 expression in cultured fibroblasts. These results suggest that GLI1 may support continued growth and survival of fibroblasts, which may block the final stages of wound healing resolution and support continued fibrosis.

### Example 3: Phase II Clinical Study

### Study Rationale

IPF is characterized by varying degrees of interstitial fibrosis. Several extracellular matrix proteins, including type I, III, and IV collagens, fibronectin, and tenascin-C, are involved in the process of fibrosis in IPF patients, together with abnormal proliferation of mesenchymal cells, distortion of pulmonary architecture, and generation of subepithelial fibroblastic foci. IL-13 and IL-4 are strong inducers of tissue fibrosis. In nonclinical models, transgenic overexpression of IL-13 in the lungs of mice is sufficient to induce collagen gene expression and profound subepithelial fibrosis (Lee at al. 2001, J Exp Med 194:809-22; Zhu et al. 1999, J Clin Invest 103:779-88). Conversely, mice with targeted disruption of IL-13 and mice that are treated with blocking antibodies specific for IL-13 show reduced extracellular matrix deposition in bleomycin- and fluorescein isothiocyanate-induced pulmonary fibrosis models (Belperio et al. 2002, Am J Respir Cell Mol Biol 27:419-27; Kolodsick et al. 2004, J Immunol 172:4068-76; Liu et al. 2004, J Immunol 173:3425-31).

Multiple studies have concluded that expression and activity of IL-13 are elevated in IPF patients. The expression of IL-13 and IL-13 receptors (IL-13Rs) IL-13Rα1 and IL-13Rα2 was found to be increased in lung biopsy samples from IPF patients compared with normal controls, both at the messenger RNA and protein level (Jakubzick et al. 2004, Am J Pathol 164:1989-2001). IL-13 was also found to be elevated in the bronchoalveolar lavage fluid from IPF patients compared with normal controls. Importantly, the level of IL-13 in these samples was negatively correlated with the key measures of lung function, percentage of predicted forced vital capacity (FVC) and diffusion capacity of the lung for carbon monoxide (DLco) (Park et al. 2009, J Korean Med Sci 24:614-20), suggesting pathogenic functions of IL-13 in IPF patients.

In addition to IL-13 itself, serum biomarkers known to be expressed downstream from IL-13 signaling have also been shown to be elevated in IPF patients. Periostin, an IL-13-inducible protein with a serum level that correlates with benefit from treatment with lebrikizumab in asthma patients (Corren et al. 2011, N Engl J Med 365(12):1088-98), is also elevated in the serum of IPF patients, and these levels have been shown to be negatively correlated with pulmonary function parameters (FVC and DLco) over a 6-month period (Okamoto et al. 2011, Eur Respir J 37:1119-27). Periostin has been proposed to be a pathogenic factor in IPF on the basis of reduced bleomycin-induced pulmonary fibrosis observed in periostin-deficient mice (Uchida et al. 2012, Am J Respir Cell Mol Biol 46:677-86), suggesting an additional mechanism by which IL-13 may contribute to disease. IL-13 signaling also induces robust production of chemokine (C-C motif) ligand 18 (CCL-18) from macrophages in vitro, and alveolar macrophages isolated from IPF patients, but not from normal controls, constitutively express CCL-18 protein (Prasse et al. 2006, Am J Respir Crit Care Med 173:781-92). Like periostin, CCL-18 is increased in the serum of IPF patients, and CCL-18 levels are reported to correlate with disease progression and prognosis, with patients that have higher levels of baseline CCL-18 experiencing a greater decline in FVC and higher mortality rates (Prasse et al. 2007, Arthritis Rheum 56:1685-93; Prasse et al. 2009, Am J Respoir Crit Care Med 179:717-23). Taken together, these data strongly suggest that IL-13 expression is elevated in IPF patients and that signals from this cytokine to multiple fibrosis-relevant cell types play a key role in driving disease pathogenesis.

### Anti-IL13 antibody (lebrikizumab) amino acid sequences

The table below shows the amino acid sequences of the CDR-H1, CDR-H2, CDR-H3, CDR-L1, CDR-L2, and CDR-L3 regions of lebrikizumab, along with VH, VL, heavy chain sequences and light chain sequences. As indicated in Table 11 below, VH and the heavy chain may include an N-terminal glutamine and the heavy chain may also include a C-terminal lysine. As is well known in the art, N-terminal glutamine residues can form pyroglutamate and C-terminal lysine residues can be clipped during manufacturing processes.

**Table 11. Anti-IL13 antibody (lebrikizumab) amino acid sequences**

| | |
|---|---|
| CDR-H1 | Ala Tyr Ser Val Asn |
| (SEQ ID NO.: 1) | |
| CDR-H2 | Met Ile Trp Gly Asp Gly Lys Ile Val Tyr Asn Ser Ala Leu Lys Ser |
| (SEQ ID NO.:2) | |
| CDR-H3 | Asp Gly Tyr Tyr Pro Tyr Ala Met Asp Asn |
| (SEQ ID NO.: 3) | |
| CDR-L1 | Arg Ala Ser Lys Ser Val Asp Ser Tyr Gly Asn Ser Phe Met His |
| (SEQ ID NO.: 4) | |
| CDR-L2 | Leu Ala Ser Asn Leu Glu Ser |
| (SEQ ID NO.: 5) | |
| CDR-L3 | Gln Gln Asn Asn Glu Asp Pro Arg Thr |
| (SEQ ID NO.: 6) | |
| VH | Val Thr Leu Arg Glu Ser Gly Pro Ala Leu Val Lys Pro Thr Gln Thr Leu Thr Leu Thr Cys Thr Val Ser Gly Phe Ser Leu Ser Ala Tyr Ser Val Asn Trp Ile Arg Gln Pro Pro Gly Lys Ala Leu Glu Trp Leu Ala Met Ile Trp Gly Asp Gly Lys Ile Val Tyr Asn Ser Ala Leu Lys Ser Arg Leu Thr Ile Ser Lys Asp Thr Ser Lys Asn Gln Val Val Leu Thr Met Thr Asn Met Asp Pro Val Asp Thr Ala Thr Tyr Tyr Cys Ala Gly Asp Gly Tyr Tyr Pro Tyr Ala Met Asp Asn Trp Gly Gln Gly Ser Leu Val Thr Val Ser Ser Gln Val Thr Leu Arg Glu Ser Gly Pro Ala Leu Val Lys Pro Thr Gln Thr Leu Thr Leu Thr Cys Thr Val Ser Gly Phe Ser Leu Ser Ala Tyr Ser Val Asn Trp Ile Arg Gln Pro Pro Gly Lys Ala Leu Glu Trp Leu Ala Met Ile Trp Gly Asp Gly Lys Ile Val Tyr Asn Ser Ala Leu Lys Ser Arg Leu Thr Ile Ser Lys Asp Thr Ser Lys Asn Gln Val Val Leu Thr Met Thr Asn Met Asp Pro Val Asp Thr Ala Thr Tyr Tyr Cys Ala Gly Asp Gly Tyr Tyr Pro Tyr Ala Met Asp Asn Trp Gly Gln Gly Ser Leu Val Thr Val Ser Ser Asp Ile Val Met Thr Gln Ser Pro Asp Ser Leu Ser Val Ser Leu Gly Glu Arg Ala Thr Ile Asn Cys Arg Ala Ser Lys Ser Val Asp Ser Tyr Gly Asn Ser Phe Met His Trp Tyr Gln Gln Lys Pro Gly Gln Pro Pro Lys Leu Leu Ile Tyr Leu Ala Ser Asn Leu Glu Ser Gly Val Pro Asp Arg Phe Ser Gly Ser Gly Ser Gly Thr Asp Phe Thr Leu Thr Ile Ser Ser Leu Gln Ala Glu Asp Val Ala Val Tyr Tyr Cys Gln Gln Asn Asn Glu Asp Pro Arg Thr Phe Gly Gly Gly Thr Lys Val Glu Ile Lys Arg VTLRESGPA LVKPTQTLTL TCTVSGFSLS AYSVNWIRQP PGKALEWLAM IWGDGKIVYN SALKSRLTIS KDTSKNQVVL TMTNMDPVDT ATYYCAGDGY YPYAMDNWGQ GSLVTVSSAS TKGPSVFPLA PCSRSTSEST AALGCLVKDY FPEPVTVSWN SGALTSGVHT FPAVLQSSGL YSLSSVVTVP SSSLGTKTYT CNVDHKPSNT KVDKRVESKY GPPCPPCPAP EFLGGPSVFL FPPKPKDTLM ISRTPEVTCV VVDVSQEDPE VQFNWYVDGV EVHNAKTKPR EEQFNSTYRV VSVLTVLHQD WLNGKEYKCK VSNKGLPSSI EKTISKAKGQ PREPQVYTLP PSQEEMTKNQ VSLTCLVKGF YPSDIAVEWE SNGQPENNYK TTPPVLDSDG SFFLYSRLTV DKSRWQEGNV FSCSVMHEAL HNHYTQKSLS LSLG |
| (SEQ ID NO.: 7) | |
| VH | |
| (SEQ ID NO.: 8) | |
| VL | |
| (SEQ ID NO.: 9) | |
| H Chain | |
| (SEQ ID NO.: 10) | |
| H Chain | QVTLRESGPA LVKPTQTLTL TCTVSGFSLS AYSVNWIRQP PGKALEWLAM IWGDGKIVYN SALKSRLTIS KDTSKNQVVL TMTNMDPVDT ATYYCAGDGY YPYAMDNWGQ GSLVTVSSAS TKGPSVFPLA PCSRSTSEST AALGCLVKDY FPEPVTVSWN SGALTSGVHT FPAVLQSSGL YSLSSVVTVP SSSLGTKTYT CNVDHKPSNT KVDKRVESKY GPPCPPCPAP EFLGGPSVFL FPPKPKDTLM ISRTPEVTCV VVDVSQEDPE VQFNWYVDGV EVHNAKTKPR EEQFNSTYRV VSVLTVLHQD WLNGKEYKCK VSNKGLPSSI EKTISKAKGQ PREPQVYTLP PSQEEMTKNQ VSLTCLVKGF YPSDIAVEWE SNGQPENNYK TTPPVLDSDG SFFLYSRLTV DKSRWQEGNV FSCSVMHEAL HNHYTQKSLS LSLG |
| (SEQ ID NO.: 11) | |
| H Chain | VTLRESGPA LVKPTQTLTL TCTVSGFSLS AYSVNWIRQP PGKALEWLAM IWGDGKIVYN SALKSRLTIS KDTSKNQVVL TMTNMDPVDT ATYYCAGDGY YPYAMDNWGQ GSLVTVSSAS TKGPSVFPLA PCSRSTSEST AALGCLVKDY FPEPVTVSWN SGALTSGVHT FPAVLQSSGL YSLSSVVTVP SSSLGTKTYT CNVDHKPSNT KVDKRVESKY GPPCPPCPAP EFLGGPSVFL FPPKPKDTLM ISRTPEVTCV VVDVSQEDPE VQFNWYVDGV EVHNAKTKPR EEQFNSTYRV VSVLTVLHQD WLNGKEYKCK VSNKGLPSSI EKTISKAKGQ PREPQVYTLP PSQEEMTKNQ VSLTCLVKGF YPSDIAVEWE SNGQPENNYK TTPPVLDSDG SFFLYSRLTV DKSRWQEGNV FSCSVMHEAL HNHYTQKSLS LSLGK |
| (SEQ ID NO.: 12) | |
| H Chain | QVTLRESGPA LVKPTQTLTL TCTVSGFSLS AYSVNWIRQP PGKALEWLAM IWGDGKIVYN SALKSRLTIS KDTSKNQVVL TMTNMDPVDT ATYYCAGDGY YPYAMDNWGQ GSLVTVSSAS TKGPSVFPLA PCSRSTSEST AALGCLVKDY FPEPVTVSWN SGALTSGVHT FPAVLQSSGL YSLSSVVTVP SSSLGTKTYT CNVDHKPSNT KVDKRVESKY GPPCPPCPAP EFLGGPSVFL FPPKPKDTLM ISRTPEVTCV VVDVSQEDPE VQFNWYVDGV EVHNAKTKPR EEQFNSTYRV VSVLTVLHQD WLNGKEYKCK VSNKGLPSSI EKTISKAKGQ PREPQVYTLP PSQEEMTKNQ VSLTCLVKGF YPSDIAVEWE SNGQPENNYK TTPPVLDSDG SFFLYSRLTV DKSRWQEGNV FSCSVMHEAL HNHYTQKSLS LSLGK |
| (SEQ ID NO.: 13) | |
| L Chain | DIVMTQSPDS LSVSLGERAT INCRASKSVD SYGNSFMHWY QQKPGQPPKL LIYLASNLES GVPDRFSGSG SGTDFTLTIS SLQAEDVAVY YCQQNNEDPR TFGGGTKVEI KRTVAAPSVF IFPPSDEQLK SGTASVVCLL NNFYPREAKV QWKVDNALQS GNSQESVTEQ DSKDSTYSLS STLTLSKADY EKHKVYACEV THQGLSSPVT KSFNRGEC |
| (SEQ ID NO.: 14) | |

### Dose, Dosing Regimen and Rationale

This is a randomized, multicenter, double-blind, placebo-controlled, parallel-group study of lebrikizumab in patients with IPF. Approximately 250 patients (125 patients per treatment group) will be enrolled in the study at approximately 100 sites located globally. The total treatment duration will be based on all subjects receiving at least 13 doses (q4wks) of blinded treatment and the observation of at least 75 events for the primary endpoint over a maximum period of 2.5 years.

Patients who provide written informed consent will commence a screening period (≤ 4 weeks) to establish entry criteria. At the end of the screening period, eligible patients will be randomized in a 1:1 ratio to double-blind treatment with subcutaneously injected (SC) lebrikizumab 250 mg or placebo, each administered from a prefilled syringe. Block randomization will be performed centrally and stratified on lung function (FVC < 50%, 50% to 75%, > 75% predicted), and by region (North America, Europe, other).

Study drug will be administered by SC injection every 4 weeks, with the first injection occurring at the randomization visit (Day 1). SC injection will be in the arm, thigh, or abdomen and all patients will receive a total of two injections per dosing visit. Patients will receive a minimum of 13 doses of blinded treatment during a minimum dosing period (Weeks 0 to 48). Patients will continue to receive blinded study treatment every 4 weeks during the extended treatment period until at least 75 events have occurred for the primary endpoint and the last patient enrolled has had the opportunity to receive at least 13 doses of blinded treatment. Once these events have occurred, all ongoing patients will return to the clinic approximately 4 weeks after their last dose of study drug to complete follow-up assessments at the end-of-treatment (EOT) visit and at two subsequent visits during an 18-week safety follow-up period. Depending on enrollment and event frequency rates, the total duration of treatment is expected to range from 1 to a maximum of 2.5 years. The treatment period will not be extended beyond 2.5 years for each patient, and the maximum study duration is expected to be 2.8 years. Patients who choose to prematurely discontinue study drug will be encouraged to remain in the study and complete all remaining assessments. If this is not feasible, the patient should enter and complete the safety follow-up period unless consent has been withdrawn.

The dose and dosing regimen rationale is as follows. Lebrikizumab is a humanized IgG4 monoclonal antibody that inhibits IL-13 signaling. Both nonclinical and clinical data suggest that IL-13 signaling plays an important role in the pathogenesis of IPF (see above). The mechanism of action of lebrikizumab is to block the interaction between IL-13 and its receptor, and hence the intracellular signaling of IL-13. Due to the fast turnover of cytokines, it is hypothesized that optimal IL-13 blockade requires maintaining sustained concentrations of lebrikizumab in the lung. Assuming IL-13 levels in the lung are in the range reported in the literature (200-2000 pg/mL; Hart 2001, Immunology and Cell Biology 79:149-53) and a serum:lung partitioning ratio of 1:500, a serum concentration of 10 µg/mL would be expected to maintain sufficiently high drug levels in the lung to neutralize IL-13. This target concentration is also consistent with the lower end of the observed Week 12 trough concentrations in a Phase II study (mean ± SD: 28.8 ± 11.9 µg/mL), where lebrikizumab showed efficacy in reducing the rate of severe asthma exacerbations in patients whose asthma was uncontrolled despite inhaled corticosteroids therapy. Similar to asthmatics, patients with IPF have elevated levels of biomarkers associated with IL-13 biology, suggesting that the concentrations of lebrikizumab producing clinical benefit in asthma may also produce biological activity in IPF.

For the Phase II study in IPF patients, a 250 mg every 4 week (q4wk) dose was selected to maintain steady state serum trough concentrations at or above this target concentration. Assuming the pharmacokinetics of lebrikizumab in IPF patients is similar to that in asthmatics, this dose/regimen would be expected to maintain a mean steady state trough concentration of -30 µg/mL. A three-fold higher concentration above the target allows for reduced lung partitioning in IPF patients due to a thicker interstitium and potentially faster clearance of lebrikizumab if autoantibodies are present (Papiris et al. 2012, Curr Opin Pulm Med 18:433-40). Selection of a 250 mg dose q4wk is also supported by the safety database from previous clinical studies of lebrikizumab in asthma.

Lebrikizumab and placebo will be supplied in a prefilled syringe. Each syringe is for single-dose SC administration only and contains no preservatives. Each prefilled syringe of active study drug contains 1 mL of sterile liquid at a concentration of 125 mg/mL lebrikizumab. The formulation also contains histidine acetate (20 mM), sucrose (60 mg/mL), polysorbate 20 (approximately 0.03%), and Sterile Water for Injection USP, pH 5.4-6.0. Each prefilled syringe of placebo contains 1 mL of sterile liquid placebo formulation consisting of Sterile Water for Injection with histidine acetate (20 mM), sucrose (75 mg/mL), and polysorbate 20 (approximately 0.03%), pH 5.4-6.0. Prefilled syringes of study drug and placebo should be refrigerated at 2°C-8°C and protected from excessive light and heat. Syringes should not be frozen, shaken, or stored at room temperature.

### Inclusion and Exclusion Criteria

Approximately 250 patients 35-80 years of age who have IPF will be enrolled in this study. The inclusion criteria include the following: diagnosis of definitive or probable IPF, based on the 2011 ATS/ERS guidelines, within the previous 5 years from time of screening and confirmed at baseline; FVC > 40% and ≤ 90% of predicted at screening; DLco > 25% and ≤ 90% of predicted at screening; for patients receiving oral corticosteroid therapy: stable dose ≤ 10 mg prednisone (or equivalent) for ≥ 4 weeks prior to Day 1; ability to walk ≥ 100 meters unassisted. The exclusion criteria include the following: history of a sever allergic reaction or anaphylactic reaction to a biologic agent or known hypersensitivity to any component of the lebrikizumab injection; evidence of other known causes of interstitial lung disease; lung transplant expected within 6 months; evidence of significant obstructive lung disease or other clinically significant lung disease other than IPF or other clinically significant medical disease including infectious diseases; class IV New York Heart Association chronic heart failure or historical evidence of left ventricular ejection fraction < 35%; hospitalization due to an exacerbation of IPF within 30 days prior to screening; body weight < 40 kg.

### Efficacy Outcome Measures

The primary efficacy outcome measure is progression-free survival (PFS), which is defined as the time from study treatment randomization to the first occurrence of any of the following events: death from any cause; non-elective hospitalization from any cause; and a decrease from baseline of ≥ 10% in FVC (L). The secondary efficacy outcome measures are: annualized rate of change in absolute FVC (L); a decrease from baseline of ≥ 15% in DLco (mL CO /min-1/mmHg-1) at Week 52; time from randomization to death or non-elective hospitalization from any cause; change from baseline to Week 52 in the A Tool to Assess Quality of Life in IPF (ATAQ-IPF); time from randomization to first decrease relative to baseline of ≥ 10% in FVC (L); annualized rate of change in absolute DLco (mL CO /min-1/mmHg-1); and change from baseline to Week 52 in 6-minute walk distance (6MWD). Exploratory outcome measures are: change from baseline to Week 52 in FVC ([L] and percent of predicted); percent change from baseline to Week 52 in FVC (L); incidence of a 200 mL or 10% change from baseline to Week 52 in absolute FVC (L); change from baseline to Week 52 in DLco ([mL CO /min-1/mmHg-1] and percent of predicted); percent change from baseline to Week 52 in DLco (mL CO /min-1/mmHg-1); change from baseline to Week 52 in resting oxygen flow rate for patients receiving supplemental oxygen therapy at baseline; time from randomization to addition of supplemental oxygen therapy for patients not receiving supplemental oxygen at baseline; time from randomization to non-elective hospitalization from any cause; time from randomization to first event of acute IPF exacerbation; time from randomization to first event of IPF deterioration; change from baseline to Week 24 and Week 52 in radiographic findings on pulmonary HRCT, including quantitative lung fibrosis (QLF) score; change from baseline in distance walked in the 6-minute walk test (6MWT); change from baseline in serum biomarkers (e.g., periostin, CCL-18, YKL40, COMP, OPN, CCL-13); and change from baseline to Week 52 in the EuroQol 5-Dimension Questionnaire (EQ-5D).

### Study Assessments

### Idiopathic Pulmonary Fibrosis Exacerbation

IPF exacerbation is defined as an event that meets the following criteria: unexplained worsening or development of dyspnea within the previous 30 days; radiologic evidence of new bilateral ground-glass abnormality and/or consolidation, superimposed on a reticular or honeycomb background pattern, that is consistent with usual interstitial pneumonitis (UIP); and absence of alternative causes, such as left heart failure, pulmonary embolism, pulmonary infection (on the basis of endotracheal aspirate or bronchoalveolar lavage, if available, or investigator judgment), or other events leading to acute lung injury (e.g., sepsis, aspiration, trauma, reperfusion pulmonary edema).

### Idiopathic Pulmonary Fibrosis Deterioration of Disease

IPF deterioration of disease is defined as an event that meets the following: (i) unexplained worsening or development of dyspnea within the previous 30 days and (ii) any two of:
- Radiologic evidence of new bilateral ground-glass abnormality and/or consolidation superimposed on a reticular or honeycomb background pattern that is consistent with UIP;
- Deterioration of lung function by meeting at least 1 of the criteria below:
   - FVC (L), by at least 10%
   - DLco (mL CO /min-1/mmHg-1), at least 15%
   - Oxygen saturation (SpO2) at least 4%;

and (iii) absence of alternative causes such as those listed above under IPF exacerbation.

### Spirometry

Spirometry, including the procedure for bronchodilator testing, will be conducted as per the study Pulmonary Function Manual, which is based on the ATS/ERS Consensus Statement (Miller et al. 2005, Eur Respir J 26:319-38). The manual will include information on equipment, procedures, patient instructions, and precautions. Spirometric measures to be collected will include FEV₁ and FVC values and peak expiratory flow values, as well as flow-volume and volume-time curves. Percentage of predicted FEV₁ and FVC will be derived from these volume measurements using the equations derived from the National Health and Nutrition Examination Survey dataset as described by Hankinson and colleagues (Hankinson et al. 1999, Am J Respir Crit Care Med 159:179-87).

Measurement of spirometry will be performed on a computerized spirometry system, configured to the requirements of the study and in accordance with guidelines published by the ATS/ERS Standardisation of Spirometry (Miller et al. 2005, Eur Respir J 26:319-38).

DLco will be performed in accordance with ATS/ERS guidelines, including correction for serum hemoglobin concentration, and will be measured along with FVC as a part of the primary and secondary endpoint assessments. The acceptability of the data, including the graphic representations of the maneuvers, will be determined by blinded over-readers. Calculations for the reproducibility of the acceptable maneuvers will be programmed.

### High-Resolution Computed Tomography

Prone pulmonary HRCT scans will be performed and recorded. Diagnosis of IPF by pulmonary HRCT scan should demonstrate a symmetrical pattern of bibasilar, peripheral, or subpleural intralobular septal thickening, fibrotic changes, honeycombing and traction bronchiectasis, or bronchiolectasis. There may be associated ground-glass opacity of the lungs.

### Six-Minute Walk Test

The 6MWT test will be conducted according to focused ATS guidelines (ATS Statement 2002, Am J Respir Crit Care Med 166:111-117).

### Patient-Reported Outcomes

Patient-reported outcomes (PRO) data will be elicited from the patients in this study to more fully characterize the clinical profile of lebrikizumab. The PRO instruments, translated as required in the local language, will be completed in their entirety by the patient at specified timepoints during the study. Two PRO tools will be used:

### A Tool to Assess Quality of Life in Idiopathic Pulmonary Fibrosis (ATAQ-IPF)

The ATAQ-IPF is a 74-item IPF-specific quality of life instrument (Swigris et al. 2010, Health and Quality of Life Outcomes 8:77). A Tool to Assess Quality of Life (ATAQ) is comprised of 13 domains: cough (6 items), dyspnea (6 items), forethought (5 items), sleep (6 items), mortality (6 items), exhaustion (5 items), emotional well-being (7 items), social participation (5 items), finances (6 items), independence (5 items), sexual health (5 items), relationships (6 items), and therapies (6 items). Each item of the ATAQ is assessed on a scale ranging from 1 (Strongly disagree) to 5 (Strongly agree). No recall period is specified in the ATAQ. The pattern of correlations between the ATAQ-IPF scores and physiologic variables known to be important in IPF, along with significant differences in the ATAQ-IPF scores between subjects using versus those not using supplemental oxygen, supports its validity.

### EuroQol 5-Dimension

The EQ-5D is generic preference-based health-related quality of life questionnaire that provides a single index value for health status (Rabin and de Charro 2001, Ann Med 33:337-43). This tool includes questions about mobility, self-care, usual activities, pain/discomfort, and anxiety/depression that are used to build a composite of the patient's health status.

Embodiments of the present invention are as follows:
1. A method of prognosing or of aiding prognosis of idiopathic pulmonary fibrosis (IPF) in a patient comprising obtaining a biological sample from the patient, measuring in the biological sample the expression of one or a combination of genes, or expression of one or a combination of proteins encoded by the one or the combination of genes, wherein the one or the combination of genes is selected from any of Table 2, Table 3, Table 4, or Table 5, wherein an elevated expression level of the one or the combination of genes, or an elevated expression level of the one or the combination of proteins, is indicative of a prognosis for shortened survival compared to median survival and wherein a reduced expression level of the one or the combination of genes, or a reduced expression level of the one or the combination of proteins, is indicative of a prognosis for increased survival compared to median survival.
2. The method of embodiment 1, wherein the one or the combination of genes, or the one or the combination of proteins, is selected from MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3, and SAA4.
3. The method of embodiment 1, wherein the one or the combination of genes is selected from CXCR3, CXCR5, CXCL13, CCR6, CCR7, CD19, MS4A1 (CD20), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@, IGHA1, IGKV2-24, IGKV1D-8, IGHM.
4. The method of embodiment 1, wherein the one or the combination of genes, or the one or the combination of proteins, is selected from COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3.
5. The method of embodiment 1, wherein the one or the combination of genes, or the one or the combination of proteins is selected from CHI3L1 (YKL-40), CCL11, CCL13, CCL17, CCL18, COMP, CXCL13, MMP3, MMP7, SAA4 (constitutive SAA), POSTN, AND SPP1 (OPN).
6. The method of embodiment 5, wherein the one or the combination of genes, or the one or the combination of proteins, is selected from MMP3 and SAA4 (constitutive SAA).
7. The method of embodiment 5 wherein the one or the combination of genes, or the one or the combination of proteins, is selected from YKL-40 and CCL18.
8. The method of embodiment 5, comprising measuring the expression level of CXCL13.
9. The method of embodiment 5, comprising measuring the expression level of MMP3.
10. The method of embodiment 5, comprising measuring the expression level of SAA4 (constitutive SAA).
11. The method of embodiment 5, wherein the patient is on immunomodulatory therapy.
12. The method of any one of embodiments 1-11, wherein the biological sample is selected from lung tissue, serum, and plasma.
13. A method of prognosing or of aiding prognosis of IPF in a patient comprising obtaining a biological sample from the patient and determining a total baseline biomarker score, wherein the determining of the total baseline biomarker score comprises measuring the protein expression level of at least one of CXCL13, OPN, and COMP and assigning a score of 0 if the expression level is below the median for CXCL13, OPN, and COMP, respectively, and assigning a score of 1 if the expression level is above the median for CXCL13, OPN, and COMP, respectively, wherein the determining of the total baseline biomarker score further comprises measuring the protein expression level of YKL-40 and assigning a score of 0 if the expression level is below the median for YKL-40 and assigning a score of 1 if the expression level is above the median for YKL-40, and wherein the determining of the total baseline biomarker score further comprises adding each individual score to obtain the total baseline biomarker score, wherein a total baseline biomarker score of two or above is indicative of a prognosis of shortened survival compared to median survival and wherein a total baseline biomarker score of zero or one is indicative of a prognosis of increased survival compared to the median survival.
14. The method of embodiment 13, wherein the total baseline biomarker score of the patient is two or above and the patient is selected for treatment with a candidate therapeutic agent in a clinical study, wherein the candidate therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120).
15. The method of embodiment 14, wherein the anti-IL-13 agent is lebrikizumab.
16. The method of embodiment 14, wherein the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6.
17. The method of embodiment 16, wherein the anti-IL-13 antibody comprises a heavy chain variable region having the amino acid sequence of SEQ ID NO.: 7 and a light chain variable region having the amino acid sequence of SEQ ID NO.: 9.
18. The method of embodiment 17, wherein the anti-IL-13 antibody comprises a heavy chain having the amino acid sequence of SEQ ID NO.: 10 and a light chain having the amino acid sequence of SEQ ID NO.: 14.
19. The method of embodiment 14, wherein the anti-IL-13/anti-IL-4 agent is a bispecific antibody.
20. A method of prognosing or of aiding prognosis of IPF in a patient comprising obtaining a biological sample from the patient and determining a total baseline biomarker score, wherein the determining of the total baseline biomarker score comprises measuring the protein expression level of at least one of MMP3 and COMP and assigning a score of 0 if the expression level is below the median for MMP3 and COMP, respectively, and assigning a score of 1 if the expression level is above the median for MMP3 and COMP, respectively, wherein the determining of the total baseline biomarker score further comprises measuring the protein expression level of YKL-40 and assigning a score of 0 if the expression level is below the median for YKL-40 and assigning a score of 1 if the expression level is above the median for YKL-40, and wherein the determining of the total baseline biomarker score further comprises adding each individual score to obtain the total baseline biomarker score, wherein a total baseline biomarker score of one or above is indicative of a prognosis of shortened survival compared to median survival and wherein a total baseline biomarker score of zero is indicative of a prognosis of increased survival compared to the median survival.
21. The method of any one of embodiments 13-20, wherein the biological sample is selected from serum and plasma.
22. The method of embodiment 20, wherein the total baseline biomarker score of the patient is one or above and the patient is selected for treatment with a candidate therapeutic agent in a clinical study, wherein the candidate therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120).
23. The method of embodiment 22, wherein the anti-IL-13 agent is lebrikizumab.
24. The method of embodiment 22, wherein the anti-IL-13/anti-IL-4 agent is a bispecific antibody.
25. The method of embodiment 22, wherein the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6.
26. The method of embodiment 25, wherein the anti-IL-13 antibody comprises a heavy chain variable region having the amino acid sequence of SEQ ID NO.: 7 and a light chain variable region having the amino acid sequence of SEQ ID NO.: 9.
27. The method of embodiment 26, wherein the anti-IL-13 antibody comprises a heavy chain having the amino acid sequence of SEQ ID NO.: 10 and a light chain having the amino acid sequence of SEQ ID NO.: 14.
28. A method of diagnosing a molecular subtype of IPF in a subject, the method comprising measuring in a biological sample obtained from the subject expression of one or a combination of genes, or expression of one or a combination of proteins encoded by the one or the combination of genes, wherein the one or the combination of genes is selected from MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3, and SAA4 wherein elevated expression of the one or the combination of genes, or elevated expression of the one or the combination of proteins, is indicative of the IPF molecular subtype.
29. A method of diagnosing a molecular subtype of IPF in a subject, the method comprising measuring in a biological sample obtained from the subject expression of one or a combination of genes, or expression of one or a combination of proteins encoded by the one or the combination of genes, wherein the one or the combination of genes is selected from CXCR3, CXCR5, CXCL13, CCR6, CCR7, CD19, MS4A1 (CD20), TNFRSF17 (BCMA), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@ (immunoglobulin kappa locus), IGHA1, IGKV2-24, IGKV1D-8, IGHM, wherein elevated expression of the one or the combination of genes, or elevated expression of the one or the combination of proteins, is indicative of the IPF molecular subtype.
30. A method of diagnosing a molecular subtype of IPF in a subject, the method comprising measuring in a biological sample obtained from the subject expression of one or a combination of genes, or expression of one or a combination of proteins encoded by the one or the combination of genes, wherein the one or the combination of genes is selected from COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3, wherein elevated expression of the one or the combination of genes, or elevated expression of the one or the combination of proteins, is indicative of the IPF molecular subtype.
31. The method of any one of embodiments 28-30, wherein the biological sample is lung tissue, whole blood, or serum.
32. The method of any one of embodiments 12, 21, or 31, wherein the biological sample is lung tissue, plasma or whole blood and the expression of the one or the combination of genes is measured using a PCR method or a microarray chip.
33. The method of embodiment 31, wherein the biological sample is serum and the expression of the one or the combination of proteins is measured using an immunoassay.
34. A method of treating IPF in a patient comprising administering an effective amount of an IPF therapeutic agent to the patient to treat the IPF, provided that elevated expression of one or a combination of genes, or expression of one or a combination of proteins encoded by the one or the combination of genes, has been detected in a biological sample obtained from the patient, wherein the one or the combination of genes is selected from MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3 and SAA4.
35. A method of treating IPF in a patient comprising administering an effective amount of an IPF therapeutic agent to the patient to treat the IPF, provided that elevated expression of one or a combination of genes, or expression of one or a combination of proteins encoded by the one or the combination of genes, has been detected in a biological sample obtained from the patient, wherein the one or the combination of genes is selected from CXCR3, CXCR5, CXCL13, CCR6, CCR7, CD19, MS4A1 (CD20), TNFRSF17 (BCMA), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@ (immunoglobulin kappa locus), IGHA1, IGKV2-24, IGKV1D-8, IGHM.
36. A method of treating IPF in a patient comprising administering an effective amount of an IPF therapeutic agent to the patient to treat the IPF, provided that elevated expression of one or a combination of genes, or expression of one or a combination of proteins encoded by the one or the combination of genes, has been detected in a biological sample obtained from the patient, wherein the one or the combination of genes is selected from COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3.
37. The method of any one of embodiments 34-36, wherein the IPF therapeutic agent is selected from an anti-IL-13 agent, an anti-IL-4 agent, a combination anti-IL-13/IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120).
38. The method of embodiment 37, wherein the IPF therapeutic agent is an anti-IL-13 agent, wherein the IL-13 agent is an anti-IL-13 antibody, and wherein the anti-IL-13 antibody is lebrikizumab.
39. The method of embodiment 37, wherein the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6.
40. The method of embodiment 39, wherein the anti-IL-13 antibody comprises a heavy chain variable region having the amino acid sequence of SEQ ID NO.: 7 and a light chain variable region having the amino acid sequence of SEQ ID NO.: 9.
41. The method of embodiment 40, wherein the anti-IL-13 antibody comprises a heavy chain having the amino acid sequence of SEQ ID NO.: 10 and a light chain having the amino acid sequence of SEQ ID NO.: 14.
42. The method of any one of embodiments 38-41, wherein the anti-IL-13 antibody is administered subcutaneously once every four weeks at a flat dose selected from 125 mg, 250 mg, and 500 mg.
43. The method of embodiment 42, wherein the anti-IL-13 antibody is administered subcutaneously once every four weeks at a flat dose of 250 mg.
44. The method of embodiment 37, wherein the IPF therapeutic agent is a combination anti-IL-13/anti-IL-4 agent.
45. The method of embodiment 44, wherein the anti-IL-13/anti-IL-4 agent is a bispecific antibody.
46. A method of treating an IPF patient previously determined to have a prognosis of shortened survival according to the method of any one of embodiments 1-13, 20, or 21 comprising administering an effective amount of an IPF therapeutic agent.
47. The method of embodiment 46, wherein the IPF therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120).
48. The method of embodiment 47, wherein the anti-IL-13 agent is lebrikizumab.
49. The method of embodiment 47 , wherein the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6.
50. The method of embodiment 49, wherein the anti-IL-13 antibody comprises a heavy chain variable region having the amino acid sequence of SEQ ID NO.: 7 and a light chain variable region having the amino acid sequence of SEQ ID NO.: 9.
51. The method of embodiment 50, wherein the anti-IL-13 antibody comprises a heavy chain having the amino acid sequence of SEQ ID NO.: 10 and a light chain having the amino acid sequence of SEQ ID NO.: 14.
52. The method of any one of embodiments 48-51, wherein the anti-IL-13 antibody is administered subcutaneously once every four weeks at a flat dose selected from 125 mg, 250 mg, and 500 mg.
53. The method of embodiment 52, wherein the anti-IL-13 antibody is administered subcutaneously once every four weeks at a flat dose of 250 mg.
54. A method of treating an IPF patient comprising administering an effective amount of an IPF therapeutic agent, provided that the patient has a baseline score of two or above determined according to the method of embodiment 13.
55. A method of treating an IPF patient comprising administering an effective amount of an IPF therapeutic agent, provided that the patient has a baseline score of one or above determined according to the method of embodiment 20.
56. The method of embodiment 54 or embodiment 55, wherein the IPF therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120).
57. The method of embodiment 56, wherein the anti-IL-13 agent is lebrikizumab.
58. The method of embodiment 56 , wherein the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6.
59. The method of embodiment 58, wherein the anti-IL-13 antibody comprises a heavy chain variable region having the amino acid sequence of SEQ ID NO.: 7 and a light chain variable region having the amino acid sequence of SEQ ID NO.: 9.
60. The method of embodiment 59, wherein the anti-IL-13 antibody comprises a heavy chain having the amino acid sequence of SEQ ID NO.: 10 and a light chain having the amino acid sequence of SEQ ID NO.: 14.
61. The method of any one of embodiments 57-60, wherein the anti-IL-13 antibody is administered subcutaneously once every four weeks at a flat dose selected from 125 mg, 250 mg, and 500 mg.
62. The method of embodiment 61, wherein the anti-IL-13 antibody is administered subcutaneously once every four weeks at a flat dose of 250 mg.
63. The method of embodiment 56, wherein the IPF therapeutic agent is a combination anti-IL-13/anti-IL-4 agent.
64. The method of embodiment 63, wherein the anti-IL-13/anti-IL-4 agent is a bispecific antibody.
65. The method of any one of embodiments 34-64, wherein the treatment extends the time to disease progression compared to no treatment, wherein disease progression is indicated by the first occurrence of one or more of the following: (i) death; (ii) non-elective hospitalization; (iii) 10% or greater decrease from baseline in FVC.
66. The method of embodiment 65, wherein disease progression is further indicated by decrease from baseline of ≥ 15% in DLco at week 52.
67. The method of any one of embodiments 34-64, wherein a decrease in DLco from baseline 52 weeks after treatment is less than 15%.
68. The method of any one of embodiments 34-64, wherein the treatment results in less reduction in decline from baseline in distance walked by the patient in a 6-minute walk test 52 weeks after treatment compared to no treatment.
69. The method of embodiment 68, wherein the reduction in decline from baseline in distance walked is greater than 50 meters, or greater than 30 meters, or greater than 10 meters.
70. The method of any one of embodiments 34-64, wherein the treatment extends the time to a first event of acute IPF exacerbation or a first event of IPF deterioration compared to no treatment.
71. A method of monitoring disease progression in an IPF patient comprising obtaining a biological sample from the patient at a first time point and one or more additional time points, measuring in the biological samples the expression of one or a combination of genes, or expression of one or a combination of proteins encoded by the one or the combination of genes, wherein the one or the combination of genes is selected from any of Table 2, Table 3, Table 4, or Table 5, wherein a change in expression level from the first time point to the one or more additional time points is indicative of disease progression.
72. The method of embodiment 71, wherein the one or the combination of genes, or the one or the combination of proteins, is selected from MUCL1, MUC4, MUC20, PRR7, PRR15, SPRR1B, SPRR2D, KRT5, KRT6B, KRT13, KRT14, KRT15, KRT17, SERPINB3, SERPINB4, SERPINB5, SERPINB13, CLCA2, TRPV4, BBS5, MMP3, and SAA4.
73. The method of embodiment 71, wherein the one or the combination of genes is selected from CXCR3, CXCR5, CXCL13, CCR6, CCR7, CD19, MS4A1 (CD20), BLK, BLNK, FCRLA, FCRL2, FCRL5, CD79A, CD79B, CD27, CD28, CD1A, CD1B, CD1C, CD1E, IGHV1-69, IGLJ3, IGJ, IGHV3-48, IGLV3-21, IGKV1-5, IGHG1, IGKC, IGLV6-57, IGK@, IGHA1, IGKV2-24, IGKV1D-8, IGHM.
74. The method of embodiment 71, wherein the one or the combination of genes, or the one or the combination of proteins, is selected from COL1A1, COL1A2, COL5A2, COL12A1, COL14A1, COL15A1, COL16A1, COL18A1, CTHRC1, HGF, IGFBP7, SCGF (CLEC11A); LOXL1, LOXL2; GLI1, GLI2, SMO; SFRP2, DIO2, CDH11, POSTN, and TGFB3.
75. The method of embodiment 71, wherein the one or the combination of genes, or the one or the combination of proteins is selected from CHI3L1 (YKL-40), CCL11, CCL13, CCL17, CCL18, COMP, CXCL13, MMP3, MMP7, SAA4 (constitutive SAA), POSTN, AND SPP1 (OPN).
76. The method of any one of embodiments 71-75, wherein the biological samples are selected from lung tissue, serum, and plasma.
77. The method of embodiment 76, wherein the biological sample is lung tissue or plasma and the expression of the one or the combination of genes is measured using a PCR method or a microarray chip.
78. The method of embodiment 76, wherein the biological sample is serum and the expression of the one or the combination of proteins is measured using an immunoassay.
79. The method of any one of embodiments 71-78, further comprising treating the patient with a candidate therapeutic agent in a clinical study.
80. The method of embodiment 79, wherein the candidate therapeutic agent is selected from an anti-IL-13 agent, an anti-IL-4 agent, a combination anti-IL-13/anti-IL-4 agent, pirfenidone, anti-LOXL2 antibody (GS-6624), N-acetylcysteine, anti-TGF-β antibody (GC1008), anti-αvβ6 integrin antibody (STX-100), anti-CTGF antibody (FG-3019), anti-CCL2 antibody (CNTO 888), somatostatin analog (SOM230, octreotide), antiotensin II inhibitor (losartan), carbon monoxide, thalidomide, tetrathiomolybdate, doxycycline, minocycline, and tyrosine kinase inhibitor (BIBF1120).
81. The method of embodiment 80, wherein the anti-IL-13 agent is lebrikizumab.
82. The method of embodiment 80 , wherein the anti-IL-13 agent is an anti-IL-13 antibody comprising three heavy chain CDRs, CDR-H1 having the amino acid sequence of SEQ ID NO.: 1, CDR-H2 having the amino acid sequence of SEQ ID NO.: 2, and CDR-H3 having the amino acid sequence of SEQ ID NO.: 3, and three light chain CDRs, CDR-L1 having the amino acid sequence of SEQ ID NO.: 4, CDR-L2 having the amino acid sequence of SEQ ID NO.: 5, and CDR-L3 having the amino acid sequence of SEQ ID NO.: 6.
83. The method of embodiment 82, wherein the anti-IL-13 antibody comprises a heavy chain variable region having the amino acid sequence of SEQ ID NO.: 7 and a light chain variable region having the amino acid sequence of SEQ ID NO.: 9.
84. The method of embodiment 83, wherein the anti-IL-13 antibody comprises a heavy chain having the amino acid sequence of SEQ ID NO.: 10 and a light chain having the amino acid sequence of SEQ ID NO.: 14.

## Claims

1. An idiopathic pulmonary fibrosis (IPF) therapeutic agent for use in treating IPF in a patient, wherein the patient has a total baseline biomarker score of two or above, wherein the total baseline biomarker score is determined by measuring the protein expression level of at least one of CXCL13, OPN, and COMP and assigning a score of 0 if the expression level is below the median for CXCL13, OPN, and COMP, respectively, and assigning a score of 1 if the expression level is above the median for CXCL13, OPN, and COMP, respectively, wherein the determining of the total baseline biomarker score further comprises measuring the protein expression level of YKL-40 and assigning a score of 0 if the expression level is below the median for YKL-40 and assigning a score of 1 if the expression level is above the median for YKL-40, and wherein the determining of the total baseline biomarker score further comprises adding each individual score to obtain the total baseline biomarker score.

2. The IPF therapeutic agent for use of claim 1, wherein the biological sample is selected from lung tissue, serum, and plasma.

3. The IPF therapeutic agent for use of claim 1 or claim 2, wherein the IPF therapeutic agent is selected from an anti-IL-13 antibody, an anti-IL-4 antibody, a bispecific anti-IL-13/anti-IL-4 antibody, pirfenidone, an anti-LOXL2 antibody, N-acetylcysteine, an anti-TGF-β antibody, an anti-αvβ6 integrin antibody, an anti-CTGF antibody, an anti-CCL2 antibody, a somatostatin analog, an antiotensin II inhibitor, carbon monoxide, thalidomide, tetrathiomolybdate, doxycycline, minocycline, and a tyrosine kinase inhibitor.

4. The IPF therapeutic agent for use of claim 3, wherein the IPF therapeutic agent is an anti-IL-13 antibody, and wherein the anti-IL-13 antibody comprises a CDR-H1 having the amino acid sequence of SEQ ID NO.: 1, a CDR-H2 having the amino acid sequence of SEQ ID NO.: 2, and a CDR-H3 having the amino acid sequence of SEQ ID NO.: 3, and a CDR-L1 having the amino acid sequence of SEQ ID NO.: 4, a CDR-L2 having the amino acid sequence of SEQ ID NO.: 5, and a CDR-L3 having the amino acid sequence of SEQ ID NO.: 6.

5. The IPF therapeutic agent for use of claim 3, wherein the IPF therapeutic agent is an anti-IL-13 antibody, and wherein the anti-IL-13 antibody comprises a heavy chain variable region having the amino acid sequence of SEQ ID NO.: 7 and a light chain variable region having the amino acid sequence of SEQ ID NO.: 9.

6. The IPF therapeutic agent for use of claim 3, wherein the IPF therapeutic agent is an anti-IL-13 antibody, and wherein the anti-IL-13 antibody comprises a heavy chain having the amino acid sequence of SEQ ID NO.: 10 and a light chain having the amino acid sequence of SEQ ID NO.: 14.

7. The IPF therapeutic agent for use of any one of claims 4-6, wherein the anti-IL-13 antibody is administered subcutaneously once every four weeks at a flat dose selected from 125 mg, 250 mg, and 500 mg.

8. The IPF therapeutic agent for use of claim 3, wherein the IPF therapeutic agent is a bispecific anti-IL-13/anti-IL-4 antibody.

9. The IPF therapeutic agent for use of any one of claims 1-8, wherein the treating extends the time to disease progression compared to no treatment, wherein disease progression is indicated by the first occurrence of one or more of the following: (i) death; (ii) non-elective hospitalization; (iii) 10% or greater decrease from baseline in FVC.

10. The IPF therapeutic agent for use of claim 9, wherein disease progression is further indicated by decrease from baseline of ≥ 15% in DL_{CO} at week 52.

11. The IPF therapeutic agent of any one of claims 1-8, wherein a decrease in DL_{CO} from baseline at 52 weeks after treatment is less than 15%.

12. The IPF therapeutic agent for use of any one of claims 1-8, wherein the treating results in less reduction in decline from baseline in distance walked by the patient in a 6-minute walk test 52 weeks after treatment compared to no treatment.

13. The IPF therapeutic agent for use of claim 12, wherein the reduction in decline from baseline in distance walked is greater than 50 meters, or greater than 30 meters, or greater than 10 meters.

14. The IPF therapeutic agent for use of any one of claims 1-8, wherein the treating extends the time to a first event of acute IPF exacerbation or a first event of IPF deterioration compared to no treatment.
